(19) Europäisches Patentamt
European Patent Office
Office européen des brevets

(11) **EP 4 538 275 A1**

(12) **EUROPEAN PATENT APPLICATION**
published in accordance with Art. 153(4) EPC

(43) Date of publication:
**16.04.2025 Bulletin 2025/16**

(21) Application number: **23823906.5**

(22) Date of filing: **12.06.2023**

(51) International Patent Classification (IPC):
*C07D 491/20* (2006.01) *A61K 31/4427* (2006.01)
*A61P 3/04* (2006.01) *A61P 43/00* (2006.01)

(52) Cooperative Patent Classification (CPC):
**A61K 31/4427; A61P 3/04; A61P 43/00; C07D 491/20**

(86) International application number:
**PCT/JP2023/021799**

(87) International publication number:
**WO 2023/243616 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022 JP 2022094833**

(71) Applicant: **Shionogi & Co., Ltd**
**Osaka-shi, Osaka 541-0045 (JP)**

(72) Inventors:
- **SUGITA, Katsuji**
  **Osaka-shi, Osaka 541-0045 (JP)**
- **TAKATA, Yusuke**
  **Osaka-shi, Osaka 541-0045 (JP)**
- **MIYANO, Tetsuya**
  **Osaka-shi, Osaka 541-0045 (JP)**

(74) Representative: **Vossius & Partner Patentanwälte Rechtsanwälte mbB**
**Siebertstrasse 3**
**81675 München (DE)**

# (54) CRYSTAL OF DIHYDROPYRIDINONE DERIVATIVE OR SOLVATE THEREOF

(57) To provide a crystal of a dihydropyridinone derivative or a solvate thereof. The present invention relates to a crystal of a compound represented by Formula:

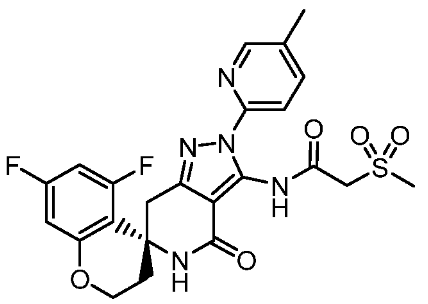
(I)

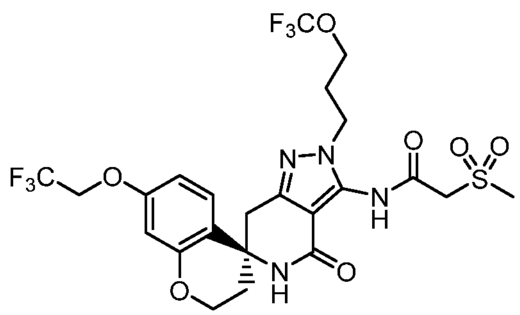
(II)

or a solvate thereof, and a pharmaceutical composition containing the same.

EP 4 538 275 A1

## Description

[TECHNICAL FIELD]

The present invention relates to a crystal of a dihydropyridinone derivative or a solvate (preferably, hydrate) thereof and a pharmaceutical composition containing the same.

[BACKGROUND ART]

Obesity is defined as an excessively high amount of body fat or adipose tissue in relation to lean body mass and recognized as a major risk factor for health problems. Body mass index (BMI) is a simple index of weight-for-height that is commonly used in classifying overweight and obesity in adult (age 15 and over) populations and individuals. It is defined as the weight in kilograms divided by the square of the height in meters ($kg/m^2$). World Health Organization defines "overweight" as a BMI of 25 $kg/m^2$ or greater and "obesity" as a BMI of 30 $kg/m^2$ or greater. On the other hand, Japan Society for the Study of Obesity defines "obesity" as a BMI of 25 $kg/m^2$ or greater. This is because the number of obesity-related disorders including diabetes and dislipidemia increases in accordance with BMI, and the mean number of obesity-related disorders is 1.0 or greater at a BMI of 25 $kg/m^2$. World Health Organization reported that about 1600 million and at least 400 million people were classified as overweight and obesity around the world in 2005, respectively. Obesity is mainly caused by taking in more calories than using up in physical activity and daily life. The number of obese people has been increasing by taking in more food including high fat and/or sugar, and it is estimated that 700 million people or more would be diagnosed as obesity around the world in 2015. Diet therapy, exercise therapy, drug therapy, and so on are performed for treatment of obesity. In the drug therapy, drugs including orlistat, mazindol, and sibutramine are used. However, they are not satisfactory in both aspects of efficacy and side effects.

One of the causes for obesity is excessive intake of neutral fat. Neutral fat (triglycerol) taken in meals is decomposed into 2-monoacylglycerol and free fatty acids by the action of pancreatic lipase in the digestive tract, and they are absorbed by small intestinal epithelial cells. An acyl group is transferred from the free fatty acids to the 2-monoacylglycerol by the action of monoacylglycerol acyltransferase (MGAT). The diacylglycerol formed is further converted into neutral fat by the action of diacylglycerol acyltransferase (DGAT).

Three isoforms of MGAT, namely, MGAT1, MGAT2, and MGAT3 have been identified. Among them, MGAT2 and MGAT3 are highly expressed in the small intestine, and believed to be involved in fat absorption in the small intestine.

It has been reported that an experiment with MGAT2 knock-out mice has demonstrated that high-fat diet promotes expression of MGAT2 in the small intestine to increase the MGAT activity (Non-patent Document 1). In addition, reduction of weight gain caused by high-fat diet, suppression of induction of insulin resistance, reduction of increase of blood cholesterol, prevention of fatty liver formation or the like, and promotion of energy consumption have been found for MGAT2 knock-out mice (Non-patent Document 2).

Patent Documents 1 and 2 describe that a dihydropyridinone derivative derivative represented by Formula:

[Chemical Formula 1]

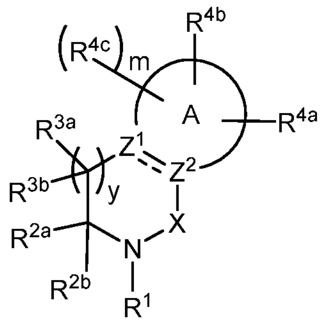

(I)

has an MGAT2 inhibitory action, and is useful for treatment and/or prevention of diseases involving MGAT2.

In Examples of Patent Documents 1 and 2, the following compound:

[Chemical Formula 2]

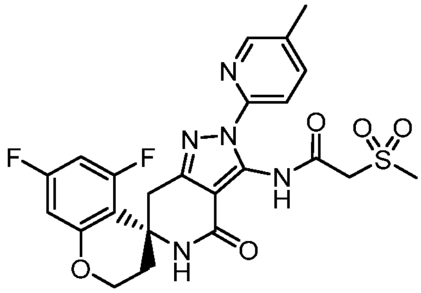

II-203

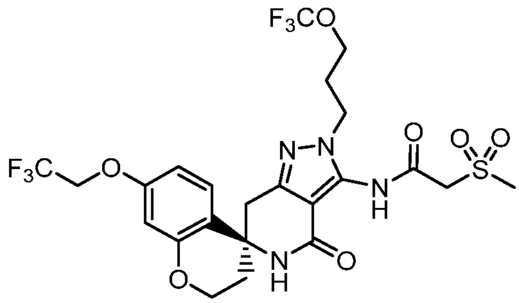

II-121

is disclosed, but a crystal of the compound is not disclosed.

[PRIOR ART REFERENCES]

[Patent Documents]

[Patent Document 1] International Publication WO 2019/013311A
[Patent Document 2] International Publication WO 2019/013312A

[Non-patent Documents]

[Non-patent Document 1] Journal of Biological Chemistry (2004), 279, 18878-18886
[Non-patent Document 2] Nature Medicine (2009), 15, (4), 442-446

[SUMMARY OF THE INVENTION]

[PROBLEMS TO BE SOLVED BY THE INVENTION]

The pharmaceutically active ingredients may have substantially different physical properties depending on the respective solid form. Such differences in physical properties may affect, for example, the method of producing or administering a pharmaceutically active ingredient, or a pharmaceutical composition comprising the pharmaceutically active ingredient. The present invention relates to crystal of a dihydropyridinone derivative or a solvate thereof that is very useful as compared to other solid forms in a method for producing or administering a pharmaceutically active ingredient, or in a pharmaceutical composition comprising a pharmaceutically active ingredient.
In general, physical properties of a crystal of a compound useful as a pharmaceutical product have a great influence on bioavailability of drugs, purity of drug substances, formulation of preparations, and the like, and thus are extremely important in development of pharmaceutical products. Furthermore, since their physical properties depend on the attributes of individual compounds, it is generally difficult to predict a crystal form of a drug substance having good physical properties, and it is required to actually variously examine each compound. In particular, the compounds described in Patent Documents 1 and 2 tend to decrease in solubility when crystallized.
Therefore, it is necessary to study which crystal form of a compound particularly useful as a pharmaceutical product among the compounds described in Patent Documents 1 and 2 has good solubility and is most excellent as a pharmaceutical product.

[MEANS FOR SOLVING THE PROBLEM]

As a result of intensive studies, the present inventors have found that a specific crystal form of the compound represented by Formula (I) or (II) is stable and has good purity and/or solubility.
The present invention relates to the following.

[1] An anhydride crystal of the compound represented by Formula (I):

[Chemical Formula 3]

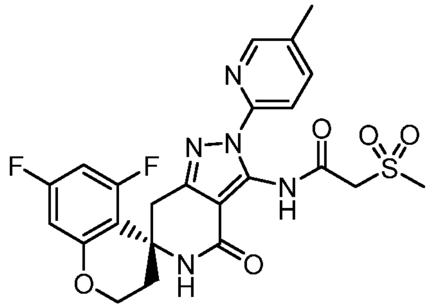

(I)

and/or a hydrate crystal of the compound represented by Formula (I) having a water content corresponding to 0.25 hydrates or less.

[2] The hydrate crystal according to [1], wherein the hydrate crystal has a water content corresponding to less than 0.25 hydrates.

[3] The hydrate crystal according to [2], which exhibits a powder X-ray diffraction pattern having peaks at diffraction angles (2θ): 13.0° ± 0.2°, 18.4° ± 0.2°, 20.0° ± 0.2°, 20.4° ± 0.2°, and 23.7° ± 0.2°.

[4] The hydrate crystal according to [3], which exhibits a powder X-ray diffraction pattern having peaks at diffraction angles (2θ): 13.0°±0.2°, 15.5°±0.2°, 16.6° ± 0.2°, 16.9° ± 0.2°, 17.4 ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2°.

[5] The 0.25 hydrate crystal according to [1].

[6] The 0.25 hydrate crystal according to [5], which exhibits a powder X-ray diffraction pattern having peaks at diffraction angles (2θ): 13.0° ± 0.2°, 18.4° ± 0.2°, 19.9° ± 0.2°, 20.5° ± 0.2°, and 23.7° ± 0.2°.

[7] The 0.25 hydrate crystal according to [6], which exhibits a powder X-ray diffraction pattern having peaks at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2° ± 0.2°.

[8] The anhydride crystal according to [1], which exhibits a powder X-ray diffraction pattern having peaks at diffraction angles (2θ): 13.1° ± 0.2°, 18.3° ± 0.2°, 20.2° ± 0.2°, 20.5° ± 0.2°, and 23.8° ± 0.2°.

[9] The anhydride crystal according to [8], which exhibits a powder X-ray diffraction pattern having peaks at diffraction angles (2θ): 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2°.

[10] The crystal according to [1], in which

when the crystal is allowed to stand in an environment of a temperature of 25°C and a relative humidity of 35% and a powder X-ray diffraction experiment is performed in the same environment, the crystal has peaks in the diffraction angles as defined in [3] or [4],

when the crystal is allowed to stand in an environment of a temperature of 25°C and a relative humidity of 60% and a powder X-ray diffraction experiment is performed in the same environment, the crystal has peaks in the diffraction angles as defined in [6] or [7],

or

when the crystal is allowed to stand in an environment of a temperature of 50°C and a relative humidity of 25% and a powder X-ray diffraction experiment is performed in the same environment, the crystal has peaks in the diffraction angles as defined in [8] or [9].

[11] The crystal according to [1], which has a melting point of 236 to 237°C.

[12] The crystal according to [1], which has a melting point of 236 to 237°C as measured by differential scanning calorimetry (DSC) or thermogravimetry/differential thermal analysis (TG/DTA).

[13] A hydrate crystal of the compound represented by Formula (I):

[Chemical Formula 4]

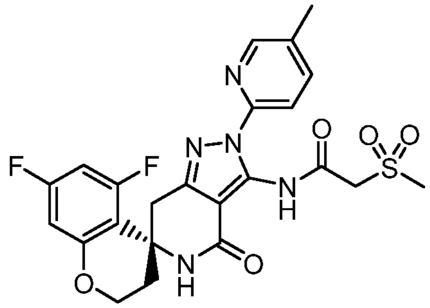

(I)

wherein water content Y (w%) is 0.0 < Y ≤ 1.5w%.

[14] The hydrate crystal of the compound represented by Formula (I) having a water content corresponding to 0.25 hydrates or less according to [1], wherein when single crystal structure analysis was performed with a CuKα radiation (λ = 1.5418 Å) at - 60°C, the hydrate crystal is characterized by the following crystallographic data:
Space group: P1

$$a = 11.61 \pm 0.05\ Å$$

$$b = 13.48 \pm 0.10\ Å$$

$$c = 15.50 \pm 0.05\ Å$$

$$\alpha = 91.5 \pm 0.6°$$

$$\beta = 105.3 \pm 0.1°$$

$$\gamma = 91.7 \pm 0.6°.$$

[15] The 0.25 hydrate crystal of the compound represented by Formula (I) according to [5], wherein when single crystal structure analysis was performed with a CuKα radiation (λ = 1.5418 Å) at -60°C, the 0.25 hydrate crystal is substantially characterized by the following crystallographic data:

Space group: P1
a = 11.5688(4) Å
b = 13.5769(4) Å
c = 15.5429(4) Å
α = 92.077(2)°
B = 105.324(3)°
γ = 92.281(3)°.

[16] The anhydride crystal according to [1], wherein when single crystal structure analysis was performed with a CuKα radiation (λ = 1.5418 Å) at -60°C, the anhydride crystal is substantially characterized by the following crystallographic data:

Space group: $P2_1$
a = 11.68520(10) Å
b = 13.35680(10) Å
c = 15.4299(2) Å
α = 90°
β = 105.3090(10)°
γ = 90°.

[17] The crystal according to [1], which exhibits a powder X-ray diffraction pattern having peaks at diffraction angles (2θ): 13.05° ± 0.25°, 18.35° ± 0.25°, 20.05° ± 0.35°, 20.45° ± 0.25°, and 23.75° ± 0.25°.

[18] The crystal according to [17], which exhibits a powder X-ray diffraction pattern having peaks at diffraction angles (2θ): 13.05° ± 0.25°, 15.55° ± 0.25°, 16.65° ± 0.25°, 17.00° ± 0.30°, 18.35° ± 0.25°, 19.35° ± 0.35°, 20.05° ± 0.35°, 20.45° ± 0.25°, 21.45° ± 0.25°, and 23.75° ± 0.25°.

[19] The crystal according to any of [1] to [18], having absorption peaks at 1448 $cm^{-1}$ ± 2 $cm^{-1}$, 1511 $cm^{-1}$ ± 2 $cm^{-1}$, and 2885 $cm^{-1}$ ± 2 $cm^{-1}$ in Raman spectrum.

[20] The crystal according to [19], having absorption peaks at 1448 $cm^{-1}$ ± 2 $cm^{-1}$, 1511 $cm^{-1}$ ± 2 $cm^{-1}$, 1650 $cm^{-1}$ ± 2 $cm^{-1}$, 2885 $cm^{-1}$ ± 2 $cm^{-1}$, and 2947$cm^{-1}$ ± 2$cm^{-1}$ in Raman spectrum.

[21] A crystal of the compound represented by Formula (II):

[Chemical Formula 5]

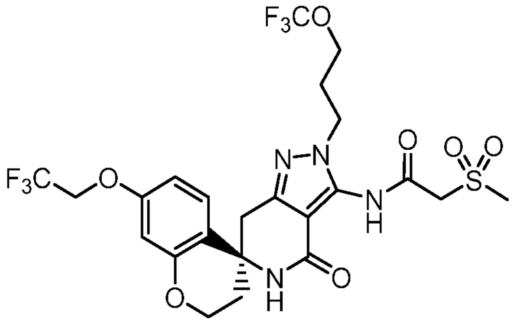

(II)

.

[22] The crystal according to [21], which exhibits a powder X-ray diffraction pattern having peaks at diffraction angles (2θ): 16.3° ± 0.2°, 18.4° ± 0.2°, 20.4° ± 0.2°, 22.8° ± 0.2°, and 23.9° ± 0.2°.

[23] The crystal according to [22], which exhibits a powder X-ray diffraction pattern having peaks at diffraction angles (2θ): 15.1° ± 0.2°, 16.3° ± 0.2°, 18.4° ± 0.2°, 19.2° ± 0.2°, 20.4° ± 0.2°, 21.1° ± 0.2°, 22.0° ± 0.2°, 22.8° ± 0.2°, 23.3° ± 0.2°, and 23.9° ± 0.2°.

[24] The crystal according to any of [21] to [23], having absorption peaks at 577 $cm^{-1}$ ± 2 $cm^{-1}$, 612 $cm^{-1}$ ± 2 $cm^{-1}$, 740 $cm^{-1}$ ± 2 $cm^{-1}$, 1571 $cm^{-1}$ ± 2 $cm^{-1}$, and 1718 $cm^{-1}$ ± 2 $cm^{-1}$ in Raman spectrum.

[25] A pharmaceutical composition containing the crystal according to any one of [1] to [24].

[1A] An anhydride crystal of the compound represented by Formula (I):

[Chemical Formula 6]

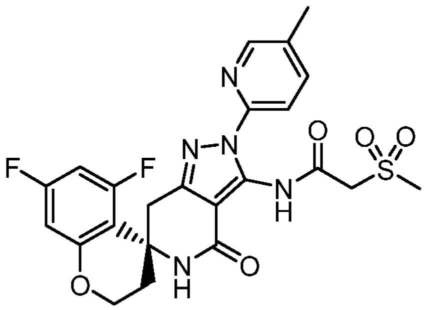

(I)

and/or a hydrate crystal having a water content corresponding to 0.25 hydrates or less.

[2A] The crystal according to [1A], which exhibits a powder X-ray diffraction pattern having peaks at diffraction angles (2θ): 13.0° ± 0.2°, 18.4° ± 0.2°, 20.0° ± 0.2°, 20.4° ± 0.2°, and 23.7° ± 0.2°.

[3A] The crystal according to [2A], which exhibits a powder X-ray diffraction pattern having peaks at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2°.

[4A] The 0.25 hydrate crystal according to [1A], which exhibits a powder X-ray diffraction pattern having peaks at diffraction angles (2θ): 13.0° ± 0.2°, 18.4° ± 0.2°, 19.9° ± 0.2°, 20.5° ± 0.2°, and 23.7° ± 0.2°.

[5A] The crystal according to [4A], which exhibits a powder X-ray diffraction pattern having peaks at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2° ± 0.2°.

[6A] The anhydride crystal according to [1A], which exhibits a powder X-ray diffraction pattern having peaks at diffraction angles (2θ): 13.1° ± 0.2°, 18.3° ± 0.2°, 20.2° ± 0.2°, 20.5° ± 0.2°, and 23.8° ± 0.2°.

[7A] The crystal according to [6A], which exhibits a powder X-ray diffraction pattern having peaks at diffraction angles (2θ): 13.1° ± 0.2°, 15.6° ± 0.2°, 16.7° ± 0.2°, 17.1 ± 0.2°, 18.3° ± 0.2°, 19.5° ± 0.2°, 20.2° ± 0.2°, 20.5° ± 0.2°, 21.5° ± 0.2°, and 23.8° ± 0.2°.

[8A] A crystal of the compound represented by Formula (II):

[Chemical Formula 7]

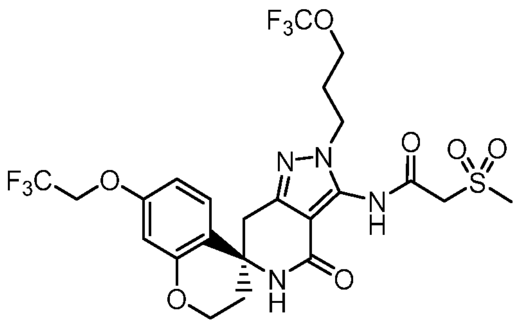

(II)

.

[9A] The crystal according to [8A], which exhibits a powder X-ray diffraction pattern having peaks at diffraction angles (2θ): 16.3° ± 0.2°, 18.4° ± 0.2°, 20.4° ± 0.2°, 22.8° ± 0.2°, and 23.9° ± 0.2°.

[10A] The crystal according to [9A], which exhibits a powder X-ray diffraction pattern having peaks at diffraction angles (2θ): 15.1° ± 0.2°, 16.3° ± 0.2°, 18.4° ± 0.2°, 19.2° ± 0.2°, 20.4° ± 0.2°, 21.1° ± 0.2°, 22.0° ± 0.2°, 22.8° ± 0.2°, 23.3° ± 0.2°, and 23.9° ± 0.2°.

[11A] A pharmaceutical composition containing the crystal according to any one of [1A] to [10A].

[EFFECT OF THE INVENTION]

A crystal of the present invention is useful as an active pharmaceutical ingredient of the compound represented by Formula (I) or (II). That is, a pharmaceutical composition containing the crystal of the present invention is very useful as a therapeutic agent or a prophylactic agent for a disease involving MGAT2.

[BRIEF DESCRIPTION OF THE DRAWINGS]

[Fig. 1] Fig. 1 shows a powder X-ray diffraction pattern of a 0.25 hydrate crystal of the compound represented by Formula (I) (measurement conditions are a temperature of 25°C and a relative humidity of 60%). The axis of abscissa represents 2θ (°), and the axis of ordinate represents the intensity (Count).

[Fig. 2] Fig. 2 shows a powder X-ray diffraction pattern of a hydrate crystal of the compound represented by Formula (I) (containing X molecules of water molecules per molecule of the compound represented by Formula (I), where $0 < X < 0.25$) (measurement conditions are a temperature of 25°C and a relative humidity of 35%). The axis of abscissa represents 2θ (°), and the axis of ordinate represents the intensity (Count).

[Fig. 3] Fig. 3 shows a powder X-ray diffraction pattern of an anhydride crystal of the compound represented by Formula (I) (measurement conditions are a temperature of 50°C and a relative humidity of 25%). The axis of abscissa represents 2θ (°), and the axis of ordinate represents the intensity (Count).

[Fig. 4] Fig. 4 shows a crystal structure (structure in the asymmetric unit) of the 0.25 hydrate crystal of the compound represented by Formula (I).

[Fig. 5] Fig. 5 shows a molecular structural diagram of the compound represented by Formula (I) (showing a molecule containing S1) of the 0.25 hydrate crystal of the compound represented by Formula (I).

[Fig. 6] Fig. 6 shows a molecular structural diagram of the compound represented by Formula (I) (showing a molecule containing S2) of the 0.25 hydrate crystal of the compound represented by Formula (I).

[Fig. 7] Fig. 7 shows a molecular structural diagram of the compound represented by Formula (I) (showing a molecule containing S3) of the 0.25 hydrate crystal of the compound represented by Formula (I).

[Fig. 8] Fig. 8 shows a molecular structural diagram of the compound represented by Formula (I) (showing a molecule containing S4) of the 0.25 hydrate crystal of the compound represented by Formula (I).

[Fig. 9] Fig. 9 shows a crystal structure (structure in the asymmetric unit) of the anhydride crystal of the compound represented by Formula (I).

[Fig. 10] Fig. 10 shows a molecular structural diagram of the compound represented by Formula (I) (showing a molecule containing S1) of the anhydride crystal of the compound represented by Formula (I).

[Fig. 11] Fig. 11 shows a molecular structural diagram of the compound represented by Formula (I) (showing a molecule containing S2) of the anhydride crystal of the compound represented by Formula (I).

[Fig. 12] Fig. 12 shows a DSC analysis result of a crystal of the compound represented by Formula (I) obtained in Example 1. The axis of ordinate represents the quantity of heat, and the axis of abscissa represents the temperature.

[Fig. 13] Fig. 13 shows a TG/DTA analysis result of the crystal of the compound represented by Formula (I) obtained in Example 1. The axis of ordinate represents the quantity of heat ($\mu$V) or the change in weight (%), and the axis of abscissa represents the temperature (°C). Cel in the diagram means degree Celsius (°C).

[Fig. 14] Fig. 14 shows a moisture adsorption/desorption isotherm of the crystal of the compound represented by Formula (I) obtained in Example 1. The axis of ordinate represents the change in weight (%) (Change In Mass), and the axis of abscissa represents the relative humidity (%) (Target% P/P0). The curve plotted with diamonds is the moisture adsorption isotherm, and the curve plotted with squares is the moisture desorption isotherm.

[Fig. 15] Fig. 15 shows a powder X-ray diffraction pattern of a crystal of the compound represented by Formula (II). The axis of abscissa represents 2$\theta$ (°), and the axis of ordinate represents the intensity (Count).

[Fig. 16] Fig. 16 shows a DSC analysis result of the crystal of the compound represented by Formula (II). The axis of ordinate represents the quantity of heat, and the axis of abscissa represents the temperature.

[Fig. 17] Fig. 17 shows TG/DTA analysis results of the crystal of the compound represented by Formula (II). The axis of ordinate represents the quantity of heat ($\mu$V) or the change in weight (%), and the axis of abscissa represents the temperature (°C). Cel in the diagram means degree Celsius (°C).

[Fig. 18] Fig. 18 shows a moisture adsorption/desorption isotherm of the crystal of the compound represented by Formula (II). The axis of ordinate represents the change in weight (%) (Change In Mass), and the axis of abscissa represents the relative humidity (%) (Target% P/P0). The curve plotted with diamonds is the moisture adsorption isotherm, and the curve plotted with squares is the moisture desorption isotherm.

[Fig. 19] Fig. 19 shows a peak of the powder X-ray diffraction pattern of the 0.25 hydrate crystal of the compound represented by Formula (I) (measurement conditions are a temperature of 25°C and a relative humidity of 60%). In the table, Position is 2$\theta$ (°), and Intensity represents the intensity.

[Fig. 20] Fig. 20 shows a peak of the powder X-ray diffraction pattern of an anhydride crystal of the compound represented by Formula (I) (measurement conditions are a temperature of 50°C and a relative humidity of 25%). In the table, Position is 2$\theta$ (°), and Intensity represents the intensity.

[Fig. 21] Fig. 21 shows a peak of the powder X-ray diffraction pattern of the hydrate crystal of the compound represented by Formula (I) (containing X molecules of water molecules per molecule of the compound represented by Formula (I), where $0 < X < 0.25$) (measurement conditions are a temperature of 25°C and a relative humidity of 35%). In the table, Position is 2$\theta$ (°), and Intensity represents the intensity.

[Fig. 22] Fig. 22 shows a Raman spectrum of the hydrate crystal of the compound represented by Formula (I) (containing X molecules of water molecules per molecule of the compound represented by Formula (I), where $0 < X < 0.25$). The axis of abscissa represents Raman shift ($cm^{-1}$), and the axis of ordinate represents the peak intensity.

[Fig. 23] Fig. 23 shows an enlarged view of Fig. 22. The axis of abscissa represents Raman shift ($cm^{-1}$), and the axis of ordinate represents the peak intensity.

[Fig. 24] Fig. 24 shows an enlarged view of Fig. 22. The axis of abscissa represents Raman shift ($cm^{-1}$), and the axis of ordinate represents the peak intensity.

[Fig. 25] Fig. 25 shows a powder X-ray diffraction pattern of an amorphous substance of the compound represented by Formula (I) obtained in Example 1. The axis of abscissa represents 2$\theta$ (°), and the axis of ordinate represents the intensity (Count).

[Fig. 26] Fig. 26 shows a moisture adsorption/desorption isotherm of the amorphous substance of the compound represented by Formula (I) obtained in Example 1. The axis of ordinate represents the change in weight (%) (Change In Mass), and the axis of abscissa represents the relative humidity (%) (Target% P/P0, unit: %). The curve plotted with diamonds is the moisture adsorption isotherm, and the curve plotted with squares is the moisture desorption isotherm.

[Fig. 27] Fig. 27 shows a Raman spectrum of the amorphous substance of the compound represented by Formula (I) obtained in Example 1. The axis of abscissa represents Raman shift ($cm^{-1}$), and the axis of ordinate represents the peak intensity.

[Fig. 28] Fig. 28 shows an enlarged view of Fig. 27. The axis of abscissa represents Raman shift ($cm^{-1}$), and the axis of ordinate represents the peak intensity.

[Fig. 29] Fig. 29 shows an enlarged view of Fig. 27. The axis of abscissa represents Raman shift ($cm^{-1}$), and the axis of ordinate represents the peak intensity.

[Fig. 30] Fig. 30 shows a peak of the powder X-ray diffraction pattern of the crystal of the compound represented by

Formula (II). In the table, Position is 2θ (°), and Intensity represents the intensity.

[Fig. 31] Fig. 31 shows a Raman spectrum of the crystal of the compound represented by Formula (II). The axis of abscissa represents Raman shift ($cm^{-1}$), and the axis of ordinate represents the peak intensity.

[Fig. 32] Fig. 32 shows an enlarged view of Fig. 31. The axis of abscissa represents Raman shift ($cm^{-1}$), and the axis of ordinate represents the peak intensity.

[Fig. 33] Fig. 33 shows an enlarged view of Fig. 31. The axis of abscissa represents Raman shift ($cm^{-1}$), and the axis of ordinate represents the peak intensity.

[Fig. 34] Fig. 34 shows a powder X-ray diffraction pattern of an amorphous substance of the compound represented by Formula (II). The axis of abscissa represents 2θ (°), and the axis of ordinate represents the intensity (Count).

[Fig. 35] Fig. 35 shows a moisture adsorption/desorption isotherm of the amorphous substance of the compound represented by Formula (II). The axis of ordinate represents the change in weight (%) (Change In Mass), and the axis of abscissa represents the relative humidity % (Target% P/P0). The curve plotted with diamonds is the moisture adsorption isotherm, and the curve plotted with squares is the moisture desorption isotherm.

[Fig. 36] Fig. 36 shows a Raman spectrum of the amorphous substance of the compound represented by Formula (II). The axis of abscissa represents Raman shift ($cm^{-1}$), and the axis of ordinate represents the peak intensity.

[Fig. 37] Fig. 37 shows an enlarged view of Fig. 36. The axis of abscissa represents Raman shift ($cm^{-1}$), and the axis of ordinate represents the peak intensity.

[Fig. 38] Fig. 38 shows an enlarged view of Fig. 37. The axis of abscissa represents Raman shift ($cm^{-1}$), and the axis of ordinate represents the peak intensity.

[Fig. 39] Fig. 39 shows a powder X-ray diffraction pattern of a B-form crystal of the compound represented by Formula (I). The axis of abscissa represents 2θ (°), and the axis of ordinate represents the intensity.

[Fig. 40] Fig. 40 shows a peak of the powder X-ray diffraction pattern of the B-form crystal of the compound represented by Formula (I). In the table, Position represents 2θ (°), and Intensity represents the intensity.

[MODE FOR CARRYING OUT THE INVENTION]

If there is no reference in particular, a numerical value in the present specification and claims is an approximate value. A numerical change originates from a device calibration, a device error, substance purity, a crystal size, a sample size, temperature, and other factors.

Terms used in this description are explained below. Each term, unless otherwise indicated, has the same meaning when it is used alone or together with other terms.

The term of "consisting of" means having only components. The term "comprising" or "containing" means not to limit to the described elements and not to exclude undescribed elements.

Furthermore, it should be understood that, throughout the present specification, the expression of a singular form includes the concept of its plural form unless specified otherwise. Therefore, it should be understood that, unless particularly stated otherwise, an article for a singular form (for example, in the case of English, "a", "an", "the", or the like) also includes the concept of a plural form thereof.

Furthermore, it should be understood that, unless particularly stated otherwise, the terms used in the present specification are used in the meanings normally used in the above-described art. Accordingly, unless otherwise defined, all terminologies and scientific and technical terms used in the present specification have the same meanings as commonly understood by those having ordinary skill in the art to which the present invention belongs. In a case of contradiction, priority is given to the present specification (including definitions).

The present invention relates to a crystal of the compound represented by Formula (I) or (II). The crystal may be either a single-phase crystal or a mixed crystal.

The selection and control of the solid form is important, especially for a compound as a drug. Careful selection and control of the solid form can reduce problems in production, formulation, or administration related with the compound.

The term "crystal" used in the present specification means a solid in which atoms, ions, molecules, and the like constituting the crystal are three-dimensionally and regularly arranged, and is distinguished from an amorphous solid not having such a regular internal structure. The crystal of the present invention may be a single crystal, a twin crystal, a polycrystal, or the like.

Furthermore, the "crystal" may include "crystal polymorphs" that have the same composition but different arrangements in the crystal, and crystals including those crystal polymorphs are referred to as "crystal forms".

The crystal form and the crystallinity can be measured by numerous technologies including, for example, powder X-ray diffraction measurement, Raman spectroscopy, infrared absorption spectrometry, water absorption and desorption measurement, differential scanning calorimetry, and dissolution characteristics.

One aspect of the present invention is an "anhydride crystal of the compound represented by Formula (I) and/or (a/the) hydrate crystal of the compound represented by Formula (I) having a water content corresponding to 0.25 hydrates or less".

The term "anhydride crystal of the compound represented by Formula (I) and/or (a/the) hydrate crystal of the compound represented by Formula (I) having a water content corresponding to 0.25 hydrates or less" used in the present specification means a crystal containing 0 to 0.25 molar equivalents of water molecules (crystal water) per molecule of the compound represented by Formula (I). The "anhydride crystal of the compound represented by Formula (I) and/or (a/the) hydrate crystal of the compound represented by Formula (I) having a water content corresponding to 0.25 hydrates or less" is affected by the external environment (relative humidity and/or temperature). Water molecules in the air may be adsorbed and/or dehydrated, and the adsorption/dehydration occurs reversibly. That is, the "anhydride crystal of the compound represented by Formula (I) and/or (a/the) hydrate crystal of the compound represented by Formula (I) having a water content corresponding to 0.25 hydrates or less" is a crystal in which water molecules in the air easily enter and leave a crystal lattice as crystal water due to external humidity and/or temperature.

The term "crystal water" used in the present specification means water present in the crystal lattice. The term "adhesion water" used in the present specification means water adhering to the crystal surface, and may be referred to as "free water". It is known that crystal water and adhesion water have different contributions to the stability and solubility of pharmaceutical products.

When the "anhydride crystal of the compound represented by Formula (I) and/or the hydrate crystal having a water content corresponding to 0.25 hydrates or less" is identified in a powder X-ray diffraction pattern (CuK$\alpha$ radiation), the crystal has characteristic peaks at diffraction angles (2$\theta$): 13.05° ± 0.25°, 18.35° ± 0.25°, 20.05° ± 0.35°, 20.45° ± 0.25°, and 23.75° ± 0.25°.

When the crystal is identified in more peaks,
the crystal has characteristic peaks at diffraction angles (2$\theta$): 13.05° ± 0.25°, 15.55° ± 0.25°, 16.65° ± 0.25°, 17.00° ± 0.30°, 18.35° ± 0.25°, 19.35° ± 0.35°, 20.05° ± 0.35°, 20.45° ± 0.25°, 21.45° ± 0.25°, and 23.75° ± 0.25°.

The term "hydrate" used in the present specification refers to, for example, one that is regularly arranged with an arbitrary number of water molecules with respect to the compound represented by Formula (I) or (II).

The term "hydrate crystal" used in the present specification refers to, for example, a crystal that is regularly arranged with an arbitrary number of water molecules with respect to the compound represented by Formula (I) or (II). Water present in the crystal lattice of the hydrate crystal is referred to as "crystal water".

The hydrate crystal of the crystal of "the compound represented by Formula (I)" of the present invention is preferably a "hydrate crystal of the compound represented by Formula (I) having a water content corresponding to 0.25 hydrates or less".

One aspect of the present invention is a "hydrate crystal of the compound represented by Formula (I) having a water content corresponding to 0.25 hydrates or less".

The "hydrate crystal of the compound represented by Formula (I) having a water content corresponding to 0.25 hydrates or less" is a "0.25 hydrate crystal of the compound represented by Formula (I)" and/or a "hydrate crystal of the compound represented by Formula (I) having a water content corresponding to less than 0.25 hydrates".

One aspect of the present invention is a "0.25 hydrate crystal of the compound represented by Formula (I)".

The term "0.25 hydrate crystal of the compound represented by Formula (I)" refers to a crystal containing 0.25 molar equivalents of water molecules per molecule of the compound represented by Formula (I), that is, a crystal in which one molecule of water is regularly arranged per four molecules of the compound represented by Formula (I).

The theoretical content of crystal water of the "0.25 hydrate crystal of the compound represented by Formula (I)" is 0.86w%. However, in the analysis of the water content, a value higher than the theoretical content of crystal water may be obtained under the influence of adhesion water and/or adhesion solvent adhering to the crystal surface. For example, the content of crystal water may be 0.86 to 1.5w%. In addition, a part of the crystal water splashes, and in the analysis of the water content, the crystal water may have a value lower than the theoretical content of crystal water.

When the 0.25 hydrate crystal is identified in the powder X-ray diffraction pattern (CuK$\alpha$ radiation, $\lambda$ = 1.5418 Å), the 0.25 hydrate crystal has characteristic peaks at diffraction angles (2$\theta$): 13.0° ± 0.2°, 18.4° ± 0.2°, 19.9° ± 0.2°, 20.5° ± 0.2°, and 23.7° ± 0.2°.

When the 0.25 hydrate crystal is identified in more peaks,
the 0.25 hydrate crystal has characteristic peaks at diffraction angles (2$\theta$): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2 ± 0.2°.

Further, when a single crystal diffraction experiment is performed using CuK$\alpha$ radiation ($\lambda$ = 1.5418 Å) at 213 K (-60°C) on the 0.25 hydrate crystal,
the 0.25 hydrate crystal is characterized by the following crystallographic data:
Space group: P1

$$a = 11.57\ \text{Å} \pm 0.05\ \text{Å}$$

$$b = 13.58\ \text{Å} \pm 0.05\ \text{Å}$$

$$c = 15.54\ \text{Å} \pm 0.05\ \text{Å}$$

$$\alpha = 92.1° \pm 0.5°$$

$$\beta = 105.3° \pm 0.2°$$

$$\gamma = 92.3° \pm 0.5°.$$

One aspect of the present invention is a "hydrate crystal of the compound represented by Formula (I) having a water content corresponding to less than 0.25 hydrates".

One aspect of the present invention is a "hydrate crystal of the compound represented by Formula (I) (containing X molecules of water molecules per molecule of the compound represented by Formula (I), where $0 < X < 0.25$)".

As used in the present specification, a "hydrate crystal of the compound represented by Formula (I) having a water content corresponding to less than 0.25 hydrates" is synonymous with a "hydrate crystal of the compound represented by Formula (I) (containing X molecules of water molecules per molecule of the compound represented by Formula (I), where $0 < X < 0.25$)".

The terms "hydrate crystal of the compound represented by Formula (I) having a water content corresponding to less than 0.25 hydrates" and "hydrate crystal of the compound represented by Formula (containing X molecules of water molecules per molecule of the compound represented by Formula (I), where $0 < X < 0.25$)" refer to a crystal containing less than 0.25 molar equivalents of water molecules per molecule of the compound represented by Formula (I).

The theoretical content of crystal water in the "hydrate crystal of the compound represented by Formula (I) having a water content corresponding to less than 0.25 hydrates", and the "hydrate crystal of the compound represented by Formula (containing X molecules of water molecules per molecule of the compound represented by Formula (I), where $0 < X < 0.25$)" is less than 0.86w%. However, in the analysis of the water content, a value higher than the theoretical content of crystal water may be obtained under the influence of adhesion water and/or adhesion solvent adhering to the crystal surface. In addition, a part of the crystal water splashes, and in the analysis of the water content, the crystal water may have a value lower than the theoretical content of crystal water.

When the hydrate crystal (hydrate crystal of the compound represented by Formula (I) having a water content corresponding to less than 0.25 hydrates) is identified in the powder X-ray diffraction pattern (CuKα radiation, λ = 1.5418 Å),

the hydrate crystal has characteristic peaks at diffraction angles (2θ): 13.0° ± 0.2°, 18.4° ± 0.2°, 20.0° ± 0.2°, 20.4° ± 0.2°, and 23.7° ± 0.2°.

When the hydrate crystal is identified in more peaks,

the hydrate crystal has characteristic peaks at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2°.

One aspect of the present invention is an "anhydride crystal of the compound represented by Formula (I)".

The term "anhydride" used in the present specification is synonymous with "ansolvate", "non-solvate", "anhydrate", and "non-hydrate".

In the "anhydride crystal of the compound represented by Formula (I)", the theoretical content of crystal water is 0w%. However, in the analysis of the water content, a value higher than the theoretical content of crystal water may be obtained under the influence of adhesion water and/or adhesion solvent adhering to the crystal surface.

When the anhydride crystal is identified in the powder X-ray diffraction pattern (CuKα radiation, λ = 1.5418 Å), the anhydride crystal has characteristic peaks at diffraction angles (2θ): 13.1° ± 0.2°, 18.3° ± 0.2°, 20.2° ± 0.2°, 20.5° ± 0.2°, and 23.8° ± 0.2°.

When the anhydride crystal is identified in more peaks,

the anhydride crystal has characteristic peaks at diffraction angles (2θ): 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2°.

Further, when a single crystal diffraction experiment is performed using CuKα radiation (λ = 1.5418 Å) at 213 K (-60°C) on the anhydride crystal,

the anhydride crystal is characterized by the following crystallographic data:

Space group: $P2_1$

$$a = 11.69\ \text{Å} \pm 0.05\ \text{Å}$$

$$b = 13.36 \text{ Å} \pm 0.05 \text{ Å}$$

$$c = 15.43 \text{ Å} \pm 0.05 \text{ Å}$$

$\alpha = 90°$

$$\beta = 105.3° \pm 0.2°$$

$\gamma = 90°$.

The "crystal of the compound represented by Formula (I)" may be converted to a hydrate by adsorption of water or may be converted to an anhydride by dehydration, depending on a change in relative humidity and/or temperature. That is, the "crystal of the compound represented by Formula (I)" may be a crystal in which water molecules in the air easily enter and leave a crystal lattice as crystal water due to external humidity and/or temperature.

The "anhydride crystal of the compound represented by Formula (I) and/or (a/the) hydrate crystal of the compound represented by Formula (I) having a water content corresponding to 0.25 hydrates or less" may be a crystal in which water molecules in the air easily enter and leave the crystal lattice as crystal water depending on the external relative humidity and/or temperature. That is, the "anhydride crystal of the compound represented by Formula (I)" and the "hydrate crystal of the compound represented by Formula (I) having a water content corresponding to 0.25 hydrates or less" may reversibly change depending on the external relative humidity and/or temperature. Similarly, the "anhydride crystal of the compound represented by Formula (I)", the "hydrate crystal of the compound represented by Formula (I) having a water content corresponding to less than 0.25 hydrates" and the "0.25 hydrate crystal of the compound represented by Formula (I)" may reversibly change depending on the external relative humidity and/or temperature.

The crystal as described above can be interpreted as substantially the same crystal as long as it has the characteristic peaks described in the present specification even when the powder X-ray diffraction pattern slightly changes with the change in water content.

Since the powder X-ray diffraction pattern of the crystal as described above is affected by the environment (relative humidity and/or temperature) in which the powder X-ray diffraction experiment is performed, the powder X-ray diffraction experiment may be performed under a specific environment in which the relative humidity and/or temperature is controlled.

One aspect of the present invention is a crystal represented by Formula (I) having characteristic peaks at diffraction angles (2θ): 13.0° ± 0.2°, 18.4° ± 0.2°, 19.9° ± 0.2°, 20.5° ± 0.2°, and 23.7° ± 0.2° when the crystal is allowed to stand under the conditions of a temperature of 25°C and a relative humidity of 60% on a powder X-ray diffractometer, and then the powder X-ray diffraction experiment (CuKα radiation, λ = 1.5418 Å) is performed under the same environment.

One aspect of the present invention is a crystal represented by Formula (I) having characteristic peaks at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2 ± 0.2° when the crystal is allowed to stand under the conditions of a temperature of 25°C and a relative humidity of 60% on a powder X-ray diffractometer, and then the powder X-ray diffraction experiment (CuKα radiation, λ = 1.5418 Å) is performed under the same environment.

Before the powder X-ray diffraction experiment, the time for allowing the crystal to stand under the control of relative humidity and temperature is, for example, 1.5 hours or more, more preferably 3 hours or more, and most preferably 20 hours or more.

One aspect of the present invention is a crystal represented by Formula (I) having characteristic peaks at diffraction angles (2θ): 13.0° ± 0.2°, 18.4° ± 0.2°, 20.0° ± 0.2°, 20.4° ± 0.2°, and 23.7° ± 0.2° when the crystal is allowed to stand under the conditions of a temperature of 25°C and a relative humidity of 35% on a powder X-ray diffractometer, and then the powder X-ray diffraction experiment (CuKα radiation, λ = 1.5418 Å) is performed under the same environment.

One aspect of the present invention is a crystal represented by Formula (I) having characteristic peaks at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2° when the crystal is allowed to stand under the conditions of a temperature of 25°C and a relative humidity of 35% on a powder X-ray diffractometer, and then the powder X-ray diffraction experiment (CuKα radiation, λ = 1.5418 Å) is performed under the same environment.

Before the powder X-ray diffraction experiment, the time for allowing the crystal to stand under the control of relative humidity and temperature is, for example, 1.5 hours or more, more preferably 3 hours or more, and most preferably 20 hours or more.

One aspect of the present invention is a crystal represented by Formula (I) having characteristic peaks at diffraction angles (2θ): 13.1° ± 0.2°, 18.3° ± 0.2°, 20.2° ± 0.2°, 20.5° ± 0.2°, and 23.8° ± 0.2° when the crystal is allowed to stand under the conditions of a temperature of 50°C and a relative humidity of 25% on a powder X-ray diffractometer, and then the powder X-ray diffraction experiment (CuKα radiation, λ = 1.5418 Å) is performed under the same environment.

One aspect of the present invention is a crystal represented by Formula (I) having characteristic peaks at 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2° when the crystal is allowed to stand under the conditions of a temperature of 50°C and a relative humidity of 25% on a powder X-ray diffractometer, and then the powder X-ray diffraction experiment (CuKα radiation) is performed under the same environment.

Before the powder X-ray diffraction experiment, the time for allowing the crystal to stand under the control of relative humidity and temperature is, for example, 1.5 hours or more, more preferably 3 hours or more, and most preferably 20 hours or more.

One aspect of the present invention is an "anhydride crystal of the compound represented by Formula (I) and/or (a/the) hydrate crystal of the compound represented by Formula (I) having a water content corresponding to 0.25 hydrates or less" having a melting point of 236 to 237°C.

One aspect of the present invention is an "anhydride crystal of the compound represented by Formula (I) and/or (a/the) hydrate crystal of the compound represented by Formula (I) having a water content corresponding to 0.25 hydrates or less" having a melting point of 236 to 237°C as measured by differential scanning calorimetry (DSC) or thermogravimetry/differential thermal analysis (TG/DTA).

One aspect of the present invention is a "hydrate crystal of the compound represented by Formula (I), in which water content Y (w%) is $0.0 < Y \leq 5.0$w%". A "hydrate crystal of the compound represented by Formula (I), in which water content Y (w%) is $0.0 < Y \leq 3.0$w%" is preferable, and a "hydrate crystal of the compound represented by Formula (I), in which water content Y (w%) is $0.0 < Y \leq 1.5$w%" is more preferable.

One aspect of the present invention is an "anhydride crystal of the compound represented by Formula (I) and/or (a/the) hydrate crystal of the compound represented by Formula (I) having a water content corresponding to 0.25 hydrates or less" having absorption peaks at 1448 $cm^{-1}$ ± 2 $cm^{-1}$, 1511 $cm^{-1}$ ± 2 $cm^{-1}$, and 2885 $cm^{-1}$ ± 2 $cm^{-1}$ in Raman spectrum.

One aspect of the present invention is an "anhydride crystal of the compound represented by Formula (I) and/or (a/the) hydrate crystal of the compound represented by Formula (I) having a water content corresponding to 0.25 hydrates or less" having absorption peaks at 1448 $cm^{-1}$ ± 2 $cm^{-1}$, 1511 $cm^{-1}$ ± 2 $cm^{-1}$, 1650 $cm^{-1}$ ± 2 $cm^{-1}$, 2885 $cm^{-1}$ ± 2 $cm^{-1}$, and 2947$cm^{-1}$ ± 2$cm^{-1}$ in Raman spectrum.

One aspect of the present invention is a crystal of the compound represented by Formula (II).

When the crystal is identified in the powder X-ray diffraction pattern (CuKα radiation, λ = 1.5418 Å),
the crystal has characteristic peaks at diffraction angles (2θ): 16.3° ± 0.2°, 18.4° ± 0.2°, 20.4° ± 0.2°, 22.8° ± 0.2°, and 23.9° ± 0.2°.

When the anhydride crystal is identified in more peaks,
the crystal has characteristic peaks at diffraction angles (2θ): 15.1° ± 0.2°, 16.3° ± 0.2°, 18.4° ± 0.2°, 19.2° ± 0.2°, 20.4° ± 0.2°, 21.1° ± 0.2°, 22.0° ± 0.2°, 22.8° ± 0.2°, 23.3° ± 0.2°, and 23.9° ± 0.2°.

One aspect of the present invention is a crystal of the compound represented by Formula (II) having absorption peaks at 577 $cm^{-1}$ ± 2 $cm^{-1}$, 612 $cm^{-1}$ ± 2 $cm^{-1}$, 740 $cm^{-1}$ ± 2 $cm^{-1}$, 1571 $cm^{-1}$ ± 2 $cm^{-1}$, and 1718 $cm^{-1}$ ± 2 $cm^{-1}$ in Raman spectrum.

One aspect of the present invention is an anhydride crystal of the compound represented by Formula (II).

The compound represented by Formula (I) or (II) may take the form of a solvate crystal. The solvate at that time refers to, for example, one that is regularly arranged with an arbitrary number of solvent molecules with respect to the compound represented by Formula (I) or (II). The solvate is preferably a hydrate crystal.

Examples of the solvent molecule other than water include acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutyl ketone, methylcyclohexane, N-methylpyrrolidone, nitromethane, pyridine, sulfolane, tetralin, toluene, 1,1,2-trichloroethene, xylene, acetic acid, anisole, 1-butanol, 2-butanol, t-butanol, n-butyl acetate, t-butyl methyl ether, cumene, dimethyl sulfoxide, ethyl acetate, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, methanol, ethanol, acetone, 1,1-diethoxypropane, 1,1-dimethoxymethane, 2,2-dimethoxypropane, isooctane, isopropyl ether, methyl isopropyl ketone, methyl tetrahydrofuran, petroleum ether, trichloroacetic acid, and trifluoroacetic acid.

Preferred examples include acetic acid, anisole, 1-butanol, 2-butanol, n-butyl acetate, t-butyl methyl ether, cumene, dimethyl sulfoxide, ethyl acetate, diethyl ether, ethyl formate, formic acid, heptane, isobutyl acetate, isopropyl acetate, methyl acetate, 3-methyl-1 butanol, methyl ethyl ketone, methyl isobutyl ketone, 2-methyl-1-propanol, pentane, 1-pentanol, 1-propanol, 2-propanol, propyl acetate, tetrahydrofuran, methanol, ethanol, acetone, 1,1-diethoxypropane, 1,1-dimethoxymethane, 2,2-dimethoxypropane, isooctane, isopropyl ether, methyl isopropyl ketone, methyl tetrahydrofuran, petroleum ether, trichloroacetic acid and trifluoroacetic acid.

More preferred examples include methanol, ethanol, acetone, 1,1-diethoxypropane, 1,1-dimethoxymethane, 2,2-dimethoxypropane, isooctane, isopropyl ether, methyl isopropyl ketone, methyl tetrahydrofuran, petroleum ether,

trichloroacetic acid, and trifluoroacetic acid.

The crystal of the present invention may be a deuterium conversion product. The crystalline form of the present invention may also be labeled with radioisotopes (for example, $^{2}H$, $^{3}H$, $^{14}C$, $^{35}S$, and $^{125}I$).

A radiolabeled compound of the compound represented by Formula (I) or (II) can be prepared using well-known methods in the art. For example, a tritium-labeled compound represented by Formula (I) can be prepared by introducing a tritium to a certain compound represented by Formula (I), through a catalytic dehalogenation reaction using a tritium. This method includes reacting a suitably halogen-substituted precursor of the compound represented by Formula (I) with tritium gas in the presence of a suitable catalyst such as Pd/C, in the presence or absence of a base. The other appropriate method of preparing a tritium-labeled compound can be referred to "Isotopes in the Physical and Biomedical Sciences, Vol. 1, Labeled Compounds (Part A), Chapter 6 (1987)". A $^{14}C$-labeled compound can be prepared by using a raw material having $^{14}C$.

A compound represented by Formula:

[Chemical Formula 8]

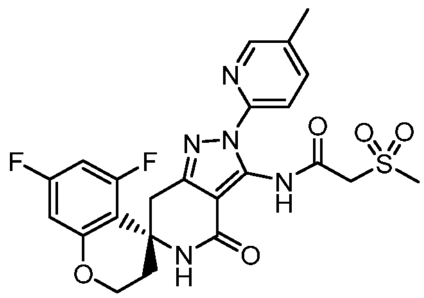

(I)

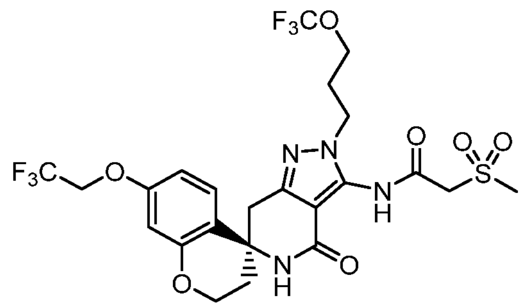

(II)

is a MGAT2 inhibitor described in Patent Document 1 (International Publication WO 2019/013311A) and Patent Document 2 (International Publication WO 2019/013312A). The compound is very useful as a therapeutic agent or a prophylactic agent for a disease involving MGAT2. The compound represented by Formula (I) or (II) can be prepared according to the methods described in Patent Documents 1 and 2, and the like.

(Powder X-ray diffraction (XRPD))

The X-ray powder diffraction (XRPD) is one of the most sensitive analytical methods for measuring the crystalline form and crystallinity of solid. When X-rays are irradiated on a crystal, the rays are reflected at crystal lattice planes, interfere with each other, and show well-ordered diffraction lines corresponding to the period of the structure. On the other hand, in an amorphous solid, a diffraction phenomenon does not occur because an amorphous solid usually does not have a well-ordered repetitive period in the structure, and an uncharacterized broad XRPD pattern (also called halo pattern) is exhibited.

Characteristic diffraction peaks used in the present specification are peaks selected from an observed diffraction pattern. The characteristic diffraction peaks are preferably selected from about 10 peaks, more preferably about 5 peaks, in a diffraction pattern.

When distinguishing a plurality of crystals, a peak that is identified in the relevant crystal and is not identified in other crystals becomes a characteristic peak preferable for characterizing the crystal, rather than the intensity of a peak. With such characteristic peaks, even one or two peaks can characterize the crystal. When the patterns obtained by measurement are compared and these characteristic peaks are found to coincide, it can be said that the powder X-ray diffraction patterns substantially coincide.

In general, since the diffraction angle (2θ) in powder X-ray diffraction may have an error within a range of $\pm$ 0.2°, the value of the diffraction angle of powder X-ray diffraction needs to be understood as including a numerical value within a range of about $\pm$ 0.2°. Therefore, not only crystals in which the diffraction angles of peaks in powder X-ray diffraction perfectly coincide, but also crystals in which the diffraction angles of peaks coincide with an error of about $\pm$ 0.2°, are included in the present invention.

It is known that generally, the intensity of a peak indicated in the following drawings may fluctuate due to many factors, for example, the effect of selective orientation of crystals with respect to an X-ray beam, the influence of coarse particles, the purity of the substance to be analyzed, or the crystallinity of a sample. The peak position can also be shifted based on the height variation of the sample. In addition, when the peak position is measured using different wavelengths, different shifts can be obtained according to Bragg equation ($n\lambda = 2d\sin\theta$); however, other XRPD patterns obtainable by using such other wavelengths are also included in the scope of the present invention.

The crystalline form of the compound represented by Formula (II) can be identified by the powder X-ray diffraction pattern and characteristic diffraction peaks. The crystalline form of the compound represented by Formula (II) can be distinguished from the other crystalline form by the presence of characteristic diffraction peaks.

The crystal of the compound represented by Formula (I) can be identified by the powder X-ray diffraction pattern and characteristic diffraction peaks.

Incidentally, in the "anhydride crystal of the compound represented by Formula (I) and/or (a/the) hydrate crystal of the compound represented by Formula (I) having a water content corresponding to 0.25 hydrates or less", water molecules in the air easily enter and leave the crystal lattice as crystal water, and the crystal reversibly change to an anhydride crystal and a hydrate crystal having a water content corresponding to 0.25 hydrates or less, depending on the external relative humidity and/or temperature. That is, in the "anhydride crystal of the compound represented by Formula (I) and/or (a/the) hydrate crystal of the compound represented by Formula (I) having a water content corresponding to 0.25 hydrates or less", water molecules in the air easily enter and leave the crystal lattice as crystal water, and the crystal reversibly change to an anhydride crystal, a hydrate crystal having a water content corresponding to less than 0.25 hydrates, and a 0.25 hydrate crystal, depending on the external relative humidity and/or temperature.

Since this change is only insertion and extraction of water molecules into and from the crystal lattice, "the anhydride crystal of the compound represented by Formula (I) and the hydrate crystal of the compound represented by Formula (I) having a water content corresponding to 0.25 hydrates or less" shows substantially the same diffraction angles ($2\theta$), but there are also diffraction angles ($2\theta$) characteristic of each of (A1) 0.25 hydrate crystal, (A2) anhydride crystal, and the hydrate crystal existing between (A1) and (A2) (containing X molecules of water molecules per molecule of the compound represented by Formula (I), where $0 < X < 0.25$).

By combining a powder X-ray diffractometer and a temperature and/or relative humidity control device, powder X-ray diffraction measurement under a specific temperature and/or relative humidity can be performed.

When an anhydride crystal changes to a hydrate crystal due to moisture adsorption, when a hydrate crystal changes to an anhydride crystal due to dehydration, and when an anhydride crystal and a hydrate crystal reversibly change due to moisture adsorption and dehydration, measurement under a specific temperature and/or relative humidity, and measurement in which a temperature and/or relative humidity are continuously changed are suitable. In such a case, it is possible to infer the relative humidity and the amount of crystal water at which changes occur.

Since the powder X-ray diffraction pattern of "the anhydride crystal of the compound represented by Formula (I) and the hydrate crystal of the compound represented by Formula (I) having a water content corresponding to 0.25 hydrates or less" is affected by an environment (relative humidity and/or temperature) in which the powder X-ray diffraction experiment is performed, the powder X-ray diffraction experiment may be performed under a specific environment in which the relative humidity and/or temperature is controlled. Also, when the crystal form of the crystal is accurately identified by powder X-ray diffraction, the crystal form is preferably measured under the specific relative humidity and/or temperature environment.

(Powder X-Ray Diffraction-Differential Scanning Calorimetry (XRD-DSC))

In the simultaneous measurement of powder X-ray diffraction and differential scanning calorimetry (XRD-DSC measurement), a change in crystal form (crystal structure) and a change in heat quantity with respect to temperature and relative humidity can be simultaneously observed.

It is suitable for the measurement when an anhydride crystal undergoes crystal transition to a hydrate crystal due to moisture adsorption, when a hydrate crystal undergoes crystal transition to an anhydride crystal due to dehydration, and when an anhydride crystal and a hydrate crystal reversibly undergo crystal transition due to moisture adsorption and dehydration.

(Single crystal structure analysis)

In one of the methods for characterizing a crystal, crystallographic parameters for the relevant crystal, as well as the atomic coordinates (values indicating the spatial positional relationship of each atom) and a three-dimensional structural model can be obtained. See "Guidance on X-ray Structural Analysis", written by Toshio Sakurai, published by Shokabo Co., Ltd. (1983); X-Ray Structure Determination: A Practical Guide, written by Stout & Jensen, Macmillan Co, New York (1968); and the like. The single crystal structural analysis is useful to identify the structures of an optical isomer, a tautomer, a geometric isomer, and a solvate (hydrate).

In the single crystal X-ray diffraction experiment, it is known that the quality of data is improved by performing measurement at a low temperature. In the single crystal X-ray diffraction experiment of hydrate and/or solvate, it is known that the quality of data is improved by coating crystals with paraffin oil or the like before measurement.

(Raman spectroscopy)

Raman spectroscopy shows the characteristics of the oscillation of a molecular or composite system. Its origin lies in the inelastic collision between molecules and photons, which are particles of light including light rays. Collision between molecules and photons results in the exchange of energy, which results in a change in energy, and thereby the wavelength of the photons changes. That is, since the Raman spectrum is a spectral line of very narrow wavelengths, which is emitted when photons are incident on a molecule of interest, lasers and the like are used as light sources. The wavelength of each Raman line is indicated by the wavenumber shift from incident light, and this is the difference between the Raman line and the reciprocal of the wavelength of incident light. Raman spectroscopy is to measure the state of oscillation of molecules, and this is determined by the molecular structure thereof.

In general, since an absorption band ($cm^{-1}$) in a Raman spectrum may have an error within a range of ± $2cm^{-1}$, the value of the absorption peak should be understood as including a numerical value within a range of about ± $2cm^{-1}$. Therefore, not only the crystals whose Raman spectral peaks in the Raman spectra perfectly coincide, but also the crystals whose Raman spectral peaks coincide with errors of about ± $2cm^{-1}$, are included in the present invention.

(Infrared absorption spectroscopy (IR method))

The infrared absorption spectroscopy is a method for measuring, for each wavenumber, a degree of absorption of infrared rays when the infrared rays pass through a sample. The infrared absorption spectrum is typically represented by a graph in which the axis of abscissa represents a wavenumber and the axis of ordinate represents a transmittance or an absorbance. The wavenumber and transmittance (or absorbance) of the absorption peak can be read on a graph, and values calculated by a data processing device can be used. The infrared absorption spectrum is determined by the chemical structure of the substance. Therefore, absorption at various wavenumbers can be measured to confirm or quantify a substance. The discrimination of a crystal polymorph can be performed by comparing absorption bands of functional groups characteristic of crystal polymorphs, that is, a functional group mainly involved in a hydrogen bond in the crystal structure such as a C = O bond, an OH bond, and an NH bond, as well as other characteristic functional groups such as a C-X (halogen) bond, a C = C bond, and a C≡C bond. The absorption bands for characteristic functional group are selected from about 20 absorption peaks, more preferably about 10 absorption peaks, and most preferably about 5 absorption peaks corresponding to the characteristic functional groups. Typically, an absorption spectrum of a sample is measured in a wavenumber range of $4000cm^{-1}$ to $400cm^{-1}$. The absorption spectrum is measured under the same operating conditions as those when the resolution, the wavenumber scale, and the wavenumber accuracy of the apparatus were confirmed.

In general, since an absorption band ($cm^{-1}$) in infrared absorption spectroscopy may have an error within a range of ± $2cm^{-1}$, the value of the absorption peak should be understood as including a numerical value within a range of about ± $2cm^{-1}$. Therefore, the present invention encompasses not only crystals in which the peaks in the absorption bands in the infrared absorption spectroscopy completely coincide with each other, but also crystals in which the peaks in the absorption bands coincide with each other with an error of about ± $2cm^{-1}$.

Examples of the method for measuring an infrared absorption spectrum include the potassium bromide tablet method, the solution method, the paste method, a liquid film method, the thin film method, the gas sample measurement method, the ATR method, and the diffuse reflection method. Among them, the attenuated total reflection (ATR) method is called the total reflection measurement method and is one of the reflection methods. In this method, a sample is brought into close contact with a surface of a prism made of a substance having a high refractive index such as KRS-5, light is incident on the prism at an angle equal to or larger than a critical angle, and light totally reflected at a boundary between the prism and the sample is measured to obtain an absorption spectrum. One of the conditions enabling the measurement by the ATR method is that the refractive index of the prism is larger than that of the sample, and thus it is necessary to change the material of the prism depending on the sample. In addition, as another condition, the prism and the sample must be in close contact with each other. Therefore, it is suitable for measurement of liquid, powder, plastic, soft rubber, and the like, and there is an advantage that measurement can be performed without chemically or physically treating the sample. On the other hand, the diffuse reflection method is a method of measuring a powder sample as it is without forming a potassium bromide tablet. When light is applied to a sample, light that is specularly reflected on the powder surface and exits to the outside and diffusely reflected light (scattered light) that enters the sample, repeats transmission and diffusion, and then exits to the surface are generated. In the diffuse reflection method, the latter is used to obtain an absorption spectrum.

(Solid $^{13}C$-NMR (nuclear magnetic resonance))

Solid state $^{13}C$-NMR is useful for specifying a crystal form because (i) the number of spectra coincides with the number of carbon atoms of a target compound, (ii) the chemical shift range is wider than that of $^{1}H$-NMR, (iii) a signal is sharper than that of solid state $^{1}H$-NMR, and (iv) even if an additive is contained, the chemical shift does not change when

there is no interaction. Note that an observed chemical shift is expected to vary slightly depending on the particular spectrometer used and the analyst's sample preparation technique. The error range in the solid $^{13}$C-NMR spectrum is approximately ± 0.5 ppm.

(Differential scanning calorimetry (DSC))

DSC is one of important measurement methods for thermal analysis and is a method of measuring the thermal properties of a substance as an aggregate of atoms and molecules.

A differential scanning calorimetric curve is obtained by measuring the temperature-related or time-related change in the calorific value of a pharmaceutically active ingredient by DSC and plotting the obtained data with respect to temperature or time. From the differential scanning calorimetric curve, information on the onset temperature (extrapolated melting point start temperature) at the time of melting of the pharmaceutically active ingredient, the maximum value of the endothermic peak curve associated with melting, and the enthalpy can be obtained.

With regard to DSC, it is known that the temperature to be observed may depend on the rate of temperature change as well as the sample preparation technique and the particular apparatus used. Therefore, the "melting point" in DSC refers to the onset temperature (extrapolated melting point start temperature) that is not likely to be affected by the sample preparation technique. The error range for the onset temperature (extrapolated melting point start temperature) obtainable from the differential scanning calorimetry curve is approximately ± 2°C. For the recognition of identity of crystals, not only the melting point but also the overall pattern are important, and there may be some variation depending on the measurement conditions and the measuring equipment.

(Thermogravimetry/differential thermal analysis (TG/DTA))

TG/DTA is one of important measurement methods for thermal analysis and is a method of measuring the weight and thermal properties of a substance as an aggregate of atoms and molecules.

TG/DTA is a method of measuring the temperature-related or time-related changes in weight and calorific value of a pharmaceutically active ingredient, and a TG (thermogravimetric) and DTA (differential thermal) curve is obtained by plotting obtained data with respect to temperature or time. From the TG/DTA curve, information on the changes in weight and calorific value in relation to degradation, dehydration, oxidation, reduction, sublimation, and evaporation of a pharmaceutically active ingredient can be obtained.

With regard to TG/DTA, it is known that the temperature to be observed and the weight change may depend on the rate of temperature change as well as the sample preparation technique and the particular apparatus used. Therefore, the "melting point" in TG/DTA refers to the onset temperature (extrapolated melting point start temperature) that is not likely to be affected by the sample preparation technique. For the recognition of identity of crystals, not only the melting point but also the overall pattern are important, and there may be some variation depending on the measurement conditions and the measuring equipment.

(Moisture adsorption/desorption isotherm measurement method (DVS))

The moisture adsorption/desorption isotherm measurement is a measurement method for measuring the adsorption and desorption behavior of moisture by measuring a weight change in a solid as a measurement target under each relative humidity condition.

As a basic measurement method, based on the dry weight at 0% RH (relative humidity 0%), the relative humidity is increased every 5% or 10%, and after the weight is stabilized at each relative humidity, the amount of adsorbed water can be determined from the weight increase from the reference value. Similarly, the desorption amount of water can be measured by decreasing the relative humidity every 5% or 10% from 100% RH or 95% RH.

By plotting the value of the weight change at each relative humidity, an adsorption/desorption isotherm can be obtained. From this result, it is possible to consider a phenomenon of adsorption and desorption of adhering moisture at each humidity.

Sorption and desorption of adhesion water and crystal water are affected by particle size, crystallinity, crystal habit, and the like, so that the measurement results may slightly change.

The differential scanning calorimetry (DSC), the thermogravimetry/differential thermal analysis (TG/DTA), the moisture adsorption/desorption isotherm measurement method (DVS), Karl Fischer moisture meter, and gas chromatography are analysis methods for detecting adhesion water and/or adhesion solvent (residual solvent) on the "surface". In this analysis, when a sample in which adhesion water and/or adhesion solvent is present on the crystal surface is measured, the sample may show a higher water content and/or solvent content than the theoretical content of crystal water in the hydrate crystal and/or the theoretical content of crystal solvent in the solvate crystal.

On the other hand, the powder X-ray diffraction and the single crystal structure analysis are measurement

methods for analyzing the "internal structure" of a crystal, and show characteristic peaks at the same position regardless of the presence or absence of adhesion water and/or adhesion solvent (residual solvent) on the crystal "surface". The same applies to Raman spectroscopy and infrared absorption spectrometry (IR method). Therefore, in the powder X-ray diffraction, the single crystal structure analysis, the Raman spectroscopy, and the infrared absorption spectrometry (IR method), the crystal can be interpreted as substantially the same crystal as long as it has the characteristic peaks described in the specification even when a higher water content and/or solvent content than the theoretical content in the crystal is shown.

As described above, the powder X-ray diffraction experiment or the single crystal structure analysis experiment may be performed in a specific environment in which the relative humidity and/or temperature is controlled, but the control environment of the experiment and the result of the analysis method for detecting adhesion water and/or adhesion solvent may not match. For example, it is a value of relative humidity to be controlled at the time of performing a powder X-ray diffraction experiment and a value of relative humidity in a moisture adsorption/desorption isotherm measurement method (DVS).

The pharmaceutical composition containing a crystal of the present invention is very useful as a therapeutic agent or a prophylactic agent for a disease involving MGAT2. Examples of the disease involving MGAT2 include obesity, metabolic syndrome, hyperlipidemia, hypertriglyceridemia, hyper-VLDL-triglyceridemia, hyperfattyacidemia, diabetes mellitus, and/or arteriosclerosis.

The crystal of the present invention can be administered to a human patient by itself, or can be administered as a pharmaceutical composition in which the crystal is mixed with an appropriate carrier or excipient. Techniques for drug formulation and administration can be appropriately selected and used in combination with pharmaceutical formulations and techniques known to those skilled in the art.

Examples of administration route of the crystal of the present invention or the pharmaceutical composition containing the crystal can include, but is not limited to, oral, rectal, transmucosal, or intestinal administration, or intramuscular, subcutaneous, intraspinal, intrathecal, direct intraventricular, intravenous, intravitreal, intraperitoneal, intranasal, and intraocular, injection. A preferred route of administration is oral administration.

The pharmaceutical composition of the present invention can be produced by a method well known in the art, for example, a process of conventional mixing, dissolving, granulating, sugar-coating, powdering, emulsifying, encapsulating, enclosing, or lyophilizing.

The crystal of the present invention or the pharmaceutical composition containing the crystal can be administered by injection using an aqueous solution, preferably a physiologically compatible buffer such as Ringer's solution or physiological saline.

The crystal of the present invention or the pharmaceutical composition containing the crystal can be administered transmucosally using a penetrant suitable for a barrier to be permeated. As the penetrant, one generally known in the art can be used.

The crystal of the present invention or the pharmaceutical composition containing the crystal may be combined with a pharmaceutically acceptable carrier well known in the art so as to be orally administered. The carrier allows the crystal of the present invention to be administered as tablets, pills, lozenges, sugar-coated tablet, capsules, solution, gel, syrups, or suspensions. Pharmaceutical compositions for oral administration can be made by adding solid excipients and, if desired, other suitable auxiliaries, followed by grinding the resulting mixture and processing the mixture of granules to obtain tablets or sugar-coated tablet cores.

For the crystals of the present invention or pharmaceutical compositions thereof, a therapeutically effective amount can first be estimated from enzyme assays. Then, a dosage of a large amount can then be formulated for use in animal models to achieve a circulating concentration range that covers IC 50 (that is, a concentration of the crystal of the present invention or pharmaceutical composition thereof with which half maximal inhibition of PK activity is achieved) as determined in the enzyme assays. Such information can then be used to more accurately determine a useful amount of the same in humans.

Therapeutic effects of the crystal of the present invention or the pharmaceutical composition thereof can be measured by a standard pharmaceutical method in purified enzymes or experimental animals. For example, evaluation may be performed according to a biological test method described in Patent Document 1. The data obtained from these enzyme assays and animal experiments can be used to formulate a range of dosages for use in humans. The dosage can be varied depending on the form of administration used and the route of administration utilized. The exact administration route of formulation and dosage can be selected by the individual physician in view of the patient's condition.

It is also an aspect of the present invention that the crystals of the present invention or pharmaceutical compositions thereof can be combined with other agents for the treatment of diseases and disorders.

The present invention provides an anhydride crystal and a hydrate crystal of the compound represented by Formula (I) and a crystal of the compound represented by Formula (II). The crystal has at least one of the following characteristics:

(1) having good stability against heat, humidity, solvent, light and the like, and high storage stability;
(2) showing no significant coloration after light irradiation;
(3) having good solubility in water or organic solvents;
(4) having a high dissolution rate with respect to water or organic solvents;
(5) having high purity;
(6) having a low rate of residual organic solvent;
(7) having excellent operability in filtration, centrifugation, and formulation;
(8) having a small specific volume;
(9) being hardly charged;
(10) being produced at a high yield under conditions with reduced loads on the environment, and being able to be mass-produced;
(11) being useful as a pharmaceutically active ingredient for an oral preparation or an injection, or an active material for the production thereof;
(12) being controllable to a pH range suitable for intravenous injection without vascular pain, thereby being advantageous for liquid amount control, reduction of excipients, etc. at the time of formulation;
(13) having good fluidity; and
(14) having a low compressibility index (%).

In particular, the crystal of the present invention has high stability even in a wide humidity range (for example, 25 to 95% RH or the like) and a severe environment (for example, under high humidity), and has good solubility in water or an organic solvent.

[EXAMPLES]

The present invention will be described in more detail by the following examples. These do not limit the present invention. For numerical values (for example, the amount, temperature, and the like), some error and deviation should be considered.

Unless otherwise noted, "%" means w% by the component and w% by the total weight of the composition, and "pressure" means a pressure at or near atmospheric pressure.

(Measurement of powder X-ray diffraction pattern)

Powder X-ray diffraction measurement was performed in accordance with the powder X-ray diffraction measuring method described in General Tests of Japanese Pharmacopoeia. Measurement conditions are shown below.

Measurement conditions 1:

Powder X-ray diffractometer: SmartLab manufactured by Rigaku Corporation Measurement method: Reflection method
Wavelength used: CuKα radiation ($\lambda$ = 1.5418 Å)
Tube current: 200 mA
Tube voltage: 45 kV
Sample plate: Aluminum
Incident angle of X-rays: 2.5°
Sampling width: 0.02°
Detector: HyPix-3000 (two-dimensional detection mode)

Measurement conditions 2:

D-8 Discover manufactured by Bruker Corporation (Operation method)
Measurement method: Reflection method
Wavelength used: CuKα radiation ($\lambda$ = 1.5418 Å)
Tube current: 40 mA
Tube voltage: 40 kV

Sample plate: Aluminum
Incident angle of X-ray: 3° and 12°

(Measurement of powder X-ray diffraction pattern under control of temperature and relative humidity)

The temperature and relative humidity of a measurement sample section were controlled using an attachment attached to an X-ray diffractometer, and powder X-ray diffraction measurement was performed according to the powder X-ray diffraction measurement method described in General Tests, Processes and Apparatus of the Japanese Pharmacopoeia. Measurement conditions are shown below.

Measurement conditions 3:

(X-Ray diffraction measurement)

Powder X-ray diffractometer: RINT2100Ultima+ manufactured by Rigaku Corporation
Measurement method: Reflection method
Wavelength used: CuKα radiation (λ = 1.5418 Å)
Tube current: 40 mA
Tube voltage: 40 kV
Sample plate: Aluminum
Measurement range of X-ray: 5° to 35°
Sampling width: 0.02°
Scan speed: 60°/min

(Control of temperature and relative humidity)

Temperature controller: ThermoPlus manufactured by Rigaku Corporation
Control temperature: 25°C
Humidity controller: HUM-1 manufactured by Rigaku Corporation
Control humidity: relative humidity 60%

Measurement conditions 4:

The controller described in Measurement conditions 3 was set to the following conditions, and X-ray diffraction measurement under Measurement conditions 3 was performed.

Control temperature: 25°C
Control humidity: relative humidity 35%

Measurement conditions 5:

The controller described in Measurement conditions 3 was set to the following conditions, and X-ray diffraction measurement under Measurement conditions 3 was performed.

Control temperature: 50°C
Control humidity: relative humidity 25%

Measurement conditions 6:

The controller described in Measurement conditions 3 was set to the following conditions, and X-ray diffraction measurement under Measurement conditions 3 was performed.
Control temperature: The temperature was raised from room temperature to 80°C at a rate of 5°C/min. (No temperature control was performed.)

(Measurement and analysis method of single crystal structure analysis)

Single crystal structure analysis of the crystal obtained in each Example was performed.
The measurement conditions and analysis method are shown below.

(Apparatus)

XtaLAB P200 MM007 manufactured by Rigaku Corporation

(Measurement conditions)

Measurement temperature: -60°C
Temperature controller: sample blowing low temperature equipment manufactured by Rigaku Corporation
Wavelength used: CuKa radiation ($\lambda$ = 1.5418 Å)
Software: CrysAlisPro 1.171.39.46e (Rigaku Oxford Diffraction, 2018)

(Data processing)

Software: CrysAlisPro 1.171.39.46e (Rigaku Oxford Diffraction, 2018)
The data were corrected for the Lorentz, polarization and absorption effects.

(Crystal structure analysis)

Phase determination was performed using a direct method program, ShelXT (Sheldrick, G. M., 2015), and regarding refinement, a full-matrix least squares method was carried out using ShelXL (Sheldrick, G. M., 2015). The temperature factors of non-hydrogen atoms were all subjected to refinement with anisotropy. Hydrogen atoms were computationally introduced using the default parameters of ShelXL and were treated as riding atoms. The hydrogen atoms, excluding water molecules, were subjected to refinement with isotropic parameters.
PLATON (Spek, 1991)/ORTEP (Johnson, 1976) was used to plot Figs. 4 to 11 (30% PROBABILITY level).

(Measurement of Raman spectrum)

The measurement of the crystalline and amorphous Raman spectra obtained in each Example and the measurement conditions under which a baseline correction was performed are shown below.

Measurement conditions 1

Measurement method: Microscopic laser Raman spectrometry
Laser wavelength: 671 nm
Cumulative number: 1 to 10 times
Exposure time: 0.5 to 10 seconds

Measurement conditions 2

Measurement method: Microscopic laser Raman spectrometry
Laser wavelength: 785 nm
Cumulative number: 1 to 10 times
Exposure time: 1 to 10 seconds

(Measurement of differential scanning calorimetry (DSC))

A DSC of a crystal obtained in each Example was measured. A sample was weighed in an aluminum pan, and

measurement was performed by simple sealing. Measurement conditions are shown below. Incidentally, in the measurement made by differential scanning calorimetry (DSC), an error can occur in the range of ± 2°C.

Apparatus: TA Instrument Q2000/TA Instrument
Measurement temperature range: -10°C to 250°C
Rate of temperature increase: 10°C/min
Atmosphere: $N_2$ 50 mL/min

(Measurement of TG/DTA)

The crystals obtained in each Example were weighed and packed into an aluminum pan, and measurement was performed in an open system. Measurement conditions are as follows.

Apparatus: Hitachi High-Technologies TG/DTA STA7200RV
Measurement temperature range: room temperature to 300°C
Rate of temperature increase: 10°C/min

(Moisture adsorption/desorption isotherm measurement method (DVS))

The crystals and amorphous substances obtained in each Example were weighed in an aluminum pan, and allowed to stand at 25°C and a relative humidity of 0%. The measurement was started after the compound was sufficiently dried and the weight was stabilized, and the weight when the relative humidity changed from 0% to 95% by 5% was recorded. Next, the weight when the relative humidity changed from 95% to 0% by 5% was recorded.
Device: DVS Advantage manufactured by Surface Measurement Systems

Example 1

An amorphous compound represented by Formula (I) was obtained in the same manner as described in Example 14 of Patent Document 1.
Ethanol (500 μL) was added to the obtained amorphous compound (5 mg) represented by Formula (I), and the mixture was stirred for 5 minutes under heating at 60°C. The mixture was allowed to stand until the temperature returned to room temperature, and the solvent was evaporated to dryness to obtain a crystal.

Example 2

The crystal of the compound represented by Formula (I) obtained in Example 1 was allowed to stand under the conditions of a temperature of 25°C and a relative humidity of 60% on a powder X-ray diffractometer, and then a powder X-ray diffraction experiment was performed under Measurement conditions 3.
As a result, in the powder X-ray diffraction pattern, peaks were observed at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2° ± 0.2°.
In the above-described powder X-ray diffraction pattern, the peaks at the diffraction angles (2θ): 13.0° ± 0.2°, 18.4° ± 0.2°, 19.9° ± 0.2°, 20.5° ± 0.2°, and 23.7° ± 0.2° are particularly characteristic as the "0.25 hydrate crystal of the compound represented by Formula (I)".
The powder X-ray diffraction pattern is shown in Fig. 1, and the peak list of the powder X-ray diffraction pattern is shown in Fig. 19.

Example 3

The crystal (5 mg) of the compound represented by Formula (I) obtained in Example 1 was added to 1 mL of a 5% water-containing acetone solution, and dissolved by heating to 60°C. Thereafter, the solution was allowed to stand, cooled to room temperature, and the solvent was evaporated to obtain a single crystal.
The obtained single crystal was coated with paraffin oil and fixed to MicroMount (MiTeGen). Thereafter, the fixed single crystal was set in a measurement section of a diffractometer set at -60°C and instantaneously cooled, and a single crystal X-ray diffraction experiment was performed.
Analysis was performed by the method described in the above (Measurement and analysis method of single crystal structure analysis). The occupancy of oxygen (labeled as 021) of water molecules was analyzed at 1, and hydrogen was not introduced into O21.

The results of the single crystal structure analysis are shown below.

R1 (I > 2.00 s(I)) was 0.0925, and it was confirmed from the final difference Fourier that there was no lack of electron density or misplacement.

Crystallographic data are shown in Table 1.

[Table 1]

| | |
|---|---|
| Space group | P1 |
| a (Å) | 11.5688(4) |
| b (Å) | 13.5769(4) |
| c (Å) | 15.5429(4) |
| $\alpha$ (°) | 92.077(2) |
| $\beta$ (°) | 105.324(3) |
| $\gamma$ (°) | 92.281(3) |
| V ($Å^3$) | 2349.87(13) |
| Z | 4 |
| Density (calculated) ($g/cm^3$) | 1.474 |
| Measurement temperature (K) | 213 |

Here, V represents a volume of a unit lattice, and Z represents the number of molecules in the unit lattice.

The atomic fraction coordinates x, y, z ($\times 10^4$) and the equivalent isotropic temperature factor U(eq) (Equivalent Isotropic Displacement Parameters, $Å^2 \times 10^3$) of the non-hydrogen atoms are shown in Tables 2 to 3. Here, U(eq) is defined as one third of the trace of the orthogonalized $U_{ij}$ tensor.

[Table 2]

| Atom | x | y | z | U(eq) | Atom | x | y | z | U(eq) |
|---|---|---|---|---|---|---|---|---|---|
| S1 | 5703(2) | 8533.1(18) | 13106.5(16) | 49.4(5) | N5 | 8664(7) | 5761(6) | 13033(5) | 41.4(16) |
| S3 | 4347(2) | 3670.4(18) | -2891.9(16) | 51.7(6) | N9 | 6222(7) | 2287(8) | 2050(5) | 55(2) |
| S2 | 5313(3) | 765(2) | 6465(2) | 68.6(8) | C79 | 1381(7) | 8159(6) | 4420(5) | 34.6(16) |
| S4 | 4901(5) | 5444(3) | 3835(3) | 103.0(16) | C55 | 3346(8) | 1995(6) | 460(6) | 38.0(17) |
| F5 | 2465(7) | 537(4) | 2260(4) | 60.7(16) | F4 | 7423(11) | 367(7) | -1198(5) | 106(3) |
| F3 | 7630(7) | 634(5) | 1830(5) | 71.2(18) | C5 | 7719(8) | 6668(7) | 10411(6) | 40.1(18) |
| O14 | 4405(6) | 1983(5) | 422(4) | 46.1(15) | C42 | 7476(8) | 2090(8) | 1062(6) | 47(2) |
| F1 | 7432(6) | 5485(4) | 7738(4) | 62.4(16) | 012 | 3142(8) | 3382(8) | -3403(6) | 75(2) |
| F8 | 2484(7) | 6134(5) | 8754(4) | 69.6(18) | C33 | 8782(8) | 3175(6) | 5849(5) | 39.5(18) |
| O19 | 5205(5) | 7162(5) | 7258(4) | 45.8(14) | 017 | 5773(8) | 5439(8) | 3341(7) | 81(3) |
| F6 | 1809(7) | 1878(6) | 4896(4) | 71.9(19) | C4 | 7808(8) | 6568(6) | 11315(5) | 36.3(17) |
| O9 | 4901(5) | 2149(6) | 2901(4) | 50.7(16) | C51 | 2326(8) | 1778(6) | -315(5) | 35.4(17) |
| O4 | 5650(6) | 6821(6) | 9677(4) | 53.7(17) | C64 | 1534(9) | 2934(7) | 2768(6) | 46(2) |
| O13 | 3558(8) | 3443(5) | -994(5) | 58.7(19) | C14 | 10680(8) | 6006(6) | 12919(6) | 39.8(18) |
| N17 | 1935(6) | 7946(5) | 5312(5) | 37.6(15) | C9 | 8855(8) | 6425(6) | 10313(6) | 38.0(17) |
| N10 | 8012(7) | 3344(6) | 6328(5) | 46.7(17) | C83 | 1652(8) | 8580(7) | 3075(6) | 41.7(19) |
| N7 | 8214(7) | 2944(6) | 4922(5) | 39.9(15) | C18 | 8761(8) | 7865(8) | 7464(6) | 45(2) |
| O18 | 4053(8) | 5832(5) | 5597(5) | 56.8(18) | C82 | 423(9) | 8614(7) | 2710(6) | 43.2(19) |
| N11 | 3059(7) | 1932(5) | -1692(4) | 39.3(15) | C10 | 9463(8) | 6000(6) | 12581(6) | 39.4(18) |

(continued)

| Atom | x | y | z | U(eq) | Atom | x | y | z | U(eq) |
|---|---|---|---|---|---|---|---|---|---|
| N16 | 3859(6) | 7417(5) | 5167(5) | 37.4(15) | C54 | 1796(7) | 2255(6) | 1286(5) | 37.2(17) |
| N1 | 7001(7) | 6745(5) | 11811(5) | 41.3(16) | C49 | 3715(9) | 2802(7) | -1496(6) | 47(2) |
| N8 | 8884(7) | 3051(6) | 4315(5) | 43.8(17) | C20 | 7879(9) | 6254(8) | 7357(6) | 47(2) |
| N19 | 4011(7) | 7555(6) | 8152(5) | 41.6(16) | C52 | 1191(8) | 1472(7) | -222(5) | 40.1(18) |
| O3 | 6488(8) | 8243(5) | 11259(5) | 61.0(19) | O7 | 6448(9) | 1229(9) | 6927(6) | 84(3) |
| O11 | 5277(8) | 3598(6) | -3333(6) | 65(2) | C13 | 11119(9) | 5785(7) | 13801(6) | 43.4(19) |
| O15 | 1237(10) | 3826(6) | 2448(5) | 73(2) | C41 | 7437(10) | 2511(8) | 243(6) | 52(2) |
| O5 | 9243(7) | 8718(5) | 7887(5) | 59.7(19) | O10 | 7354(13) | 3456(6) | 127(6) | 97(4) |
| N20 | 2132(7) | 8384(6) | 3932(5) | 40.4(16) | C74 | 3212(7) | 7623(7) | 6586(5) | 36.0(17) |
| N6 | 6224(7) | 2417(6) | 5006(5) | 43.1(17) | C87 | 3033(9) | 8522(10) | 9960(7) | 59(3) |
| O1 | 6914(7) | 8486(8) | 13609(6) | 73(2) | C50 | 2226(7) | 1673(6) | -1226(5) | 34.3(16) |
| F7 | 2221(8) | 7061(9) | 11627(5) | 107(4) | C53 | 954(8) | 1457(7) | 671(6) | 42.2(19) |
| O20 | 3402(8) | 9437(6) | 9930(5) | 62.4(19) | C34 | 8507(9) | 3537(7) | 7211(6) | 47(2) |
| O8 | 5805(8) | 832(6) | 4460(6) | 67(2) | C59 | -1134(9) | 781(7) | -3765(6) | 47(2) |
| F2 | 8238(7) | 6821(8) | 5215(4) | 92(3) | C72 | 4305(8) | 6491(6) | 5180(6) | 41.0(18) |
| N2 | 8936(6) | 6257(5) | 11684(5) | 37.1(14) | C66 | 1802(10) | 1967(9) | 4024(6) | 54(3) |
| N15 | 3040(6) | 2152(6) | 1229(4) | 37.8(15) | C3 | 6351(9) | 7568(7) | 11718(6) | 46(2) |
| N4 | 7054(6) | 7004(6) | 8897(5) | 40.9(16) | C35 | 9718(10) | 3608(7) | 7611(6) | 48(2) |
| O2 | 4771(9) | 8396(6) | 13553(7) | 77(3) | C68 | 2100(10) | 1305(7) | 2709(6) | 47(2) |
| N14 | 1303(7) | 825(5) | -3005(5) | 41.3(16) | C88 | 2867(8) | 7795(8) | 9240(6) | 44(2) |
| N12 | 1085(6) | 1306(5) | -1608(4) | 37.8(15) | C7 | 8296(8) | 7188(7) | 8864(6) | 40.5(19) |
| N18 | 1322(6) | 8090(6) | 5953(5) | 41.0(16) | C69 | 1802(8) | 2161(7) | 2265(6) | 40.8(18) |
| C78 | 4237(7) | 7425(7) | 7330(5) | 37.4(17) | C29 | 5938(8) | 2302(7) | 2848(6) | 44(2) |
| N3 | 9578(7) | 6165(6) | 11062(5) | 42.4(16) | C73 | 3088(8) | 7657(7) | 5681(5) | 38.9(18) |
| O6 | 4393(9) | 722(8) | 6951(7) | 79(3) | C56 | 543(8) | 1035(6) | -2522(5) | 37.9(17) |
| N13 | 442(7) | 1174(6) | -987(5) | 41.3(16) | C80 | 143(8) | 8152(7) | 4116(6) | 39.8(18) |

[Table 3]

| Atom | x | y | z | U(eq) | Atom | x | y | z | U(eq) |
|---|---|---|---|---|---|---|---|---|---|
| C57 | 842(10) | 602(7) | -3881(6) | 49(2) | C85 | 3289(10) | 9214(9) | 8376(7) | 53(2) |
| C30 | 7322(8) | 2710(8) | 1859(5) | 48(2) | C89 | 2579(9) | 6850(9) | 9401(6) | 50(2) |
| C28 | 6969(8) | 2539(7) | 3620(6) | 44(2) | C67 | 2112(11) | 1184(8) | 3576(7) | 55(2) |
| C60 | -689(8) | 1001(6) | -2860(6) | 40.7(18) | C11 | 9114(9) | 5533(7) | 13884(6) | 46(2) |
| C12 | 10324(9) | 5562(6) | 14318(6) | 45(2) | C71 | 5179(12) | 6397(9) | 4616(10) | 67(3) |
| C37 | 10009(8) | 3199(7) | 6173(6) | 41.6(19) | C48 | 4713(10) | 2915(8) | -1955(8) | 53(2) |
| C75 | 2101(8) | 7874(7) | 6706(5) | 38.6(17) | C25 | 4751(11) | 1433(10) | 5483(9) | 66(3) |
| C36 | 10483(9) | 3431(7) | 7076(6) | 47(2) | C16 | 8663(12) | 8263(8) | 9204(7) | 59(3) |
| C77 | 3008(7) | 8101(7) | 8331(5) | 40.4(19) | C46 | 7440(11) | 1920(9) | -516(7) | 59(3) |
| C32 | 8119(8) | 2779(7) | 3538(6) | 43(2) | C86 | 4078(11) | 9560(8) | 9287(7) | 58(3) |

(continued)

| Atom | x | y | z | U(eq) | Atom | x | y | z | U(eq) |
|---|---|---|---|---|---|---|---|---|---|
| C76 | 1842(8) | 7843(8) | 7598(6) | 48(2) | C84 | -54(9) | 8870(10) | 1746(6) | 55(3) |
| C43 | 7565(9) | 1086(8) | 1074(6) | 50(2) | C47 | 4378(15) | 4875(10) | -2459(10) | 75(3) |
| C27 | 7063(8) | 2629(7) | 4519(5) | 38.9(18) | C31 | 8438(8) | 2684(9) | 2668(6) | 51(2) |
| C8 | 9097(8) | 6456(8) | 9423(6) | 42.5(19) | C62 | 1382(13) | 3275(8) | 1002(7) | 60(3) |
| C22 | 8282(10) | 6908(11) | 6104(7) | 64(3) | C15 | 10761(11) | 5361(9) | 15281(7) | 62(3) |
| C2 | 5421(9) | 7589(8) | 12241(7) | 50(2) | C44 | 7529(11) | 462(11) | 308(8) | 67(3) |
| C6 | 6738(8) | 6853(7) | 9656(6) | 39.8(18) | C1 | 5500(13) | 9640(9) | 12558(9) | 69(3) |
| C19 | 8323(8) | 7090(7) | 7888(6) | 37.9(17) | C45 | 7469(13) | 941(11) | -459(7) | 72(4) |
| C92 | 2723(10) | 8267(13) | 10735(7) | 71(4) | C61 | -850(13) | 378(9) | -5288(7) | 64(3) |
| C23 | 8726(9) | 7765(10) | 6551(7) | 57(3) | C17 | 9626(13) | 8678(10) | 8878(8) | 70(3) |
| C21 | 7853(10) | 6121(10) | 6464(7) | 60(3) | C90 | 2343(10) | 6567(11) | 10210(7) | 63(3) |
| C81 | -322(8) | 8392(7) | 3231(6) | 41.6(18) | 016 | 3673(10) | 5689(11) | 3251(7) | 106(4) |
| C26 | 5659(10) | 1519(8) | 4929(7) | 50(2) | C63 | 1773(18) | 4042(10) | 1690(9) | 86(5) |
| C58 | -377(9) | 587(7) | -4302(6) | 46(2) | C40 | 7800(20) | 4048(16) | 1001(19) | 133(8) |
| C39 | 7116(12) | 3748(9) | 1613(9) | 66(3) | C24 | 5550(20) | -418(13) | 6122(19) | 131(9) |
| C38 | 10201(13) | 3857(10) | 8611(7) | 68(3) | C70 | 4590(30) | 4359(13) | 4220(20) | 172(14) |
| C91 | 2417(10) | 7311(13) | 10826(6) | 72(4) | O21 | 4770(30) | 5012(19) | 10280(20) | 269(18) |
| C65 | 1524(10) | 2846(8) | 3666(6) | 52(2) | | | | | |

Next, the atomic coordinates x, y, z (× $10^4$) and the isotropic temperature factor U(eq) (Isotropic Displacement Parameters, $Å^2 \times 10^3$) of the hydrogen atoms are shown in Table 4.

[Table 4]

| Atom | x | y | z | U(eq) | Atom | x | y | z | U(eq) |
|---|---|---|---|---|---|---|---|---|---|
| H11A | 3160.22 | 1532.2 | -2113 | 47 | H48B | 5436.17 | 3201.78 | -1523.57 | 64 |
| H16 | 4063.81 | 7856.51 | 4833.08 | 45 | H25A | 4008.54 | 1095.73 | 5118.72 | 79 |
| H1 | 6904.73 | 6317.48 | 12193.03 | 50 | H25B | 4558.74 | 2095.01 | 5656.44 | 79 |
| H19 | 4496.85 | 7295.83 | 8603.43 | 50 | H16A | 7963.48 | 8665.27 | 9017.97 | 71 |
| H6 | 6069.05 | 2871.66 | 5362.48 | 52 | H16B | 8913.39 | 8290.51 | 9858.41 | 71 |
| H15 | 3610.68 | 2196.77 | 1724.2 | 45 | H46 | 7422.23 | 2210.1 | -1059.19 | 71 |
| H4 | 6489.54 | 6991.97 | 8399.28 | 49 | H86A | 4794.31 | 9171.76 | 9441.05 | 69 |
| H9 | 5692.98 | 1994.29 | 1599.28 | 65 | H86B | 4335.72 | 10255.4 | 9282.49 | 69 |
| H14 | 11199.4 | 6155.11 | 12562.32 | 48 | H84A | -48.02 | 9581.81 | 1708.58 | 83 |
| H83 | 2166.62 | 8698.05 | 2708.75 | 50 | H84B | -868.69 | 8593.98 | 1516.25 | 83 |
| H13 | 11951.8 | 5784.19 | 14056.77 | 52 | H84C | 449.09 | 8599.03 | 1395.94 | 83 |
| H53A | 1105.47 | 805.68 | 917.48 | 51 | H47A | 5204.69 | 5115.68 | -2209.01 | 112 |
| H53B | 114.15 | 1594.61 | 622.35 | 51 | H47B | 3959.54 | 4887.29 | -1994.07 | 112 |
| H34 | 7985.15 | 3628.37 | 7575.96 | 57 | H47C | 3988.68 | 5293.33 | -2931.15 | 112 |
| H59 | -1966.78 | 763.41 | -4022.98 | 56 | H31A | 8827.99 | 2061.62 | 2636.5 | 61 |
| H80 | -356.18 | 7994.58 | 4485.02 | 48 | H31B | 9011.58 | 3224.71 | 2639.39 | 61 |

(continued)

| Atom | x | y | z | U(eq) | Atom | x | y | z | U(eq) |
|---|---|---|---|---|---|---|---|---|---|
| H57 | 1371.74 | 448.85 | -4226.08 | 59 | H62A | 1680.54 | 3448.03 | 489.86 | 72 |
| H60 | -1200.05 | 1120.71 | -2491.44 | 49 | H62B | 503.05 | 3245.39 | 804.36 | 72 |
| H37 | 10506.8 | 3063.53 | 5797.92 | 50 | H15A | 11445.57 | 4950.22 | 15371.21 | 93 |
| H36 | 11318 | 3468.55 | 7325.64 | 57 | H15B | 10124.5 | 5022.33 | 15473.06 | 93 |
| H76A | 1524.37 | 7183.01 | 7678.18 | 58 | H15C | 10996.34 | 5979.58 | 15627.67 | 93 |
| H76B | 1238.86 | 8317.88 | 7636.34 | 58 | H44 | 7544.89 | -228.72 | 329.71 | 81 |
| H8A | 8938.07 | 5798.3 | 9122.37 | 51 | H1A | 5752.11 | 10190.9 | 12992.01 | 104 |
| H8B | 9941.31 | 6654.66 | 9493.57 | 51 | H1B | 4659.41 | 9683.21 | 12248.6 | 104 |
| H2A | 5379.66 | 6948.9 | 12505.56 | 60 | H1C | 5976.93 | 9659.91 | 12130.58 | 104 |
| H2B | 4634.99 | 7679.65 | 11827.21 | 60 | H61A | -1445.83 | -168.15 | -5400.48 | 97 |
| H92 | 2726.01 | 8750.1 | 11184.54 | 85 | H61B | -196 | 207.14 | -5538.28 | 97 |
| H23 | 9004.95 | 8283.75 | 6262.09 | 68 | H61C | -1215.73 | 959 | -5564.19 | 97 |
| H21 | 7560.18 | 5529.61 | 6130.76 | 72 | H17A | 10326.27 | 8274.22 | 9053.26 | 84 |
| H81 | -1157.16 | 8400.7 | 2992.03 | 50 | H17B | 9858.01 | 9345.3 | 9146.2 | 84 |
| H39A | 6260 | 3815.32 | 1334.37 | 79 | H90 | 2150.42 | 5909.85 | 10309.89 | 75 |
| H39B | 7348.42 | 4182.59 | 2154.27 | 79 | H63A | 1518.51 | 4680.33 | 1451.71 | 103 |
| H38A | 9612.09 | 4213.58 | 8823.19 | 102 | H63B | 2650.74 | 4075.77 | 1903.07 | 103 |
| H38B | 10938.8 | 4263.36 | 8719.71 | 102 | H40A | 8652.49 | 3942.3 | 1260.38 | 160 |
| H38C | 10359.3 | 3252 | 8925.46 | 102 | H40B | 7714.95 | 4752.64 | 898.12 | 160 |
| H65 | 1331.6 | 3375.79 | 4000.37 | 63 | H24A | 5596.83 | -855.94 | 6607.32 | 197 |
| H85A | 3697.28 | 9377.55 | 7917.77 | 64 | H24B | 6303.11 | -415.71 | 5951.59 | 197 |
| H85B | 2538.39 | 9558.59 | 8253.12 | 64 | H24C | 4898.89 | -645.61 | 5613.88 | 197 |
| H67 | 2324.12 | 588.56 | 3853.06 | 66 | H70A | 4280.19 | 3885.94 | 3715.83 | 258 |
| H11 | 8569.74 | 5340.88 | 14208.23 | 56 | H70B | 3988.79 | 4436.71 | 4543.92 | 258 |
| H71A | 5980.15 | 6332.85 | 5017.06 | 81 | H70C | 5311.58 | 4122.58 | 4606.96 | 258 |
| H71B | 5199.9 | 7013.33 | 4309.91 | 81 | | | | | |
| H48A | 4897.15 | 2259.61 | -2153.49 | 64 | | | | | |

The structure in the asymmetric unit of the crystal structure is shown in Fig. 4. Furthermore, four molecular structural diagrams of the compound represented by Formula (I) are shown in Figs. 5 to 8, respectively.

The Flack parameter was 0.039(8), and as shown in Figs. 4 to 8, it was confirmed that the present compound was the compound represented by Formula (I) including the steric structure.

The numbers of non-hydrogen atoms in Tables 2 to 3 correspond to the numbers shown in Figs. 4 to 8, respectively.

The crystal structure was identified as a "0.25 hydrate crystal of the compound represented by Formula (I)" since four molecules of the compound represented by Formula (I) and one molecule of water molecule were present in the asymmetric unit.

From the crystal structure, it was confirmed that the powder X-ray diffraction pattern ($\lambda$ = 1.5418 Å) calculated using Mercury (The Cambridge Crystallographic Data Centr, Ver. 4.0.0) was substantially consistent with the powder X-ray diffraction pattern of Example 2 (Fig. 1). From this, it was presumed that Example 2 was a 0.25 hydrate crystal.

Although slight differences were confirmed between them, since the measurement temperature of Example 2 was 25°C and the measurement temperature of Example 3 was -60°C, the slight differences were presumed to be caused by the expansion and contraction of the crystal lattice due to the difference in the measurement temperature.

Example 4

About 2 mg of the crystal of the compound represented by Formula (I) obtained in Example 1 was weighed in an aluminum pan, and the result of performing differential scanning calorimetry (DSC) is shown in Fig. 12. The onset temperature showed an endothermic peak at about 237°C. In addition, since a small endothermic peak was observed at an onset temperature of about 34°C, it is considered that some change such as dehydration occurred. Together with the result of Example 6, it was determined that the endothermic peak at about 34°C was due to dehydration.

In addition, differential scanning calorimetry was performed on an "anhydride crystal of the compound represented by Formula (I) and/or (a/the) hydrate crystal of the compound represented by Formula (I) having a water content corresponding to 0.25 hydrates or less" that were stored under different conditions or subjected to a pulverization treatment. In both cases, the onset temperature showed an endothermic peak at about 237°C.

Example 5

The results of the thermogravimetry/differential thermal analysis (TG/DTA) of the crystal of the compound represented by Formula (I) obtained in Example 1 are shown in Fig. 13. The onset temperature showed an endothermic peak at about 236°C.

In addition, a weight reduction rate of 0.8% was confirmed at about 100°C from immediately after the start of the measurement. The weight reduction rate was equivalent to about 0.23 hydrate when converted as a water molecule (crystal water) in the "hydrate crystal of the compound represented by Formula (I)". From this, the crystal was identified as a "hydrate crystal having a water content corresponding to 0.25 hydrates or less". It is highly possible that the crystal is a "hydrate crystal having a water content corresponding to less than 0.25 hydrates" from the weight reduction rate, but it was not possible to determine whether the crystal is a 0.25 hydrate crystal or a "hydrate crystal having a water content corresponding to less than 0.25 hydrates" since the weight reduction was confirmed immediately after the start of the measurement.

In addition, it was confirmed that crystal water (if present, also adhesion water) was dehydrated to form anhydride crystals at about 100°C from immediately after the start of the measurement. Therefore, the melting point of the "anhydride crystal of the compound represented by Formula (I)" was identified to be about 236 (TG/DTA result) to 237 (DSC result) °C.

The result of Example 5 coincides with that, in Example 4, both of the "anhydride crystal of the compound represented by Formula (I) and/or (a/the) hydrate crystal of the compound represented by Formula (I) having a water content corresponding to 0.25 hydrates or less" that were stored under different conditions or subjected to a pulverization treatment showed an endothermic peak at an onset temperature of about 237°C. The hydrate crystal having a water content corresponding to 0.25 hydrates or less" was determined to show the melting point of the anhydride after being transferred to the "anhydride crystal of the compound represented by Formula (I)".

Example 6

The results of the moisture adsorption/desorption isotherm measurement (DVS) of the crystal of the compound represented by Formula (I) obtained in Example 1 are shown in Fig. 14 and Table 5. The difference in water content between 0%RH (relative humidity 0%) and 95%RH (relative humidity 95%) was about 1.4%. The water content was larger than the water content of crystal water contained in the 0.25 hydrate crystal of the compound represented by Formula (I) (the theoretical value was 0.86w%), but it was determined to be affected by adhesion water adhering to the crystal surface. Therefore, the value of the relative humidity in the moisture adsorption/desorption isotherm measurement (DVS) does not necessarily coincide with the relative humidity to be controlled when the powder X-ray diffraction experiment is performed.

In addition, in the adsorption/desorption isotherm, a rapid change depending on humidity was not observed, and a clear crystal phase transition could not be confirmed. Therefore, it can be presumed that a large crystal structure change due to adsorption/desorption caused by a change in relative humidity is not accompanied.

[Table 5]

| Relative humidity (%) | Moisture adsorption amount (%) | Moisture desorption amount (%) |
|---|---|---|
| 0.0 | 0.000 | 0.063 |
| 5.0 | 0.064 | 0.153 |
| 10.0 | 0.115 | 0.230 |
| 15.0 | 0.174 | 0.316 |

(continued)

| Relative humidity (%) | Moisture adsorption amount (%) | Moisture desorption amount (%) |
|---|---|---|
| 20.0 | 0.234 | 0.395 |
| 25.0 | 0.321 | 0.509 |
| 30.0 | 0.449 | 0.638 |
| 35.0 | 0.549 | 0.740 |
| 40.0 | 0.639 | 0.830 |
| 45.0 | 0.719 | 0.917 |
| 50.0 | 0.798 | 0.989 |
| 55.0 | 0.867 | 1.052 |
| 60.0 | 0.925 | 1.113 |
| 65.0 | 1.003 | 1.179 |
| 70.0 | 1.070 | 1.237 |
| 75.0 | 1.150 | 1.288 |
| 80.0 | 1.196 | 1.314 |
| 85.0 | 1.266 | 1.369 |
| 90.0 | 1.350 | 1.407 |
| 95.0 | 1.426 | 1.426 |

From the results of Example 3 (single crystal structure analysis and powder X-ray diffraction pattern simulated from the crystal structure), Example 5 (thermogravimetry/differential thermal analysis (TG/DTA)), and Example 6 (moisture adsorption/desorption isotherm measurement method (DVS)), the crystal obtained in Example 2 was identified as a "0.25 hydrate crystal of the compound represented by Formula (I)".

Example 7

The crystal of the compound represented by Formula (I) obtained in Example 1 was allowed to stand under the conditions of a temperature of 50°C and a relative humidity of 25% on a powder X-ray diffractometer, and then a powder X-ray diffraction experiment was performed under Measurement conditions 5.

As a result, in the powder X-ray diffraction pattern, peaks were observed at diffraction angles (2θ): 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2°.

In the above-described powder X-ray diffraction pattern, the peaks at the diffraction angles (2θ): 13.1° ± 0.2°, 18.3° ± 0.2°, 20.2° ± 0.2°, 20.5° ± 0.2°, and 23.8° ± 0.2° are particularly characteristic as the "anhydride crystal of the compound represented by Formula (I)".

The powder X-ray diffraction pattern is shown in Fig. 3, and the peak list of the powder X-ray diffraction pattern is shown in Fig. 20.

In the differential scanning calorimetry (DSC) of Example 4, the endothermic peak before the melting point was at an onset temperature of about 34°C. In the thermogravimetry/differential thermal analysis (TG/DTA) of Example 5, the weight loss ended at about 50°C. Therefore, it can be presumed that under the sample static conditions and the measurement conditions (a temperature of 50°C and a relative humidity of 25%) of Example 7, the water molecule of the hydrate crystal was dehydrated and was an anhydride crystal. That is, the powder X-ray diffraction pattern obtained in Example 7 (Fig. 3) was determined to be a powder X-ray diffraction pattern of an "anhydrate crystal of the compound represented by Formula (I)".

Example 8

A hydrate crystal (single crystal) of the compound represented by Formula (I) was immobilized on MicroMount (MiTeGen). At that time, oil coating was not performed, and an extremely small amount of paraffin oil was used for the

contact section. Next, the fixed single crystal was set in a measurement section of a diffractometer set at 25°C, and nitrogen was blown at 25°C for 30 minutes. Thereafter, the setting of the sample low temperature equipment was lowered to - 60°C at once, and a single crystal X-ray diffraction experiment was performed at - 60°C.

The results of the single crystal structure analysis are shown below.

R1 (I > 2.00 s(I)) was 0.0765, and it was confirmed from the final difference Fourier that there was no lack of electron density or misplacement.

Crystallographic data are shown in Table 6.

[Table 6]

| | |
|---|---|
| Space group | $P2_1$ |
| a (Å) | 11.68520(10) |
| b (Å) | 13.35680(10) |
| c (Å) | 15.4299(2) |
| α (°) | 90 |
| β (°) | 105.3090(10) |
| γ (°) | 90 |
| V ($Å^3$) | 2322.80(4) |
| Z | 4 |
| Density (calculated) ($g/cm^3$) | 1.480 |
| Measurement temperature (K) | 213 |

Here, V represents a volume of a unit lattice, and Z represents the number of molecules in the unit lattice.

The atomic fraction coordinates x, y, z ($\times 10^4$) and the equivalent isotropic temperature factor U(eq) (Equivalent Isotropic Displacement Parameters, $Å^2 \times 10^3$) of the non-hydrogen atoms are shown in Table 7. Here, U(eq) is defined as one third of the trace of the orthogonalized $U_{ij}$ tensor.

[Table 7]

| Atom | x | y | z | U(eq) | Atom | x | y | z | U(eq) |
|---|---|---|---|---|---|---|---|---|---|
| S1 | 5635.0(17) | 6479.9(14) | 7985.7(12) | 52.0(4) | C8 | 9096(5) | 4248(5) | 4400(4) | 41.3(14) |
| S2 | 5342(2) | 3111(2) | -1015.9(16) | 71.4(6) | C35 | -382(6) | 6237(5) | -1860(4) | 41.8(14) |
| O9 | 5197(4) | 5027(4) | 2234(3) | 42.5(10) | C10 | 9510(6) | 3809(5) | 7608(4) | 37.9(13) |
| F1 | 7421(5) | 3331(3) | 2731(3) | 61.8(12) | C20 | 7826(6) | 4097(6) | 2321(4) | 44.4(14) |
| O4 | 5703(4) | 4703(5) | 4693(3) | 50.3(12) | C3 | 6409(6) | 5497(6) | 6668(4) | 43.3(14) |
| N7 | 1912(4) | 5774(4) | 244(3) | 34.6(10) | C21 | 7766(8) | 3984(7) | 1425(5) | 58.3(19) |
| N2 | 8988(4) | 4066(4) | 6697(3) | 36.5(11) | C26 | 4345(6) | 4371(5) | 91(4) | 41.2(13) |
| N9 | 3937(4) | 5279(4) | 3115(3) | 38.4(11) | C29 | 4191(5) | 5229(5) | 2300(4) | 35.2(12) |
| N5 | 8756(5) | 3585(4) | 8083(4) | 43.1(12) | C22 | 8158(7) | 4806(7) | 1027(4) | 52.3(17) |
| N4 | 7058(4) | 4879(4) | 3881(3) | 38.6(11) | C12 | 11182(6) | 3599(5) | 8846(4) | 45.3(15) |
| F2 | 8104(5) | 4734(5) | 134(3) | 81.7(17) | O10 | 3151(10) | 6786(14) | 5015(7) | 158(7) |
| N6 | 3867(5) | 5300(4) | 117(3) | 39.4(11) | C14 | 9218(7) | 3377(6) | 8956(5) | 48.4(16) |
| O3 | 6595(5) | 6172(4) | 6200(4) | 59.4(14) | C30 | 2876(6) | 5726(6) | 3292(4) | 47.5(17) |
| O5 | 9115(6) | 6574(5) | 2772(4) | 70.5(17) | C37 | 1559(6) | 6363(5) | -2046(4) | 42.6(14) |
| O8 | 4207(5) | 3673(4) | 551(4) | 59.9(14) | C13 | 10443(7) | 3396(5) | 9373(4) | 47.2(15) |
| N1 | 7053(5) | 4623(5) | 6817(4) | 42.3(12) | C11 | 10737(6) | 3808(5) | 7943(4) | 41.0(14) |
| N8 | 1283(4) | 5873(5) | 872(3) | 41.1(12) | C31 | 1754(6) | 5552(7) | 2518(4) | 49.5(18) |

(continued)

| Atom | x | y | z | U(eq) | Atom | x | y | z | U(eq) |
|---|---|---|---|---|---|---|---|---|---|
| N3 | 9616(5) | 3964(5) | 6063(3) | 41.0(12) | C23 | 8603(6) | 5640(7) | 1459(5) | 50.6(17) |
| O1 | 6806(6) | 6354(6) | 8560(4) | 79.0(18) | C38 | -170(7) | 6708(7) | -3394(5) | 60(2) |
| N10 | 2068(5) | 6166(4) | -1191(3) | 39.3(11) | C2 | 5447(6) | 5535(6) | 7146(5) | 52.0(17) |
| F4 | 2472(6) | 3649(6) | 3517(6) | 105(2) | O6 | 6267(6) | 2692(6) | -295(5) | 81(2) |
| O2 | 4652(6) | 6364(5) | 8375(5) | 76.5(19) | C16 | 8669(8) | 6100(6) | 4132(5) | 54.3(18) |
| C6 | 6755(5) | 4728(5) | 4647(4) | 37.7(13) | C46 | 2649(13) | 5390(30) | 5717(8) | 185(15) |
| C33 | 1348(6) | 5983(5) | -672(4) | 37.1(12) | C43 | 2530(8) | 4207(13) | 4221(9) | 95(5) |
| C27 | 3079(5) | 5490(5) | 625(4) | 34.7(12) | C15 | 10887(9) | 3225(7) | 10381(5) | 65(2) |
| C5 | 7759(5) | 4508(5) | 5418(4) | 34.9(12) | C42 | 2735(7) | 5218(11) | 4164(5) | 79(4) |
| C34 | 120(6) | 6003(5) | -966(4) | 39.4(13) | C25 | 4987(9) | 4296(7) | -651(7) | 70(2) |
| C32 | 2054(5) | 5649(5) | 1636(4) | 38.7(13) | C39 | 3078(9) | 6866(8) | 3432(6) | 78(3) |
| C7 | 8275(5) | 5033(5) | 3815(4) | 34.4(12) | F3 | 2417(8) | 3904(18) | 6491(6) | 313(14) |
| C18 | 8661(6) | 5732(5) | 2375(4) | 42.8(14) | C1 | 5495(10) | 7599(8) | 7451(7) | 76(3) |
| O7 | 5655(6) | 3295(6) | -1820(5) | 76.0(19) | C41 | 2867(9) | 5850(20) | 4938(6) | 133(8) |
| C36 | 344(6) | 6448(5) | -2424(4) | 43.0(14) | C45 | 2484(15) | 4490(40) | 5737(16) | 280(30) |
| C28 | 3177(5) | 5425(5) | 1540(4) | 34.5(12) | C24 | 4160(10) | 2348(9) | -1183(9) | 93(4) |
| C19 | 8268(5) | 4932(5) | 2825(4) | 34.3(12) | C17 | 9571(12) | 6501(11) | 3789(8) | 91(3) |
| C9 | 8861(5) | 4242(5) | 5303(4) | 35.6(12) | C44 | 2427(9) | 3740(20) | 4989(11) | 177(12) |
| C4 | 7865(5) | 4402(5) | 6326(4) | 35.2(12) | C40 | 3775(12) | 7094(11) | 4373(9) | 117(6) |

Next, the atomic coordinates x, y, z (× $10^4$) and the isotropic temperature factor U(eq) (Isotropic Displacement Parameters, $Å^2$ × $10^3$) of the hydrogen atoms are shown in Table 8.

[Table 8]

| Atom | x | y | z | U(eq) | Atom | x | y | z | U(eq) |
|---|---|---|---|---|---|---|---|---|---|
| H9 | 4454.69 | 5021.35 | 3571.84 | 46 | H16A | 7976.39 | 6542.59 | 3961.99 | 65 |
| H4 | 6491.69 | 4886.75 | 3384.83 | 46 | H16B | 8944.13 | 6098.94 | 4789.81 | 65 |
| H6 | 4063.33 | 5783.66 | -193.9 | 47 | H46 | 2632.76 | 5783.19 | 6218.02 | 222 |
| H1 | 6952.77 | 4203.34 | 7221.61 | 51 | H15A | 10218.88 | 3081.98 | 10624.44 | 97 |
| H34 | -352.84 | 5863.9 | -573.97 | 47 | H15B | 11291.67 | 3821.46 | 10665.51 | 97 |
| H8A | 8939.21 | 3584.86 | 4123.35 | 50 | H15C | 11432.25 | 2663.65 | 10496.62 | 97 |
| H8B | 9928.28 | 4418.98 | 4456.04 | 50 | H25A | 5730.57 | 4671.55 | -448.69 | 84 |
| H35 | -1211.66 | 6253.15 | -2086.87 | 50 | H25B | 4500.38 | 4646.84 | -1177.81 | 84 |
| H21 | 7482.18 | 3396.12 | 1105.07 | 70 | H39A | 3504.86 | 7119.03 | 3009.94 | 93 |
| H12 | 12007.42 | 3598 | 9100.46 | 54 | H39B | 2309.02 | 7206.73 | 3307.08 | 93 |
| H14 | 8698.08 | 3211.68 | 9306.46 | 58 | H1A | 5449.84 | 8128.69 | 7870.25 | 114 |
| H37 | 2058.78 | 6453.94 | -2427.8 | 51 | H1B | 4777.97 | 7599.22 | 6959.41 | 114 |
| H11 | 11240.49 | 3942.63 | 7571.98 | 49 | H1C | 6176.93 | 7709.57 | 7215.52 | 114 |
| H31A | 1436.27 | 4882.32 | 2570.08 | 59 | H24A | 3465.74 | 2686.86 | -1549.49 | 140 |
| H31B | 1146.29 | 6044.7 | 2552.7 | 59 | H24B | 4024.34 | 2171.53 | -608.11 | 140 |

(continued)

| Atom | x | y | z | U(eq) | Atom | x | y | z | U(eq) |
|---|---|---|---|---|---|---|---|---|---|
| H23 | 8874.03 | 6159.72 | 1153.62 | 61 | H24C | 4307.71 | 1745.19 | -1486.77 | 140 |
| H38A | -931.61 | 6378.44 | -3614.32 | 90 | H17A | 10273.12 | 6069.55 | 3950.27 | 110 |
| H38B | 364.51 | 6485.52 | -3741.15 | 90 | H17B | 9794.63 | 7166.91 | 4044.49 | 110 |
| H38C | -275.84 | 7426.86 | -3455.56 | 90 | H44 | 2331.92 | 3048.38 | 5043.03 | 213 |
| H2A | 5406.41 | 4882.57 | 7427.46 | 62 | H40A | 4541.08 | 6748.91 | 4499.32 | 140 |
| H2B | 4686.96 | 5644.09 | 6700.91 | 62 | H40B | 3928.98 | 7815.74 | 4432.67 | 140 |

The structure in the asymmetric unit of the crystal structure is shown in Fig. 9. Furthermore, two molecular structural diagrams of the compound represented by Formula (I) are shown in Figs. 10 to 11, respectively.

The Flack parameter was 0.021(7), and as shown in Figs. 9 to 11, it was confirmed that the present compound was the compound represented by Formula (I) including the steric structure.

The numbers of non-hydrogen atoms in Table 7 correspond to the numbers shown in Figs. 9 to 11, respectively.

The crystal structure was identified as an "anhydride crystal of the compound represented by Formula (I)" since two molecules of the compound represented by Formula (I) were present in the asymmetric unit and no other solvent or the like is present.

From the crystal structure, it was confirmed that the powder X-ray diffraction pattern ($\lambda$ = 1.5418 Å) calculated using Mercury (The Cambridge Crystallographic Data Centr, Ver. 4.0.0) was substantially consistent with the powder X-ray diffraction pattern of Example 7 (Fig. 3). From this, Example 7 was identified as a powder X-ray diffraction pattern of an anhydride crystal.

This is also consistent with the fact that it can be presumed that it was an anhydride from the measurement conditions of Example 7 (a temperature of 50°C and a relative humidity of 25%), the results of differential scanning calorimetry (DSC) of Example 4, and the thermogravimetry/differential thermal analysis (TG/DTA) of Example 5.

Incidentally, slight differences were confirmed between the powder X-ray diffraction pattern calculated from the crystal structure and the powder X-ray diffraction pattern of Example 7. However, since the measurement temperature of Example 7 was 50°C and the measurement temperature of Example 8 was -60°C, which were greatly different, the slight differences were presumed to be caused by the expansion and contraction of the crystal lattice due to the difference in the measurement temperature.

Further, the lattice constant of the "0.25 hydrate crystal of the compound represented by Formula (I)" obtained in Example 3 and "the lattice constant of the anhydride crystal of the compound represented by Formula (I)" obtained in Example 8 are different in space group, but are very similar in that the difference in the a-axis is about 0.12 Å, the difference in the b-axis is about 0.22 Å, the difference in the c-axis is about 0.11 Å, the difference in $\alpha$ is about 2.1°, the difference in $\beta$ is about 0.02°, and the difference in y is about 2.3°.

Furthermore, the crystal structures of both are very similar. The same applies to the point that the amide moiety of the dihydropyridine of the compound molecule represented by Formula (I) forms an intermolecular hydrogen bond with the amide moiety of the dihydropyridine of the adjacent compound molecule represented by Formula (I). From this, it can be presumed that the 0.25 hydrate crystal and the anhydride crystal have a structure in which water reversibly enters and leaves in a state where the crystal structure is substantially maintained.

In pharmaceutical products, it is generally known that crystal polymorphs such as hydrates and anhydrates are significantly different in lattice constant and crystal structure than those described above. The 0.25 hydrate crystal and the anhydride crystal of the compound represented by Formula (I) have the same crystal form rather than being considered as general crystal polymorphs, and it is considered that a slight change occurs due to reversible insertion and extraction of water. The same applies to a "hydrate crystal having a water content corresponding to less than 0.25 hydrates" which is an intermediate state between the two.

In addition, regarding not only the single crystal structure analysis but also the powder X-ray diffraction pattern, Example 2 (Fig. 1) and Example 7 (Fig. 3) were very similar, and the characteristic peaks were substantially the same. Strictly speaking, slight differences were confirmed in the diffraction angles (2$\theta$): 16.0° to 17.8°, 19.5° to 21.0°, and 23.5° to 24.2°. These slight differences can be presumed to be due to the presence or absence of water molecules.

Example 9

After the powder X-ray diffraction experiment of Example 2, the sample environment on the powder X-ray diffractometer was changed to a temperature of 25°C and a relative humidity of 35%, the sample was allowed to stand, and

a powder X-ray diffraction experiment was performed under Measurement conditions 4.

As a result, in the powder X-ray diffraction pattern, peaks were observed at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1 ± 0.2°.

In the above-described powder X-ray diffraction pattern, the peaks at the diffraction angles (2θ): 13.0° ± 0.2°, 18.4° ± 0.2°, 20.0° ± 0.2°, 20.4° ± 0.2°, and 23.7° ± 0.2° are particularly characteristic as the "hydrate crystal of the compound represented by Formula (I) having a water content corresponding to less than 0.25 hydrates".

The powder X-ray diffraction pattern is shown in Fig. 2, and the peak list of the powder X-ray diffraction pattern is shown in Fig. 21.

The powder X-ray diffraction pattern (Fig. 2) obtained in Example 9 was very similar to the powder X-ray diffraction pattern (Fig. 1) of the "0.25 hydrate crystal of the compound represented by Formula (I)" obtained in Example 2, and the characteristic peaks were substantially the same. On the other hand, slight differences were confirmed in the diffraction angles (2θ): 16.0° to 17.8°, 19.5° to 21.0°, and 23.5° to 24.2°.

In Example 2 and Example 9, the powder X-ray diffraction experiment was performed on the completely same sample (same crystal), but it was confirmed that the powder X-ray diffraction pattern and the diffraction angle (2θ) were slightly different although they were very similar depending on the environment (relative humidity and/or temperature) in which the powder X-ray diffraction experiment was performed. In other words, in the "0.25 hydrate crystal of the compound represented by Formula (I)", it has become clear that the powder X-ray diffraction pattern and the diffraction angle (2θ) change depending on the environment (relative humidity and/or temperature) in which the powder X-ray diffraction experiment is performed.

Also, the powder X-ray diffraction pattern obtained in Example 9 (Fig. 2) was very similar to the powder X-ray diffraction pattern of the "anhydride crystal of the compound represented by Formula (I)" obtained in Example 7 (Fig. 3), and the characteristic peaks were substantially the same. On the other hand, slight differences were confirmed in the diffraction angles (2θ): 16.0° to 17.8°, 19.5° to 21.0°, and 23.5° to 24.2°.

That is, the powder X-ray diffraction pattern obtained in Example 9 was very similar to the powder X-ray diffraction pattern of the "0.25 hydrate crystal of the compound represented by Formula (I)" and the powder X-ray diffraction pattern of the "anhydride crystal of the compound represented by Formula (I)", but slight differences were confirmed in the diffraction angles (2θ): 16.0° to 17.8°, 19.5° to 21.0°, and 23.5° to 24.2°.

The powder X-ray diffraction patterns of Examples 2, 7 and 9 were very similar, and the crystal structures of the 0.25 hydrate crystal and the anhydride crystal obtained in Examples 3 and 8 were very similar. Therefore, it is considered that the "crystal of the compound represented by Formula (I) in an environment of 25°C and 35% relative humidity" of Example 9 is similar to the crystal structures of the 0.25 hydrate crystal and the anhydride crystal, and has a crystal structure intermediate between the two.

From the moisture adsorption/desorption isotherm measurement (DVS) of Example 6, it was presumed that the powder X-ray diffraction pattern obtained in Example 9 was the powder X-ray diffraction pattern of the "hydrate crystal of the compound represented by Formula (I) having a water content corresponding to less than 0.25 hydrates".

In addition, since the moisture adsorption/desorption isotherm of Example 6 was a gentle curve and did not show a rapid change at a specific relative humidity, it was presumed that a large change in the crystal structure due to adsorption/desorption of crystal water is not accompanied. The result is also consistent with the fact that the powder X-ray diffraction patterns of Examples 2, 7, and 9 were very similar, and the crystal structures of Examples 3 and 8 were very similar.

Therefore, the results of Examples 2, 7, and 9 show that, in the "anhydride crystal of the compound represented by Formula (I) and/or (a/the) hydrate crystal of the compound represented by Formula (I) having a water content corresponding to 0.25 hydrates or less", the powder X-ray diffraction pattern and the diffraction angles (2θ) change depending on the environment (relative humidity and/or temperature) in which the powder X-ray diffraction experiment is performed.

Slight differences in the three powder X-ray diffraction patterns obtained in Examples 2, 7, and 9 are differences in each crystal form of the "anhydride crystal of the compound represented by Formula (I)", the "hydrate crystal of the compound represented by Formula (I) having a water content corresponding to less than 0.25 hydrates", and the "0.25 hydrate crystal of the compound represented by Formula (I)", and these differences are considered to be due to the content of crystal water.

Example 9B

For the crystal of the compound represented by Formula (I) obtained in Example 1, and a powder X-ray diffraction experiment was performed under Measurement conditions 6.

As a result, from the powder X-ray diffraction pattern, it was confirmed that the sample (crystal) was changed from the "0.25 hydrate crystal of the compound represented by Formula (I)" to the "hydrate crystal of the compound represented

by Formula (I) having a water content corresponding to less than 0.25 hydrates", and continuously changed from the "hydrate crystal of the compound represented by Formula (I) having a water content corresponding to less than 0.25 hydrates" to the "anhydride crystal of the compound represented by Formula (I)".

From the results of Examples 2, 7, 9, and 9B, it was revealed that when powder X-ray diffraction experiments were performed under the atmosphere without controlling the "anhydride crystal of the compound represented by Formula (I) and/or (a/the) hydrate crystal of the compound represented by Formula (I) having a water content corresponding to 0.25 hydrates or less" under a specific temperature and/or relative humidity, the powder X-ray diffraction patterns were slightly different under the influence of the temperature and relative humidity (weather, climate, and the like) at the time of measurement.

Example 9C

On a certain day, for the crystal of the compound represented by Formula (I) obtained in Example 1, a powder X-ray diffraction experiment under Measurement conditions 3 (temperature controller and humidity controller were not used) in the atmosphere without controlling the temperature and the relative humidity. As a result, it was confirmed that the results were consistent with the results of Example 9. At the time of the measurement, it was determined that the crystal was a "hydrate crystal of the compound represented by Formula (I) having a water content corresponding to less than 0.25 hydrates".

On another day, for the crystal of the compound represented by Formula (I) obtained in Example 1, a powder X-ray diffraction experiment under Measurement conditions 3 (temperature controller and humidity controller were not used) in the atmosphere without controlling the temperature and the relative humidity. As a result, it was confirmed that the results were consistent with the results of Example 2. At the time of the measurement, it was determined that the crystal was a "0.25 hydrate crystal of the compound represented by Formula (I)".

Example 10

From the results of Examples 2, 7, 9, and 9B (powder X-ray diffraction experiment), Examples 3 and 8 (single crystal structure analysis), Examples 4 and 5 (thermal analysis), and Example 6 (moisture adsorption/desorption isotherm measurement (DVS)), it is natural that the "anhydride crystal of the compound represented by Formula (I)", the "hydrate crystal of the compound represented by Formula (I) having a water content corresponding to less than 0.25 hydrates", and the "0.25 hydrate crystal of the compound represented by Formula (I)" have the same crystal form rather than being considered as three crystal forms, and a slight change is considered to occur due to reversible insertion and extraction of water.

Therefore, the diffraction angle (2θ) and variation of the powder X-ray diffraction pattern to be the basis for specifying the "anhydride crystal of the compound represented by Formula (I) and/or (a/the) hydrate crystal of the compound represented by Formula (I) having a water content corresponding to 0.25 hydrates or less" of the present invention were calculated as follows.

Diffraction angles (2θ) observed in the powder X-ray diffraction patterns of Examples 2, 7, and 9 were extracted, and values obtained by adding measurement errors of ± 0.2° thereof were listed. The results are shown in Table 9.

[Table 9]

| Example 2 (unit: °) | | | | Example 7 (unit: °) | | | | Example 9 (unit: °) | | |
|---|---|---|---|---|---|---|---|---|---|---|
| A | B | C | | D | E | F | | G | H | I |
| Experiment al value -0.2 | Diffraction angle (Experimental value) | Experiment al value +0.2 | | Experiment al value -0.2 | Diffraction angle (Experimental value) | Experiment al value +0.2 | | Experiment al value -0.2 | Diffraction angle (Experimental value) | Experiment al value +0.2 |
| 12.8 | 13.0 | 13.2 | | 12.9 | 13.1 | 13.3 | | 12.8 | 13.0 | 13.2 |
| 15.3 | 15.5 | 15.7 | | 15.4 | 15.6 | 15.8 | | 15.3 | 15.5 | 15.7 |
| 16.4 | 16.6 | 16.8 | | 16.5 | 16.7 | 16.9 | | 16.4 | 16.6 | 16.8 |
| 16.7 | 16.9 | 17.1 | | 16.9 | 17.1 | 17.3 | | 16.7 | 16.9 | 17.1 |
| 17.3 | 17.5 | 17.7 | | | | | | 17.2 | 17.4 | 17.6 |
| 18.2 | 18.4 | 18.6 | | 18.1 | 18.3 | 18.5 | | 18.2 | 18.4 | 18.6 |
| 19.0 | 19.2 | 19.4 | | 19.3 | 19.5 | 19.7 | | 19.1 | 19.3 | 19.5 |
| 19.7 | 19.9 | 20.1 | | 20.0 | 20.2 | 20.4 | | 19.8 | 20.0 | 20.2 |
| 20.3 | 20.5 | 20.7 | | 20.3 | 20.5 | 20.7 | | 20.2 | 20.4 | 20.6 |
| 21.3 | 21.5 | 21.7 | | 21.3 | 21.5 | 21.7 | | 21.2 | 21.4 | 21.6 |
| 23.5 | 23.7 | 23.9 | | 23.6 | 23.8 | 24.0 | | 23.5 | 23.7 | 23.9 |
| 24.0 | 24.2 | 24.4 | | | | | | 23.9 | 24.1 | 24.3 |

Next, the minimum values in consideration of the measurement error (minimum values of A, D, G) and the maximum values in consideration of the measurement error (minimum values of C, F, I) were extracted with respect to the values indicating substantially the same diffraction angles in Table 9. The results are shown in J and K of Table 10. Further, average values and variations of the minimum value J and the maximum value K were obtained, and are shown in L and M of Table 10.

[Table 10]

| Diffraction angle (unit: °) common to Examples 2, 7, and 9 | | | |
|---|---|---|---|
| J | K | L | M |
| Minimum value of experimental values - 0.2 (A, D, G) | Maximum value of experimental values - 0.2 (C, F, I) | Average value of J and K | Difference between average value (L) and J, K |
| 12.8 | 13.3 | 13.05 | 0.25 |
| 15.3 | 15.8 | 15.55 | 0.25 |
| 16.4 | 16.9 | 16.65 | 0.25 |
| 16.7 | 17.3 | 17.00 | 0.30 |
| - | | | |
| 18.1 | 18.6 | 18.35 | 0.25 |
| 19.0 | 19.7 | 19.35 | 0.35 |
| 19.7 | 20.4 | 20.05 | 0.35 |
| 20.2 | 20.7 | 20.45 | 0.25 |
| 21.2 | 21.7 | 21.45 | 0.25 |
| 23.5 | 24.0 | 23.75 | 0.25 |
| - | | | |

From this result, it can be said that the "anhydride crystal of the compound represented by Formula (I) and/or (a/the) hydrate crystal of the compound represented by Formula (I) having a water content corresponding to 0.25 hydrates or less" has, in the powder X-ray diffraction pattern,
characteristic peaks at diffraction angles (2θ): 13.05° ± 0.25°, 18.35° ± 0.25°, 20.05° ± 0.35°, 20.45° ± 0.25°, and 23.75° ± 0.25°.

When the anhydride crystal is identified in more peaks,
the crystal has characteristic peaks at diffraction angles (2θ): 13.05° ± 0.25°, 15.55° ± 0.25°, 16.65° ± 0.25°, 17.00° ± 0.30°, 18.35° ± 0.25°, 19.35° ± 0.35°, 20.05° ± 0.35°, 20.45° ± 0.25°, 21.45° ± 0.25°, and 23.75° ± 0.25°.

Example 11

The result of the Raman spectrum of the crystal of the compound represented by Formula (I) obtained in Example 1 (Measurement conditions 1) is shown in Figs. 22 to 24. The absorption peaks are shown below.

[Table 11]

| Raman peak $cm^{-1}$ | Raman peak $cm^{-1}$ | Raman peak $cm^{-1}$ | Raman peak $cm^{-1}$ |
|---|---|---|---|
| 425 | 692 | 1026 | 1413 |
| 456 | 728 | 1071 | 1448 |
| 496 | 754 | 1088 | 1485 |
| 521 | 774 | 1112 | 1511 |
| 541 | 819 | 1162 | 1609 |
| 559 | 848 | 1194 | 1650 |
| 580 | 887 | 1215 | 1720 |

(continued)

| Raman peak $cm^{-1}$ | Raman peak $cm^{-1}$ | Raman peak $cm^{-1}$ | Raman peak $cm^{-1}$ |
|---|---|---|---|
| 602 | 902 | 1227 | 2885 |
| 622 | 917 | 1266 | 2928 |
| 644 | 939 | 1320 | 2947 |
| 655 | 965 | 1364 | 2999 |
| 671 | 994 | 1387 | 3078 |

From the results of Examples 9 and 6, it was presumed that the results were a "hydrate crystal of the compound represented by Formula (I) having a water content corresponding to less than 0.25 hydrates".

In one embodiment, the "hydrate crystal of the compound represented by Formula (I) having a water content corresponding to less than 0.25 hydrates" has main absorption peaks at 1448 $cm^{-1}$ ± 2 $cm^{-1}$, 1511 $cm^{-1}$ ± 2 $cm^{-1}$, 1650 $cm^{-1}$ ± 2 $cm^{-1}$, 2885 $cm^{-1}$ ± 2 $cm^{-1}$, and 2947$cm^{-1}$ ± 2$cm^{-1}$.

In one embodiment, the "hydrate crystal of the compound represented by Formula (I) having a water content corresponding to less than 0.25 hydrates" has one or more absorption peaks selected from the group consisting of absorption peaks at 1448 $cm^{-1}$ ± 2 $cm^{-1}$, 1511 $cm^{-1}$ ± 2 $cm^{-1}$, 1650 $cm^{-1}$ ± 2 $cm^{-1}$, 2885 $cm^{-1}$ ± 2 $cm^{-1}$, and 2947$cm^{-1}$ ± 2$cm^{-1}$.

Also for the anhydride crystal and 0.25 hydrate crystal of the compound represented by Formula (I), Raman spectrum measurement was similarly performed under the conditions of maintaining each crystal form to confirm that these crystals have similar absorption peaks.

Example 12

Methyl isobutyl ketone (MIBK) (1000 μL) was added to 10 mg of the crystal of the compound represented by Formula (I) obtained in Example 1, and the mixture was heated to 60°C, shaken, and allowed to stand at room temperature. Thereafter, the solvent was removed with a centrifugal evaporator to obtain a B-form crystal of the compound represented by Formula (I).

For the B-form crystal, a powder X-ray diffraction experiment was performed. The powder X-ray diffraction pattern is shown in Fig. 39, and a list of peak positions and intensities is shown in Fig. 40.

The powder X-ray diffraction pattern of the B-form crystal was different from all of the followings:

a powder X-ray diffraction pattern having peaks at diffraction angles (2θ): 13.0° ± 0.2°, 18.4° ± 0.2°, 20.0° ± 0.2°, 20.4° ± 0.2°, and 23.7° ± 0.2°,

a powder X-ray diffraction pattern having peaks at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2°,

a powder X-ray diffraction pattern having peaks at diffraction angles (2θ): 13.0° ± 0.2°, 18.4° ± 0.2°, 19.9° ± 0.2°, 20.5° ± 0.2°, and 23.7° ± 0.2°,

a powder X-ray diffraction pattern having peaks at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2° ± 0.2°,

a powder X-ray diffraction pattern having peaks at diffraction angles (2θ): 13.1° ± 0.2°, 18.3° ± 0.2°, 20.2° ± 0.2°, 20.5° ± 0.2°, and 23.8° ± 0.2°, and

a powder X-ray diffraction pattern having peaks at diffraction angles (2θ): 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2°.

For the B-form crystal, thermogravimetry/differential thermal analysis (TG/DTA) was performed. As a result, an endothermic peak at an onset temperature of about 159 to 161°C and an endothermic peak at an onset temperature of about 236°C were shown, and an exothermic peak was shown between the two endothermic peaks. In addition, a weight reduction rate of about 2.0 to 6.5% was confirmed at about 170°C immediately after the start of the measurement. Although the weight reduction rate was different for each measurement, it is considered to be caused by the influence of the external environment (relative humidity and/or temperature) before the measurement since the weight reduction starts immediately after the start of the measurement.

From the results of thermogravimetry/differential thermal analysis (TG/DTA), it was confirmed that the melting point of the B-form crystal was about 159 to 161°C. It was presumed that the B-form crystal is transferred to the anhydride

crystal of the compound represented by Formula (I) after melting.

Therefore, from the results of the powder X-ray diffraction experiment and the thermogravimetry/differential thermal analysis (TG/DTA), it was confirmed that the B-form crystal had a crystal form different from that of "the anhydride crystal of the compound represented by Formula (I), or the hydrate crystal of the compound represented by Formula (I) having a water content corresponding to 0.25 hydrates or less".

In addition, since the melting point of the B-form crystal was lower than the melting point of "the anhydride crystal of the compound represented by Formula (I), or the hydrate crystal of the compound represented by Formula (I) having a water content corresponding to 0.25 hydrates or less" (strictly speaking, the anhydride crystal of the compound represented by Formula (I), it was determined to be a metastable crystal.

Example 13

For the crystal of the amorphous substance of the compound represented by Formula (I) obtained in Example 1, and a powder X-ray diffraction experiment was performed under Measurement conditions 1.

The powder X-ray diffraction pattern is shown in Fig. 25. No characteristic peak was observed, and a halo pattern was confirmed.

Example 14

The results of the moisture adsorption/desorption isotherm measurement of the amorphous substance of the compound represented by Formula (I) obtained in Example 1 are shown in Fig. 26 and Table 12.

[Table 12]

| Relative humidity (%) | Moisture adsorption amount (%) | Moisture desorption amount (%) |
|---|---|---|
| 0.0 | 0.002 | -0.433 |
| 5.0 | 0.676 | 0.606 |
| 10.0 | 1.020 | 1.196 |
| 15.0 | 1.312 | 1.674 |
| 20.0 | 1.574 | 2.131 |
| 25.0 | 1.807 | 2.495 |
| 30.0 | 2.015 | 2.831 |
| 35.0 | 2.213 | 3.157 |
| 40.0 | 2.414 | 3.423 |
| 45.0 | 2.589 | 3.699 |
| 50.0 | 2.780 | 3.913 |
| 55.0 | 2.976 | 4.135 |
| 60.0 | 3.185 | 4.411 |
| 65.0 | 3.384 | 4.683 |
| 70.0 | 3.623 | 5.151 |
| 75.0 | 3.914 | 5.420 |
| 80.0 | 4.320 | 5.865 |
| 85.0 | 4.850 | 6.219 |
| 90.0 | 5.604 | 6.599 |
| 95.0 | 6.974 | 6.974 |

The difference in water content between 0%RH (relative humidity 0%) and 95%RH (relative humidity 95%) was as high as about 7.0%. In general, a solid having a large change in water content due to relative humidity was determined not to be suitable as a drug substance since the content of a drug contained per weight greatly varies depending on the

humidity to be weighed.

Example 15

The result of the Raman spectrum of the amorphous substance of the compound represented by Formula (I) (Measurement conditions 1) is shown in Figs. 27 to 29. The absorption peaks are shown below.

[Table 13]

| Raman peak $cm^{-1}$ | Raman peak $cm^{-1}$ | Raman peak $cm^{-1}$ | Raman peak $cm^{-1}$ |
|---|---|---|---|
| 421 | 644 | 1195 | 1489 |
| 457 | 726 | 1227 | 1606 |
| 485 | 744 | 1261 | 1666 |
| 497 | 815 | 1317 | 1716 |
| 512 | 890 | 1330 | 2930 |
| 543 | 1023 | 1365 | |
| 568 | 1087 | 1388 | |
| 619 | 1152 | 1408 | |

Example 16

An amorphous compound represented by Formula (II) was obtained in the same manner as described in Example 12 of Patent Document 1.

Acetone (200 μL) was added to the obtained amorphous compound represented by Formula (II) (5 mg), and the mixture was stirred by a magnetic stirrer at room temperature for 5 days. The solvent was distilled off by filter filtration to obtain a crystal.

Example 17

For the crystal of the compound represented by Formula (II) obtained in Example 16, a powder X-ray diffraction experiment was performed under Measurement conditions 2.

As a result, in the powder X-ray diffraction pattern, peaks were observed at diffraction angles (2θ): 15.1° ± 0.2°, 16.3° ± 0.2°, 18.4° ± 0.2°, 19.2° ± 0.2°, 20.4° ± 0.2°, 21.1° ± 0.2°, 22.0° ± 0.2°, 22.8° ± 0.2°, 23.3° ± 0.2°, and 23.9° ± 0.2°.

In the above-described powder X-ray diffraction pattern, the peaks at the diffraction angles (2θ): 16.3° ± 0.2°, 18.4° ± 0.2°, 20.4° ± 0.2°, 22.8° ± 0.2°, and 23.9° ± 0.2° are particularly characteristic as the crystal of the compound represented by Formula (II).

The powder X-ray diffraction pattern is shown in Figs. 15 and 30.

Example 18

About 1 mg of the crystal of the compound represented by Formula (II) obtained in Example 16 was weighed in an aluminum pan, and the result of performing DSC analysis is shown in Fig. 16. The onset temperature was about 181°C.

Example 19

The TG/DTA analysis result of the crystal of the compound represented by Formula (II) obtained in Example 16 is shown in Fig. 17.

Example 20

The results of moisture adsorption/desorption isotherm measurement of the crystal of the compound represented by Formula (II) obtained in Example 16 are shown in Fig. 18 and Table 14. The difference in water content between 0%RH (relative humidity 0%) and 95%RH (relative humidity 95%) was about 0.4%, and it was confirmed that the crystal had no problem in hygroscopicity.

[Table 14]

| Relative humidity (%) | Moisture adsorption amount (%) | Moisture desorption amount (%) |
|---|---|---|
| 0.0 | 0.0000 | 0.0690 |
| 5.0 | 0.0149 | 0.1029 |
| 10.0 | 0.0250 | 0.1252 |
| 15.0 | 0.0352 | 0.1435 |
| 20.0 | 0.0494 | 0.1610 |
| 25.0 | 0.0623 | 0.1773 |
| 30.0 | 0.0758 | 0.1922 |
| 35.0 | 0.0893 | 0.2077 |
| 40.0 | 0.1042 | 0.2206 |
| 45.0 | 0.1204 | 0.2348 |
| 50.0 | 0.1387 | 0.2497 |
| 55.0 | 0.1563 | 0.2659 |
| 60.0 | 0.1746 | 0.2822 |
| 65.0 | 0.2016 | 0.3038 |
| 70.0 | 0.2334 | 0.3289 |
| 75.0 | 0.2619 | 0.3464 |
| 80.0 | 0.2937 | 0.3627 |
| 85.0 | 0.3268 | 0.3782 |
| 90.0 | 0.3695 | 0.4013 |
| 95.0 | 0.4243 | 0.4243 |

Example 21

The result of the Raman spectrum of the crystal of the compound represented by Formula (II) (Measurement conditions 2) is shown in Figs. 31 to 33. The absorption peaks are shown below.

[Table 15]

| Raman peak $cm^{-1}$ | Raman peak $cm^{-1}$ | Raman peak $cm^{-1}$ | Raman peak $cm^{-1}$ |
|---|---|---|---|
| 447 | 768 | 1058 | 1410 |
| 470 | 788 | 1083 | 1433 |
| 489 | 805 | 1118 | 1483 |
| 531 | 852 | 1150 | 1531 |
| 577 | 882 | 1220 | 1571 |
| 591 | 908 | 1246 | 1596 |
| 612 | 927 | 1270 | 1614 |
| 671 | 955 | 1299 | 1652 |
| 728 | 998 | 1323 | 1695 |
| 740 | 1028 | 1390 | 1718 |

In one embodiment, the crystal of the compound represented by Formula (II) has main absorption peaks at 577 $cm^{-1} \pm 2$ $cm^{-1}$, 612 $cm^{-1} \pm 2$ $cm^{-1}$, 740 $cm^{-1} \pm 2$ $cm^{-1}$, 1571 $cm^{-1} \pm 2$ $cm^{-1}$, and 1718 $cm^{-1} \pm 2$ $cm^{-1}$.

In one embodiment, the crystal of the compound represented by Formula (II) has one or more absorption peaks selected from the group consisting of absorption peaks at 577 $cm^{-1}$ ± 2 $cm^{-1}$, 612 $cm^{-1}$ ± 2 $cm^{-1}$, 740 $cm^{-1}$ ± 2 $cm^{-1}$, 1571 $cm^{-1}$ ± 2 $cm^{-1}$, and 1718 $cm^{-1}$ ± 2 $cm^{-1}$.

Example 22

For the amorphous substance of the compound represented by Formula (II) obtained in Example 16, and a powder X-ray diffraction experiment was performed under Measurement conditions 1.
The powder X-ray diffraction pattern is shown in Fig. 34. No characteristic peak was observed, and a halo pattern was confirmed.

Example 23

The results of the moisture adsorption/desorption isotherm measurement of the amorphous substance of the compound represented by Formula (II) obtained in Example 16 are shown in Fig. 35 and Table 16. The difference in water content between 0%RH (relative humidity 0%) and 95%RH (relative humidity 95%) was about 2.6%.

[Table 16]

| Relative humidity (%) | Moisture adsorption amount (%) | Moisture desorption amount (%) |
|---|---|---|
| 0.0 | 0.001 | -0.004 |
| 5.0 | 0.242 | 0.453 |
| 10.0 | 0.353 | 0.715 |
| 15.0 | 0.457 | 0.916 |
| 20.0 | 0.556 | 1.067 |
| 25.0 | 0.651 | 1.194 |
| 30.0 | 0.741 | 1.311 |
| 35.0 | 0.831 | 1.412 |
| 40.0 | 0.922 | 1.510 |
| 45.0 | 1.003 | 1.601 |
| 50.0 | 1.096 | 1.688 |
| 55.0 | 1.186 | 1.776 |
| 60.0 | 1.285 | 1.870 |
| 65.0 | 1.382 | 1.964 |
| 70.0 | 1.498 | 2.062 |
| 75.0 | 1.612 | 2.166 |
| 80.0 | 1.744 | 2.278 |
| 85.0 | 1.915 | 2.398 |
| 90.0 | 2.168 | 2.522 |
| 95.0 | 2.597 | 2.597 |

Example 24

The result of the Raman spectrum of the amorphous substance of the compound represented by Formula (II) (Measurement conditions 2) is shown in Figs. 36 to 38. The absorption peaks are shown below.

[Table 17]

| Raman peak $cm^{-1}$ | Raman peak $cm^{-1}$ | Raman peak $cm^{-1}$ | Raman peak $cm^{-1}$ |
|---|---|---|---|
| 430 | 748 | 1080 | 1490 |
| 452 | 808 | 1118 | 1598 |
| 493 | 851 | 1147 | 1613 |
| 527 | 903 | 1243 | 1645 |
| 607 | 968 | 1272 | 1700 |
| 651 | 998 | 1305 | |
| 669 | 1028 | 1377 | |
| 727 | 1064 | 1426 | |

Test Example 1

Solid stability test

For the solid stability of the crystal of the compound represented by Formula (I) obtained in Example 1 and the crystal of the compound represented by Formula (II) obtained in Example 16, the chemical stability under temperature, temperature and humidity conditions, and light irradiation was evaluated. The proportion (%) of the analogous substance produced by an HPLC method and the change in appearance were evaluated.

(Storage stability test)

About 10 mg of the crystal was accurately weighed in a 2 mL glass container with a polyethylene stopper. After the glass container was closed, the glass container was stored at 40°C or 60°C for 2 weeks, 1 month, 2 months, 3 months, or 6 months. A sample stored at 40°C is referred to as a 40°C hermetically sealed product, and a sample stored at 60°C is referred to as a 60°C hermetically sealed product. In a state where the glass container was opened, the glass container was stored at a relative humidity of 75 ± 5% at 40°C or a relative humidity of 89 ± 5% for 2 weeks, 1 month, 2 months, 3 months or 6 months, or in the presence of silica gel at 40°C for 1 month, 2 months, 3 months or 6 months. A sample stored at 40°C and a relative humidity of 75 ± 5% or 89 ± 5% is referred to as a 40°C and 75% relative humidity (also referred to as 40 ± 2°C/75 ± 5% RH) or 40°C and 89% relative humidity (also referred to as 40 ± 2°C/89% RH) stored product, and a sample stored in the presence of silica gel at 40°C is referred to as a 40°C stored product. The -40°C hermetically sealed product was used as a standard product.

(Light stability test)

The crystal of the compound represented by Formula (I) obtained in Example 1 was subjected to a light stability test. About 10 mg of the crystal was accurately weighed in a glass cell to obtain a thin and uniform state. At 25°C, a D65 lamp light with a cumulative illumination of 1,216,000 lx · hr was irradiated. This sample is referred to as a light irradiated product.

Here, a sample having no change in appearance was defined as (-), and a sample having a slight change in appearance was defined as ( ± ).

In addition, stability (content of analogous substance) was measured by an absolute calibration curve method by an HPLC method under the following conditions using a sample light-shielded with aluminum foil and stored under the same conditions as a standard product.

(Method for measuring amount of analogous substance)

The amount of the analogous substance was measured by an area percentage method by the HPLC method under the following conditions.

HPLC Conditions 1 were used for the crystal and amorphous substance of the compound represented by Formula (I) obtained in Example 1, and HPLC conditions 2 were used for the crystal of the compound represented by Formula (II) obtained in Example 16.

(HPLC Conditions 1)

· Detector: ultraviolet absorptiometer (measurement wavelength: 260 nm)
· Column: YMC-Triart C18 ExRS, S-1.9 pm, 3.0 × 100 mm
· Column temperature: constant temperature at near 40°C
· Mobile phase A: 0.1% formic acid aqueous solution
· Mobile phase B: acetonitrile
· Flow rate: about 0.6 mL/min
. Injection amount: 3 μL
· Sample cooler temperature: about 6°C
· Feed of mobile phase: The mixing ratio of mobile phase A and mobile phase B was changed as shown in Table 18 to control concentration gradient.

[Table 18]

| Time (min) | A (%) | B (%) |
|---|---|---|
| 0.5 | 90 | 10 |
| 19 | 40 | 60 |
| 21 | 10 | 90 |
| 23 | 10 | 90 |
| 23.01 | 90 | 10 |
| 29.5 | 90 | 10 |

(HPLC Conditions 2)

Column: Cortecs (2.7 μm 3.0 × 100 mm)
Column temperature: 40°C
UV detection wavelength: 254 nm
Mobile phase: [A] 0.1% trifluoroacetic acid aqueous solution and [B] acetonitrile were gradient eluted as shown in Table 19.
Flow rate: 0.6 mL/min

[Table 19]

| Time (min) | A(%) | B(%) |
|---|---|---|
| 0 | 70 | 30 |
| 6 | 59 | 41 |
| 11.5 | 59 | 41 |
| 19 | 35 | 65 |
| 21 | 10 | 90 |
| 24 | 10 | 90 |
| 24.05 | 70 | 30 |
| 29.5 | 70 | 30 |

As a result, it was confirmed that the crystal of the compound represented by Formula (I) obtained in Example 1 and the crystal of the compound represented by Formula (II) obtained in Example 16 were crystals excellent in solid stability under any conditions.

The amount (%) of the total analogous substance under representative measurement conditions is described in the following table.

[Table 20]

| | Storage conditions | Storage period | Amount (%) of total analogous substance |
|---|---|---|---|
| Crystal of compound represented by Formula (I) obtained in Example 1 | (Standard product) -40°C hermetically sealed | 1 month | 0.22 |
| | | 2 months | 0.22 |
| | 60°C hermetically sealed | 2 weeks | 0.23 |
| | | 1 month | 0.24 |
| | 40°C hermetically sealed | 2 weeks | 0.22 |
| | | 1 month | 0.22 |
| | | 2 months | 0.23 |
| | 40°C Relative humidity 75% | 2 months | 0.27 |

[Table 21]

| | Storage conditions | Storage period | Amount (%) of total analogous substance |
|---|---|---|---|
| Crystal of compound represented by Formula (II) obtained in Example 16 | (Standard product) -40°C hermetically sealed | 1 month | 0.61 |
| | | 2 months | 0.62 |
| | 60°C hermetically sealed | 2 weeks | 0.76 |
| | | 1 month | 0.85 |
| | 40°C hermetically sealed | 2 weeks | 0.64 |
| | | 1 month | 0.65 |
| | | 2 months | 0.66 |
| | 40°C Relative humidity 75% | 2 months | 0.68 |

Test Example 2

The crystal and amorphous substance of the compound represented by Formula (I) obtained in Example 1 were stored (40°C, 89%RH 3M) in a hermetically sealed container at 40°C and a relative humidity of 89% for 3 months, and subjected to a solid stability test to evaluate the amount of an analogous substance.

(Preparation of sample solution)

A sample solution was prepared by the following method.
The crystal or amorphous substance of the compound represented by Formula (I) obtained in Example 1 was dissolved in an acetonitrile aqueous solution (acetonitrile: water = 1: 1) to prepare a sample solution to be exactly 500 μg/mL.

(Method for measuring amount of analogous substance)

The purity and the amount of the analogous substance were measured by an area percentage method under the HPLC conditions 1 described in Test Example 1.

(Results)

The results of the crystal and amorphous substance of the compound represented by Formula (I) obtained in Example 1 are described in the following table. Impurities detected at a retention time (RT, retention time) of 17.527 minutes indicate an increase amount with respect to a standard product.
In the amorphous substance of the compound represented by Formula (I) obtained in Example 1, the impurities detected at a retention time (RT, retention time) of 17.527 minutes greatly increased, but the increase in impurities could be

greatly suppressed in the crystal.

[Table 22]

| | Storage conditions | Purity | Increase amount of impurities |
|---|---|---|---|
| Crystal | Standard product | 99.71 | 0.12 |
| | 40°C89%RH 3M | 99.59 | |
| Amorphous substance | Standard product | 99.48 | 0.58 |
| | 40°C89%RH 3M | 98.90 | |

Test Example 3:

(Powder solubility test)

The solubility of the crystal of the present invention was evaluated with respect to the solubility when JP-1 solution, JP-2 solution, and JP-2 solution added with taurocholic acid (TCA/JP2) were used as a measurement medium. Evaluation was performed at a dissolved concentration (μg/mL) by an HPLC method.

About 1 mg of the crystal of the present invention was weighed in a 4 mL glass container, and 2 mL of JP-2 solution (1000 mL of water was added to 1.70 g of potassium dihydrogen phosphate and 1.775 g of anhydrous disodium hydrogen phosphate) was added to each container. The mixture was sealed with a polyethylene stopper with Teflon and silicone packing, shaken at 37°C for 1 hour, and then filtered, and 400 μL of methanol was added to 800 μL of the filtrate to perform 1.5 fold dilution. Whether there were bubbles and precipitates was confirmed, and the compound of the crystal of the present invention was quantified using HPLC by an absolute calibration curve method to calculate the solubility.

The solubility of each compound crystal for I-236, I-275, and II-168 described in Patent Documents 1 and 2 is also shown in the following table.

(Results)

[Table 23]

| Crystal | JP1_μg/mL | JP2_μg/mL | TCA/JP2_μg/mL |
|---|---|---|---|
| Compound crystal of I-236 | 0.94 | 1.03 | 10.5 |
| Compound crystal of I-275 | 4.36 | 3.35 | 16.9 |
| Compound crystal of II-168 | 1.43 | 1.87 | 14.9 |
| Compound crystal of Formula (I) | 59.4 | 74.1 | 143.1 |
| Compound crystal of Formula (II) | 33.6 | 37.5 | 73.6 |

The crystal of the present invention was confirmed to have high solubility. Usually, the solubility decreases as the compound changes from amorphous to crystalline. It was confirmed that the crystal of the present invention has better solubility than the crystals of Example Compounds I-236, 1-275, II-168 and the like described in Patent Documents 1 and 2.

Test Example 4:

(Solubility test under DMSO addition conditions)

Solubility was determined under 1%DMSO addition conditions.

The solution of the compound (2 μL) was added to 198 μL each of a JP-1 solution and a JP-2 solution. The mixture was shaken for 1 hour or more at a room temperature, and the mixed liquids were filtered. The filtrates were diluted 10- or 100-fold with methanol/water = 1/1 (V/V) or acetonitrile/methanol/water = 1/1/2 (V/V/V), and concentrations in the filtrates were measured by an absolute calibration curve method using LC/MS/MS or solid-phase extraction (SPE)/MS.

The composition of the JP-1 solution is as follows.

Water was added to 2.0 g of sodium chloride and 7.0 mL of hydrochloric acid to make 1000 mL.

The composition of the JP-2 solution is as follows.

3.40 g of potassium dihydrogen phosphate and 3.55 g of dibasic sodium phosphate anhydrous were dissolved in

water to make 1000 mL, and to 1 volume of the resultant, 1 volume of water was added.

Compounds of the present invention and Compounds of I-236, 1-275 and II-168 described in Patent Documents 1 and 2 were tested as described above.

(Results)

The solubility under the DMSO addition conditions was 50 μmol/L in all cases, and a good result was shown.

Test Example 5

(Residual solvent)

With respect to the crystal and amorphous substance of the compound represented by Formula (I) obtained in Example 1, residual solvents were evaluated by $^1$H-NMR or gas chromatography.

The amorphous substance of the compound represented by Formula (I) obtained in Example 1 was subjected to NMR measurement. As a result, 3.66% by weight of hexane was detected.

(Amount of residual solvent listed in the ICH Q3C guideline)

Hexane is a solvent whose residual amount in a pharmaceutical product should be regulated (class 2). Therefore, it is necessary to adjust the residual amount of hexane in the active pharmaceutical ingredient to a specified value or less. Table below shows solvents of class 2 listed in the ICH Q3C guideline.

[Table 24]

| Solvent | PDE (mg/day) | Concentration limit value (ppm) |
|---|---|---|
| Acetonitrile | 4.1 | 410 |
| Chlorobenzene | 3.6 | 360 |
| Chloroform | 0.6 | 60 |
| Cyclohexane | 38.8 | 3830 |
| 1,2-Dichloroethene | 18.7 | 1870 |
| Dichloromethane | 6.0 | 600 |
| 1,2-Dimethoxyethane | 1.0 | 100 |
| N,N-Dimethylacetamide | 10.9 | 1090 |
| N,N-Dimethylformamide | 8.8 | 880 |
| 1,4-Dioxane | 3.8 | 380 |
| 2- Ethoxyethanol | 1.6 | 160 |
| Ethylene glycol | 6.2 | 620 |
| Formamide | 2.2 | 220 |
| Hexane | 2.9 | 290 |
| Methanol | 30.0 | 3000 |
| 2-Methoxyethanol | 0.5 | 50 |
| Methyl butyl ketone | 0.5 | 50 |
| Methylcyclohexane | 11.8 | 1180 |
| N-Methylpyrrolidone | 48.4 | 4840 |
| Nitromethane | 0. 5 | 50 |
| Pyridine | 2.0 | 200 |
| Sulfolane | 1.6 | 160 |
| Tetralin | 1.0 | 100 |
| Toluene | 8.9 | 890 |
| 1,1,2-Trichloroethene | 0.8 | 80 |
| Xylene* | 21.7 | 2170 |

Since the amorphous substance of the compound represented by Formula (I) obtained in Example 1 exceeded

the concentration limit value of hexane, it was found that the amorphous substance of the compound represented by Formula (I) obtained in Example 1 is not suitable as an active pharmaceutical ingredient.

The crystal of the compound represented by Formula (I) obtained in Example 1 was subjected to gas chromatography measurement. As a result, a residual solvent in an amount detectable by NMR was not confirmed. The crystal of the compound represented by Formula (I) obtained in Example 1 was found to be excellent as an active pharmaceutical ingredient.

The preparation examples shown below are only for illustrative purposes and are by no means intended to limit the scope of the invention.

The compound of the present invention can be administered as a pharmaceutical composition by any conventional route, particularly enterally, for example, orally, for example, in the form of a tablet or a capsule; parenterally, for example, in the form of an injectable preparation or a suspension; and topically, for example, in the form of a lotion, a gel, an ointment or a cream, or as a pharmaceutical composition in a transnasal form or a suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets, granules, or capsules containing excipients, disintegrants, binders, lubricants and the like and active ingredients. Compositions for injection can be solutions or suspension, may be sterilized, and may contain preservatives, stabilizers, buffering agents, and the like.

[INDUSTRIAL APPLICABILITY]

The crystal of the present invention is useful as an active pharmaceutical ingredient. In addition, the pharmaceutical composition containing a crystal of the present invention is very useful as a therapeutic agent or a prophylactic agent for a disease involving MGAT2.

## Claims

1. An anhydride crystal of the compound represented by Formula (I):

[Chemical Formula 1]

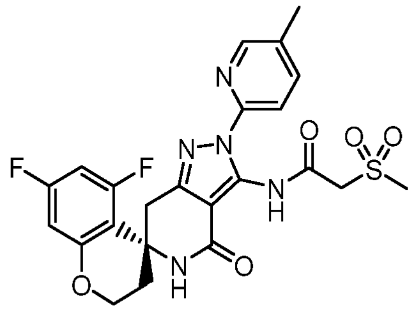

(I)

and/or a hydrate crystal of the compound represented by Formula (I) having a water content corresponding to 0.25 hydrates or less.

2. The hydrate crystal according to claim 1, wherein the hydrate crystal has a water content corresponding to less than 0.25 hydrates.

3. The hydrate crystal according to claim 2, which exhibits a powder X-ray diffraction pattern having peaks at diffraction angles (2θ): 13.0° ± 0.2°, 18.4° ± 0.2°, 20.0° ± 0.2°, 20.4° ± 0.2°, and 23.7° ± 0.2°.

4. The hydrate crystal according to claim 3, which exhibits a powder X-ray diffraction pattern having peaks at diffraction angles (2θ): 13.0° ± 0.2°, 15.5° ± 0.2°, 16.6° ± 0.2°, 16.9° ± 0.2°, 17.4° ± 0.2°, 18.4° ± 0.2°, 19.3° ± 0.2°, 20.0° ± 0.2°, 20.4° ± 0.2°, 21.4° ± 0.2°, 23.7° ± 0.2°, and 24.1° ± 0.2°.

5. The 0.25 hydrate crystal according to claim 1.

6. The 0.25 hydrate crystal according to claim 5, which exhibits a powder X-ray diffraction pattern having peaks at diffraction angles (2θ): 13.0° ± 0.2°, 18.4° ± 0.2°, 19.9° ± 0.2°, 20.5° ± 0.2°, and 23.7° ± 0.2°.

7. The 0.25 hydrate crystal according to claim 6, which exhibits a powder X-ray diffraction pattern having peaks at diffraction angles (2θ): 13.0° ± 0.2°, 15.5° ± 0.2°, 16.6° ± 0.2°, 16.9° ± 0.2°, 17.5° ± 0.2°, 18.4° ± 0.2°, 19.2° ± 0.2°, 19.9° ± 0.2°, 20.5° ± 0.2°, 21.5° ± 0.2°, 23.7° ± 0.2°, and 24.2° ± 0.2°.

8. The anhydride crystal according to claim 1, which exhibits a powder X-ray diffraction pattern having peaks at diffraction angles (2θ): 13.1° ± 0.2°, 18.3° ± 0.2°, 20.2° ± 0.2°, 20.5° ± 0.2°, and 23.8° ± 0.2°.

9. The anhydride crystal according to claim 8, which exhibits a powder X-ray diffraction pattern having peaks at diffraction angles (2θ): 13.1° ± 0.2°, 15.6° ± 0.2°, 16.7° ± 0.2°, 17.1° ± 0.2°, 18.3° ± 0.2°, 19.5° ± 0.2°, 20.2° ± 0.2°, 20.5° ± 0.2°, 21.5° ± 0.2°, and 23.8° ± 0.2°.

10. The crystal according to claim 1, wherein

when the crystal is allowed to stand in an environment of a temperature of 25°C and a relative humidity of 35% and a powder X-ray diffraction experiment is performed in the same environment, the crystal has peaks in the diffraction angles as defined in claim 3 or 4,
when the crystal is allowed to stand in an environment of a temperature of 25°C and a relative humidity of 60% and a powder X-ray diffraction experiment is performed in the same environment, the crystal has peaks in the diffraction angles as defined in claim 6 or 7,
or
when the crystal is allowed to stand in an environment of a temperature of 50°C and a relative humidity of 25% and a powder X-ray diffraction experiment is performed in the same environment, the crystal has peaks in the diffraction angles as defined in claim 8 or 9.

11. The crystal according to claim 1, which has a melting point of 236 to 237°C.

12. A hydrate crystal of the compound represented by Formula (I):

[Chemical Formula 2]

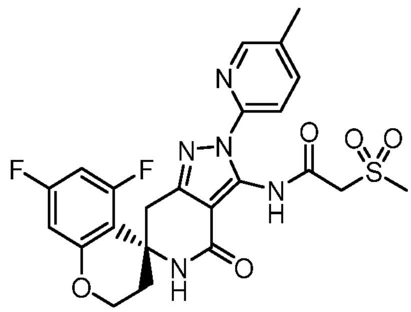

(I)

wherein water content Y (% by weight) is 0.0 < Y ≤ 1.5% by weight.

13. The crystal according to any of claims 1 to 12, having absorption peaks at 1448 $cm^{-1}$ ± 2 $cm^{-1}$, 1511 $cm^{-1}$ ± 2 $cm^{-1}$, and 2885 $cm^{-1}$ ± 2 $cm^{-1}$ in Raman spectrum.

14. A crystal of the compound represented by Formula (II):

[Chemical Formula 3]

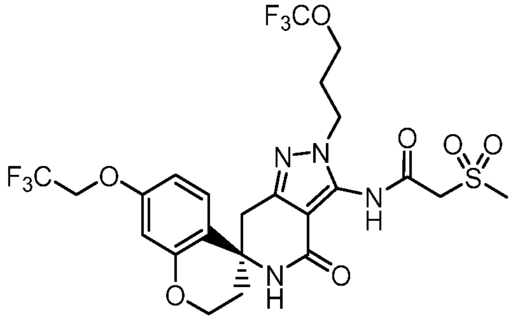

(II)

**15.** The crystal according to claim 14, which exhibits a powder X-ray diffraction pattern having peaks at diffraction angles (2θ): 16.3° ± 0.2°, 18.4° ± 0.2°, 20.4° ± 0.2°, 22.8° ± 0.2°, and 23.9° ± 0.2°.

**16.** The crystal according to claim 15, which exhibits a powder X-ray diffraction pattern having peaks at diffraction angles (2θ): 15.1° ± 0.2°, 16.3° ± 0.2°, 18.4° ± 0.2°, 19.2° ± 0.2°, 20.4° ± 0.2°, 21.1° ± 0.2°, 22.0° ± 0.2°, 22.8° ± 0.2°, 23.3° ± 0.2°, and 23.9° ± 0.2°.

**17.** The crystal according to any of claims 14 to 16, having absorption peaks at 577 $cm^{-1}$ ± 2 $cm^{-1}$, 612 $cm^{-1}$ ± 2 $cm^{-1}$, 740 $cm^{-1}$ ± 2 $cm^{-1}$, 1571 $cm^{-1}$ ± 2 $cm^{-1}$, and 1718 $cm^{-1}$ ± 2 $cm^{-1}$ in Raman spectrum.

[Fig. 1]

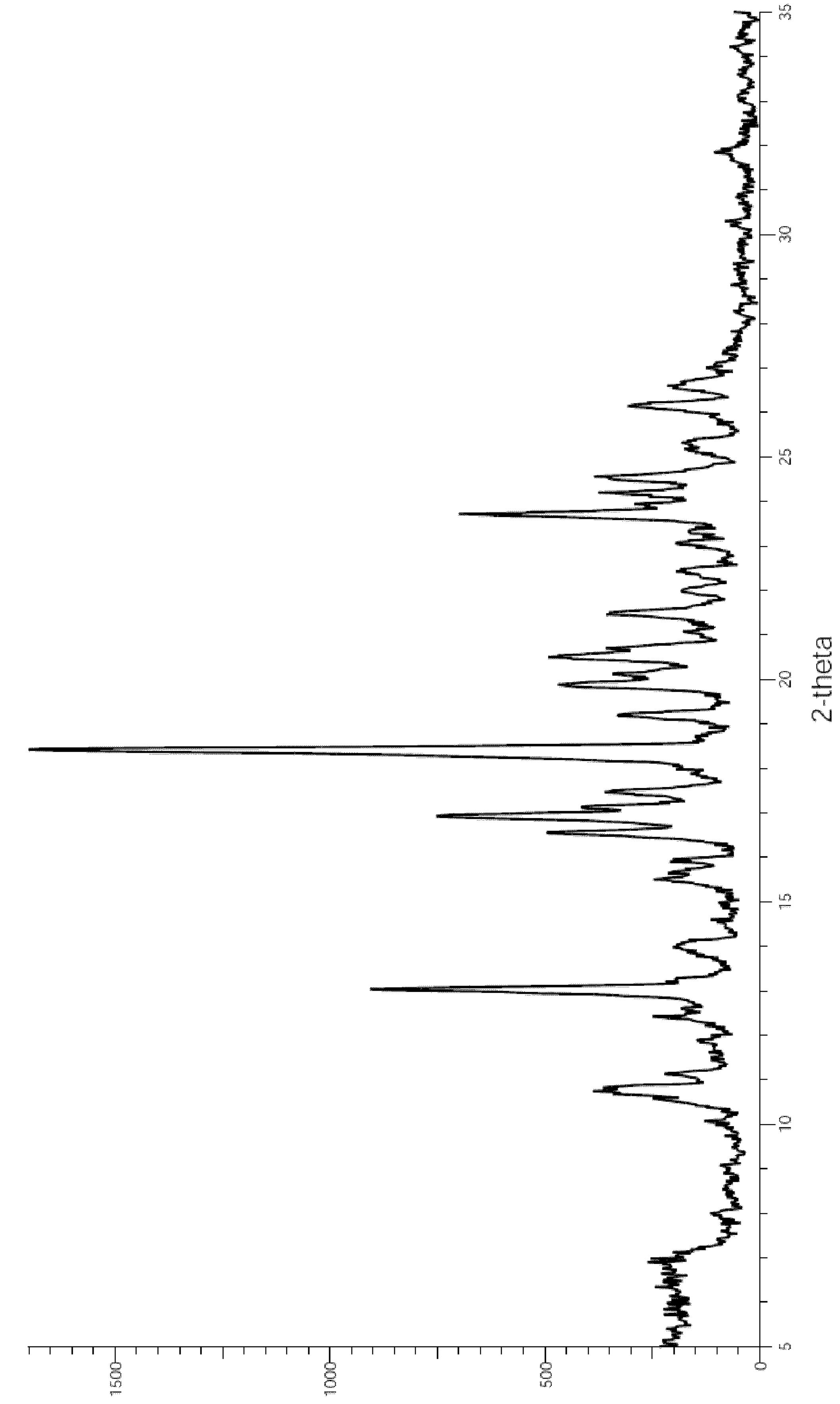

[Fig. 2]

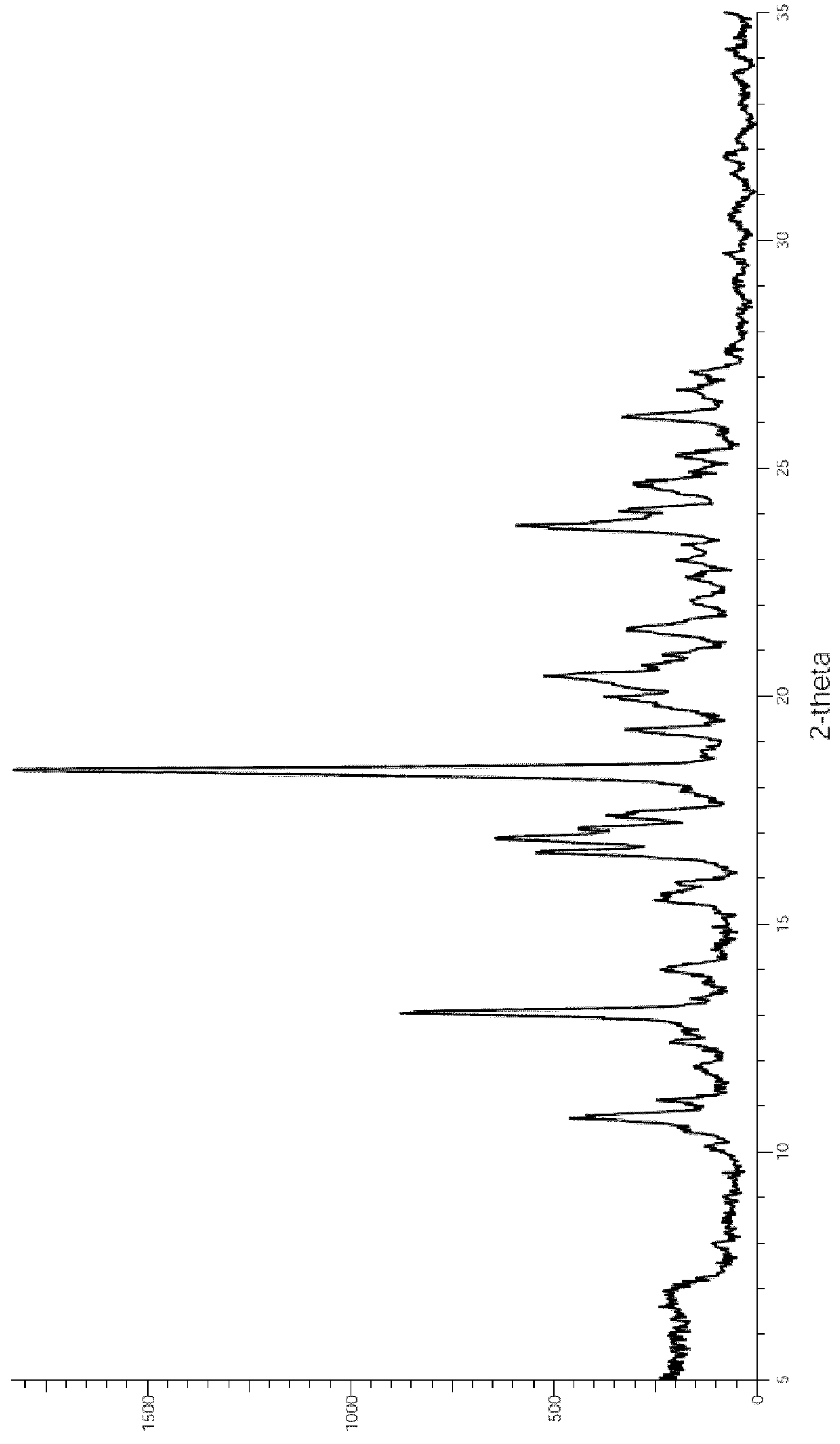

[Fig. 3]

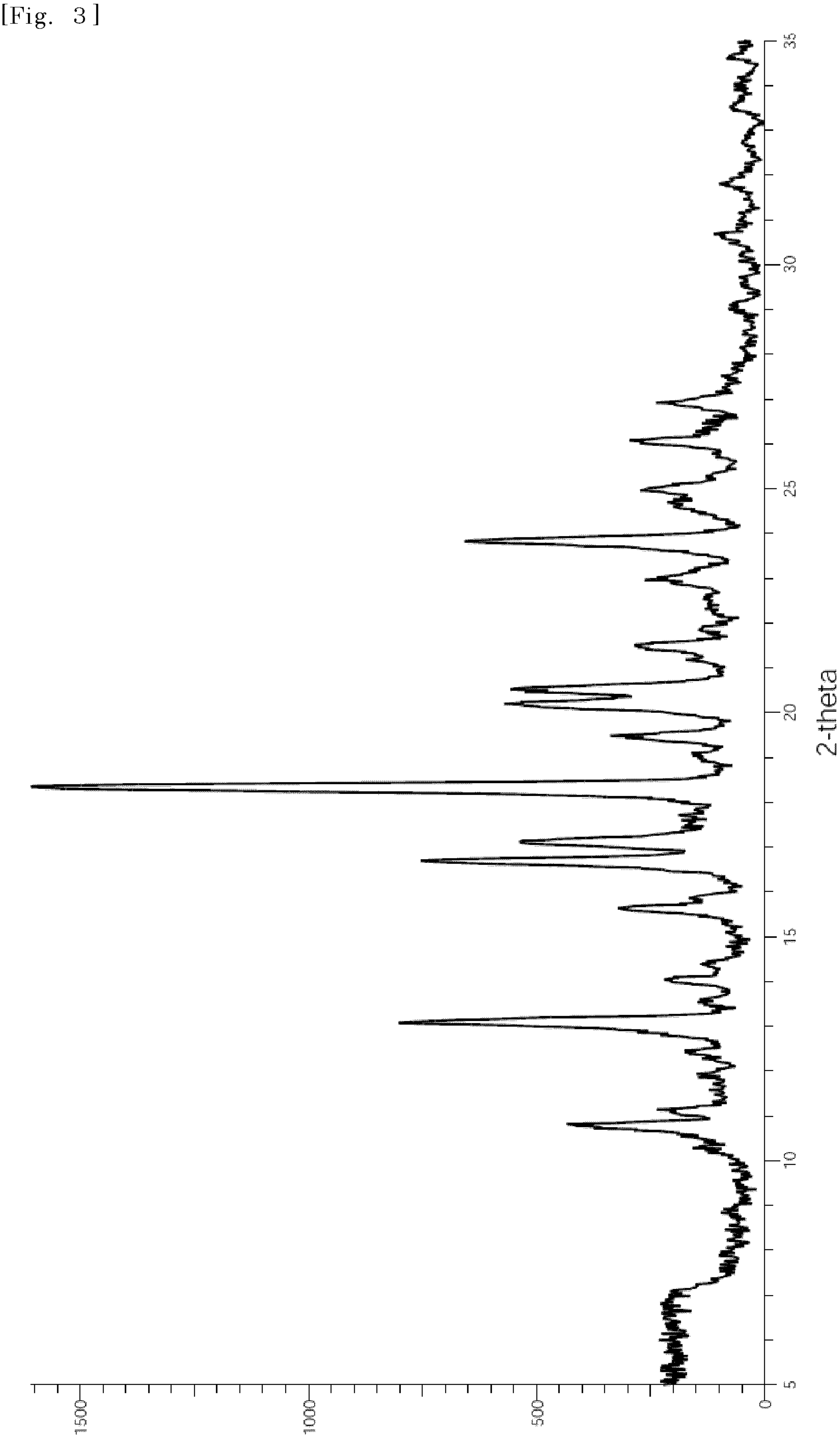

[Fig. 4]

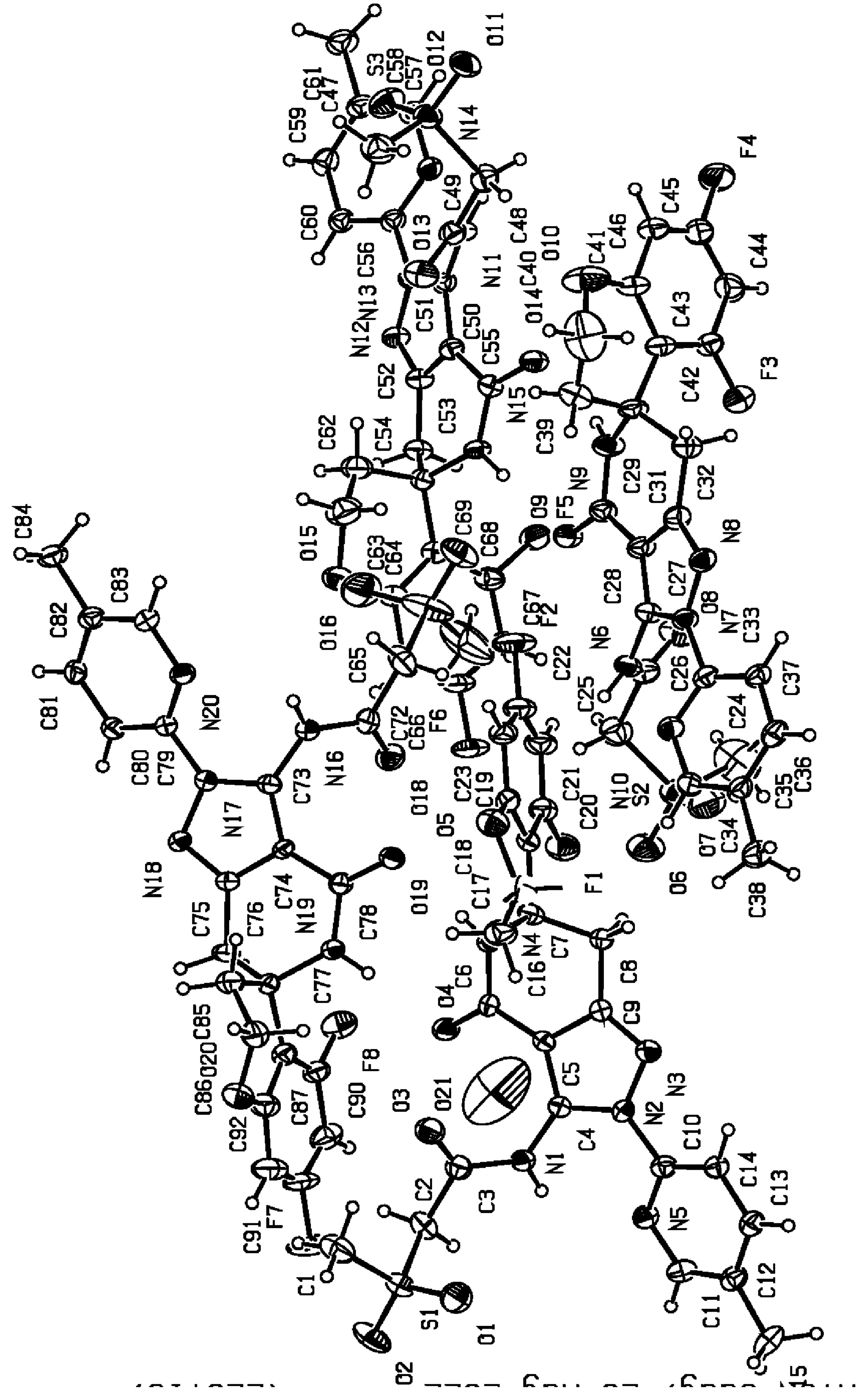

[Fig. 5]

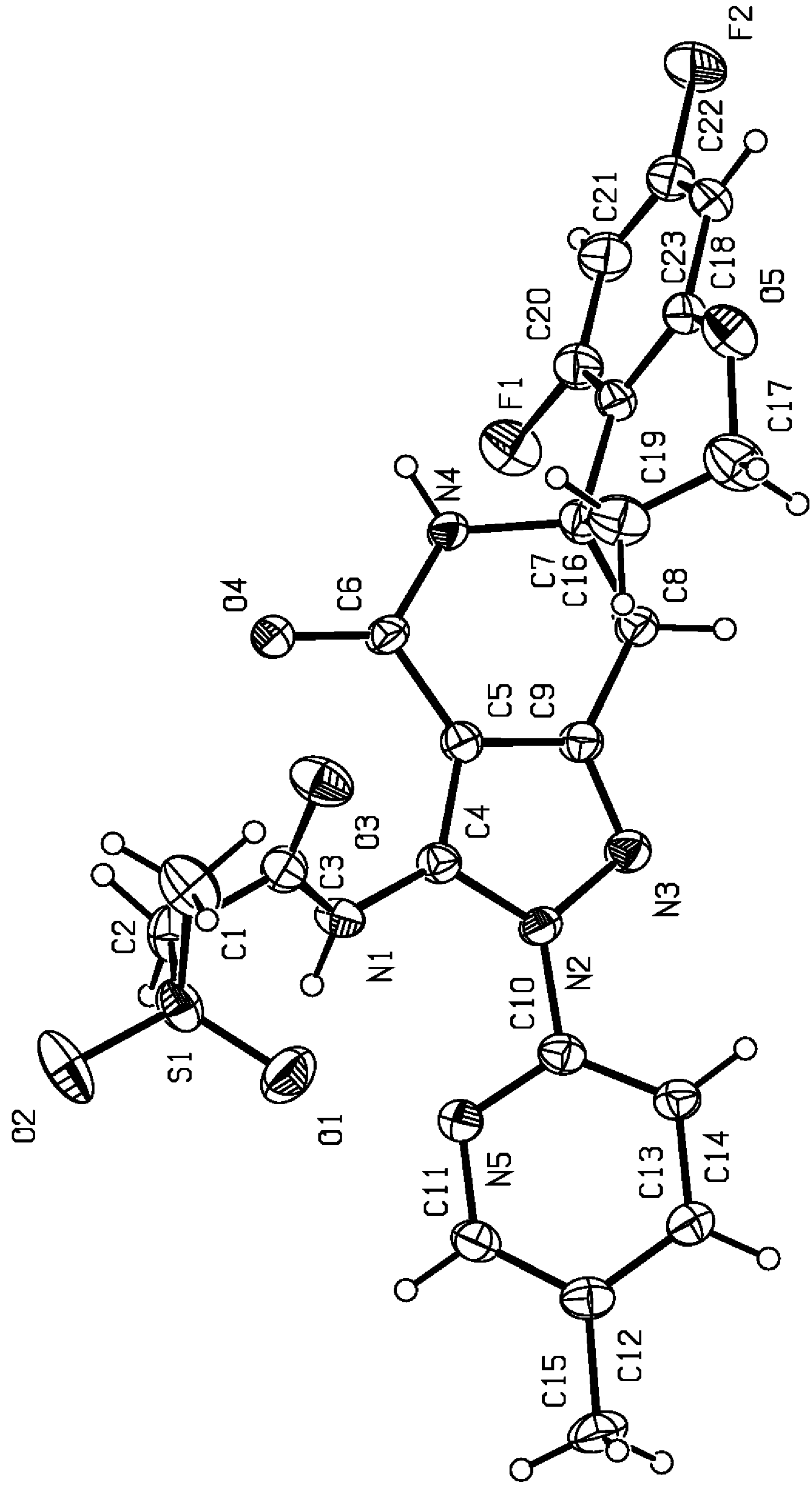

[Fig. 6]

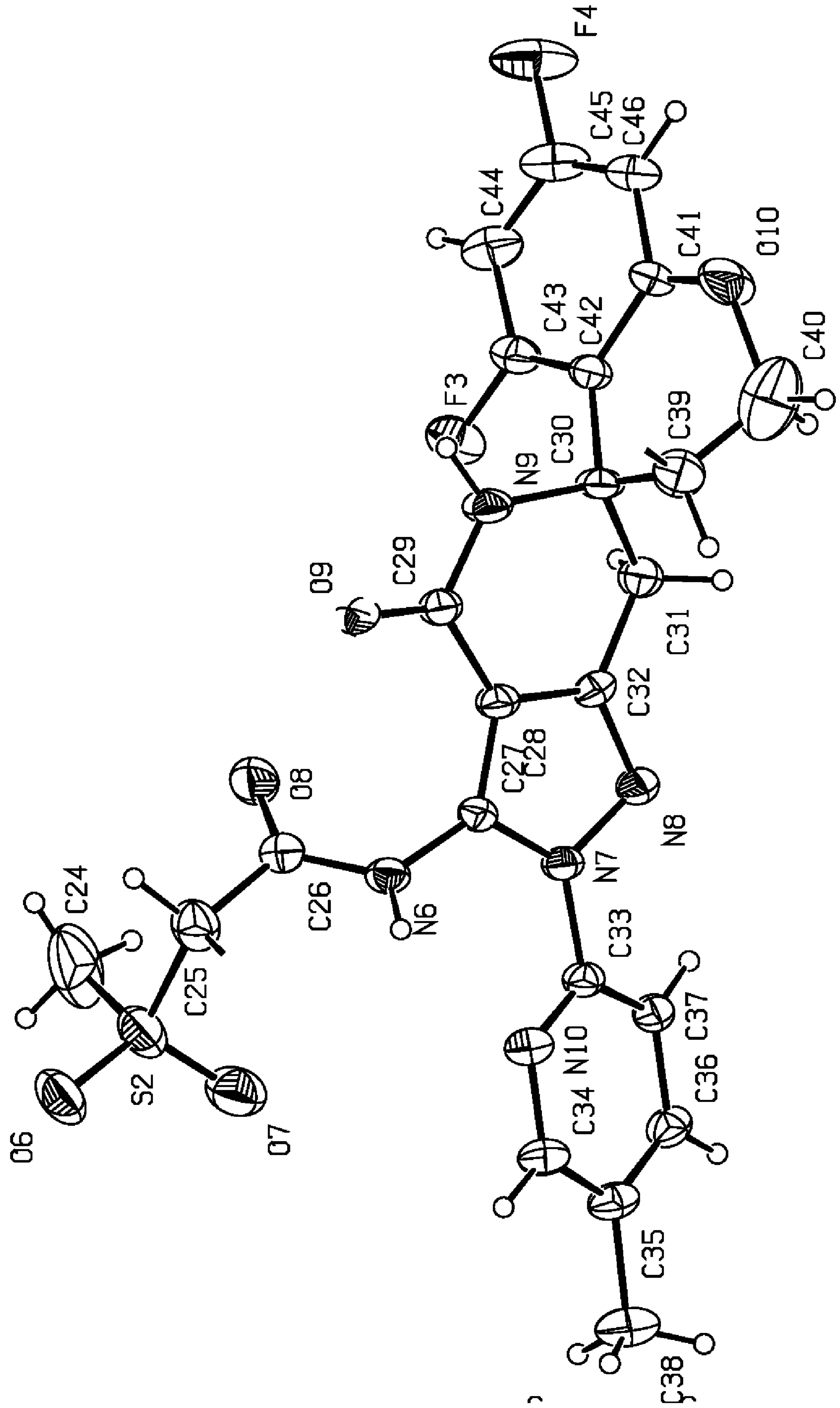

[Fig. 7]

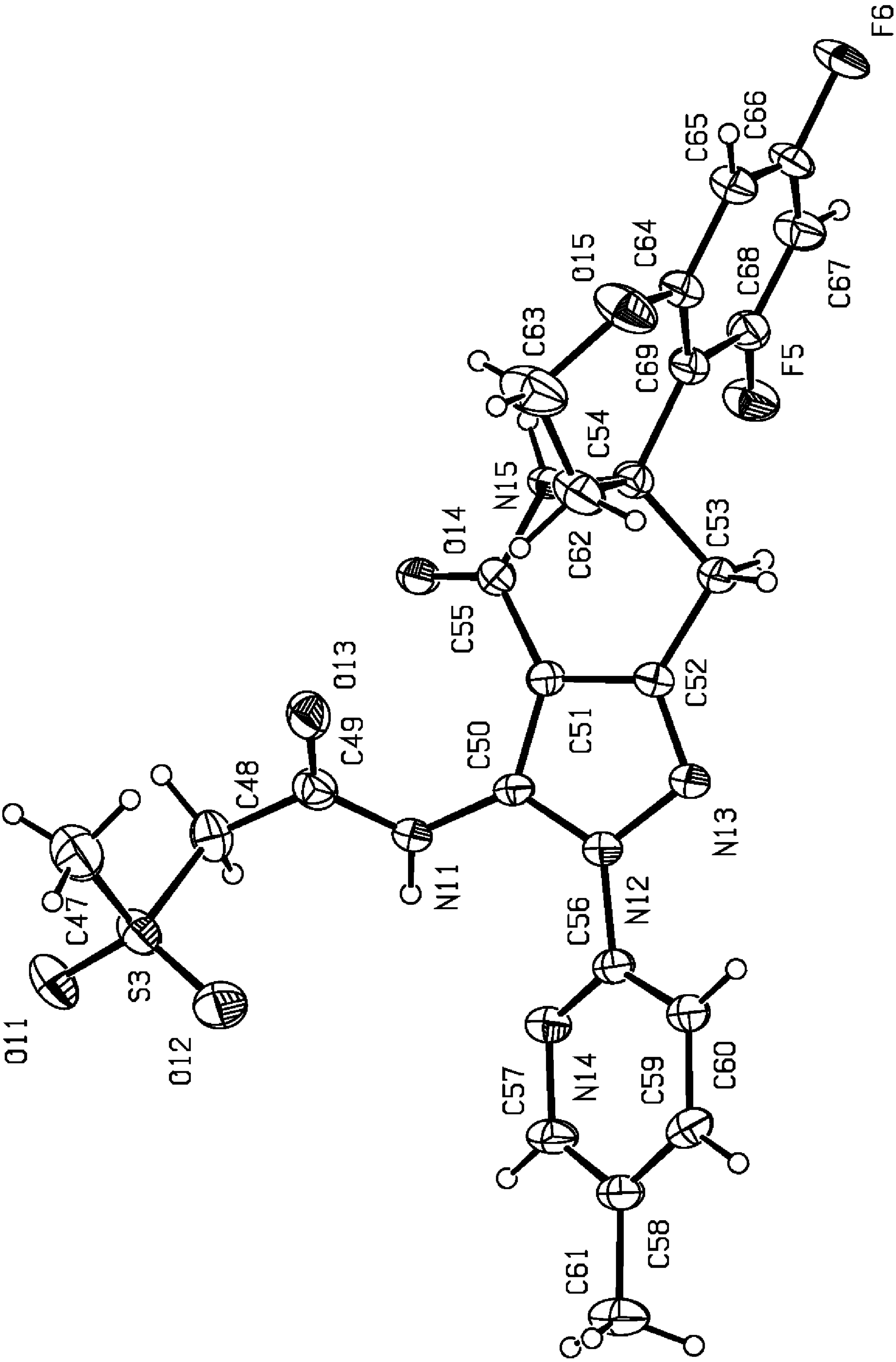

[Fig. 8]

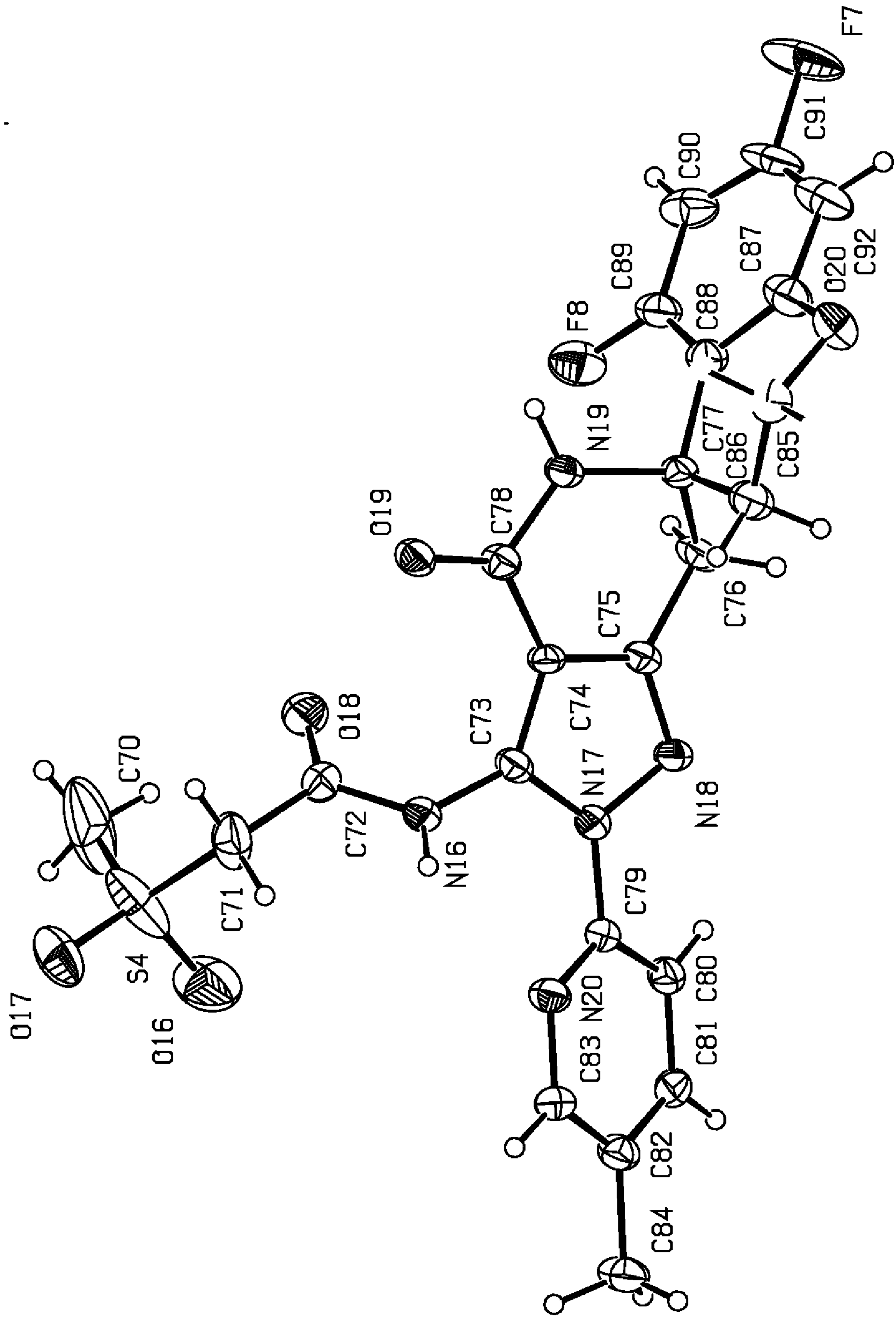

[Fig. 9]

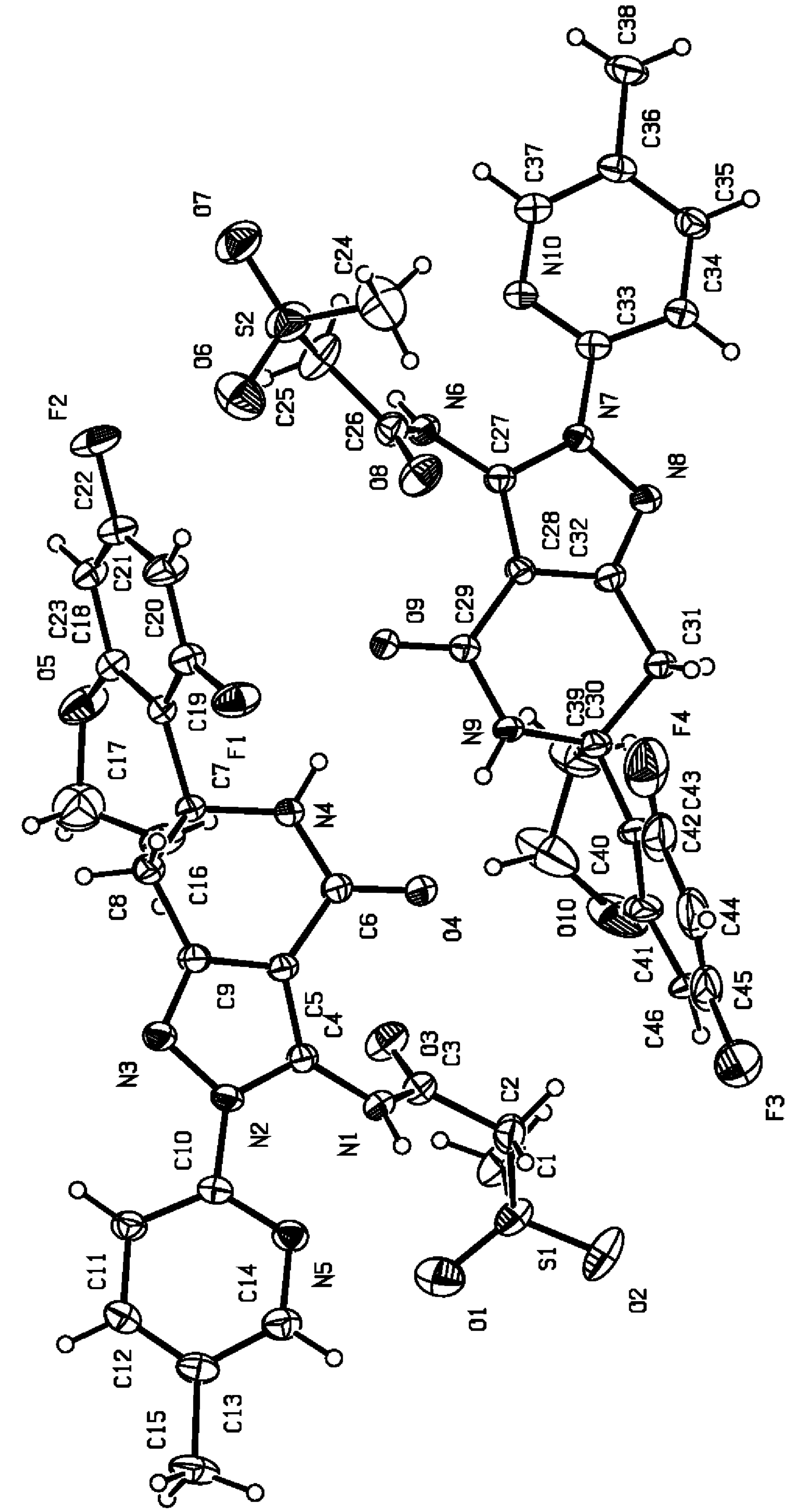

[Fig. 1 0]

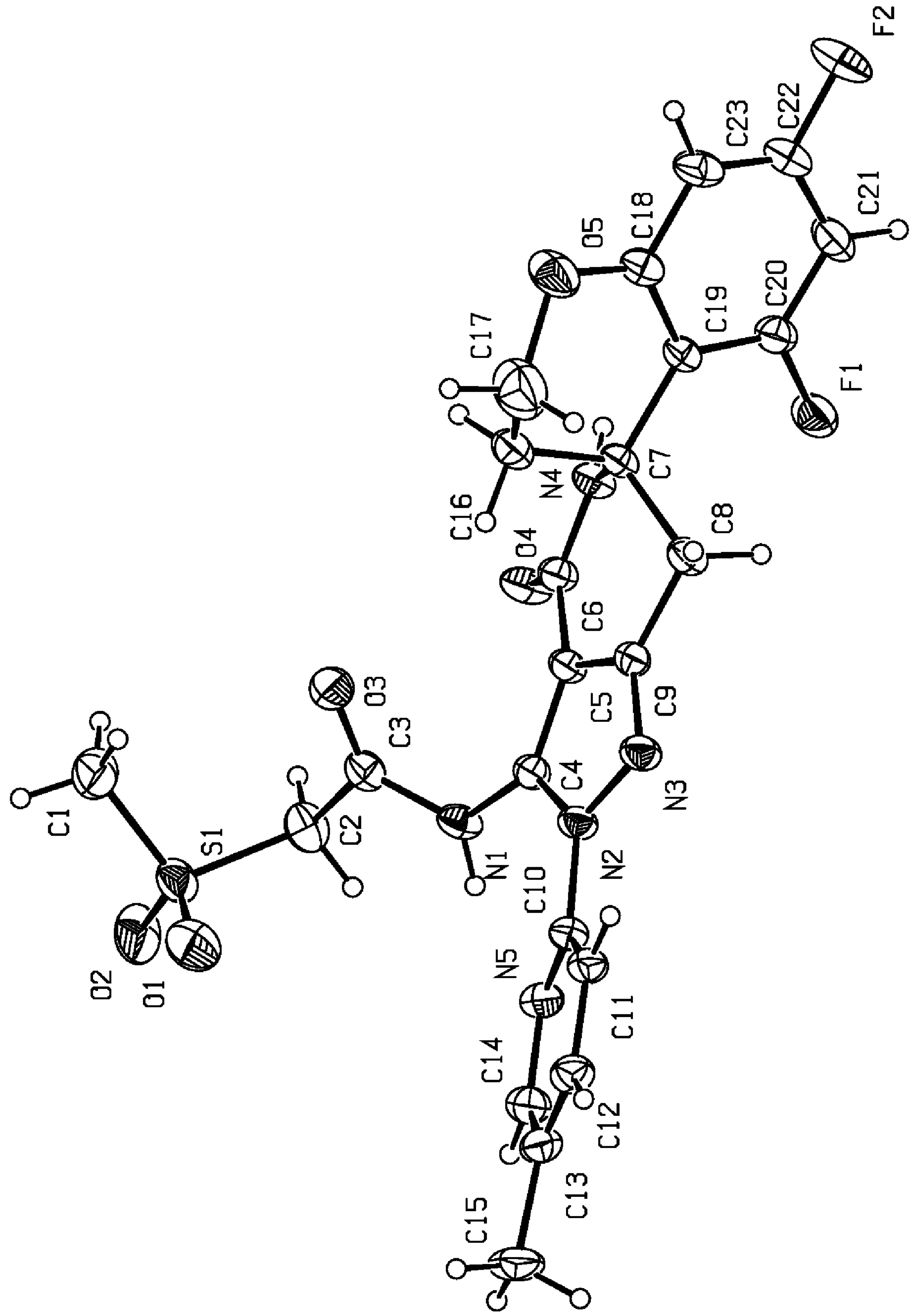

[Fig. 1 1]

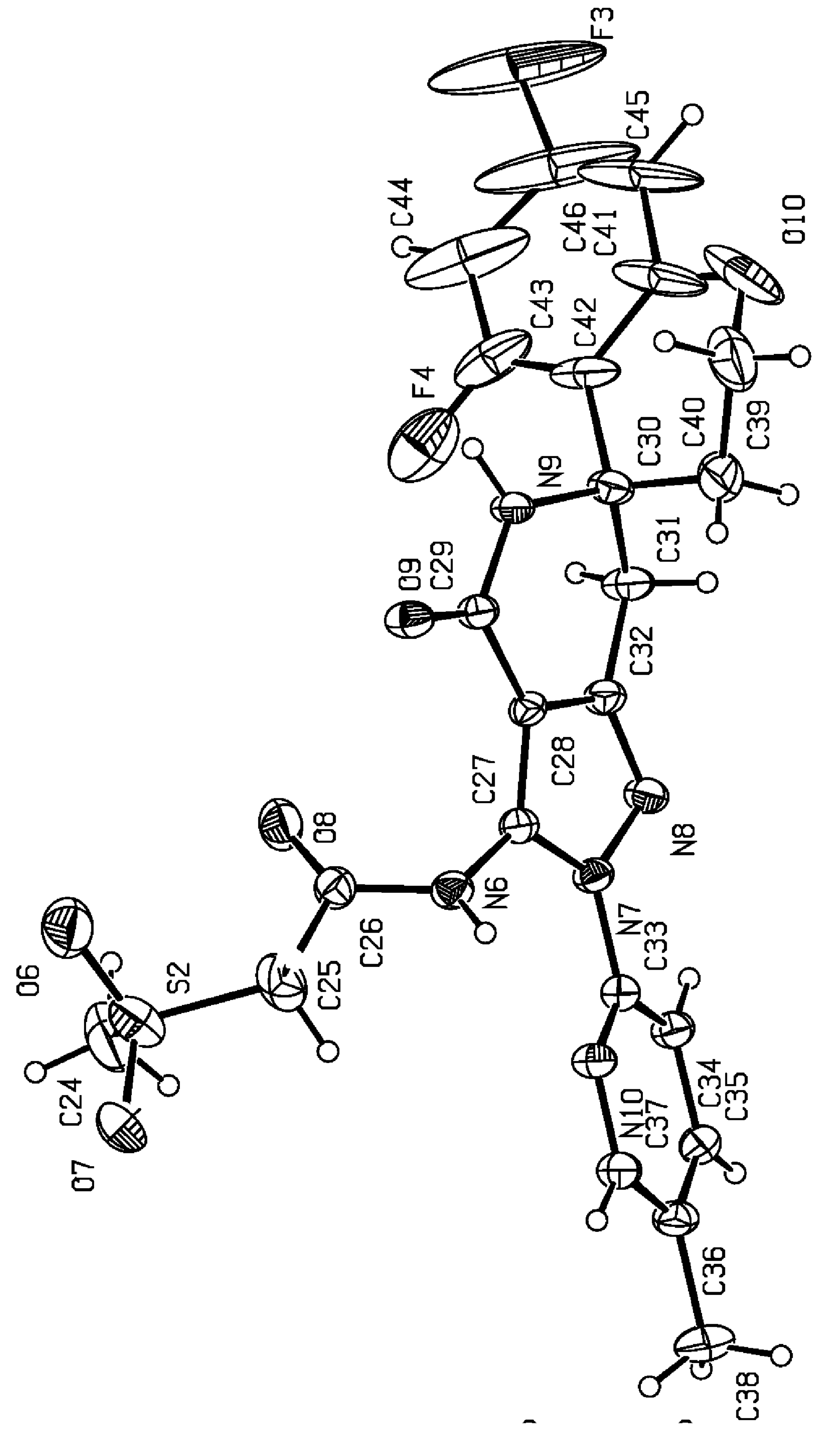

[Fig. 1 2]

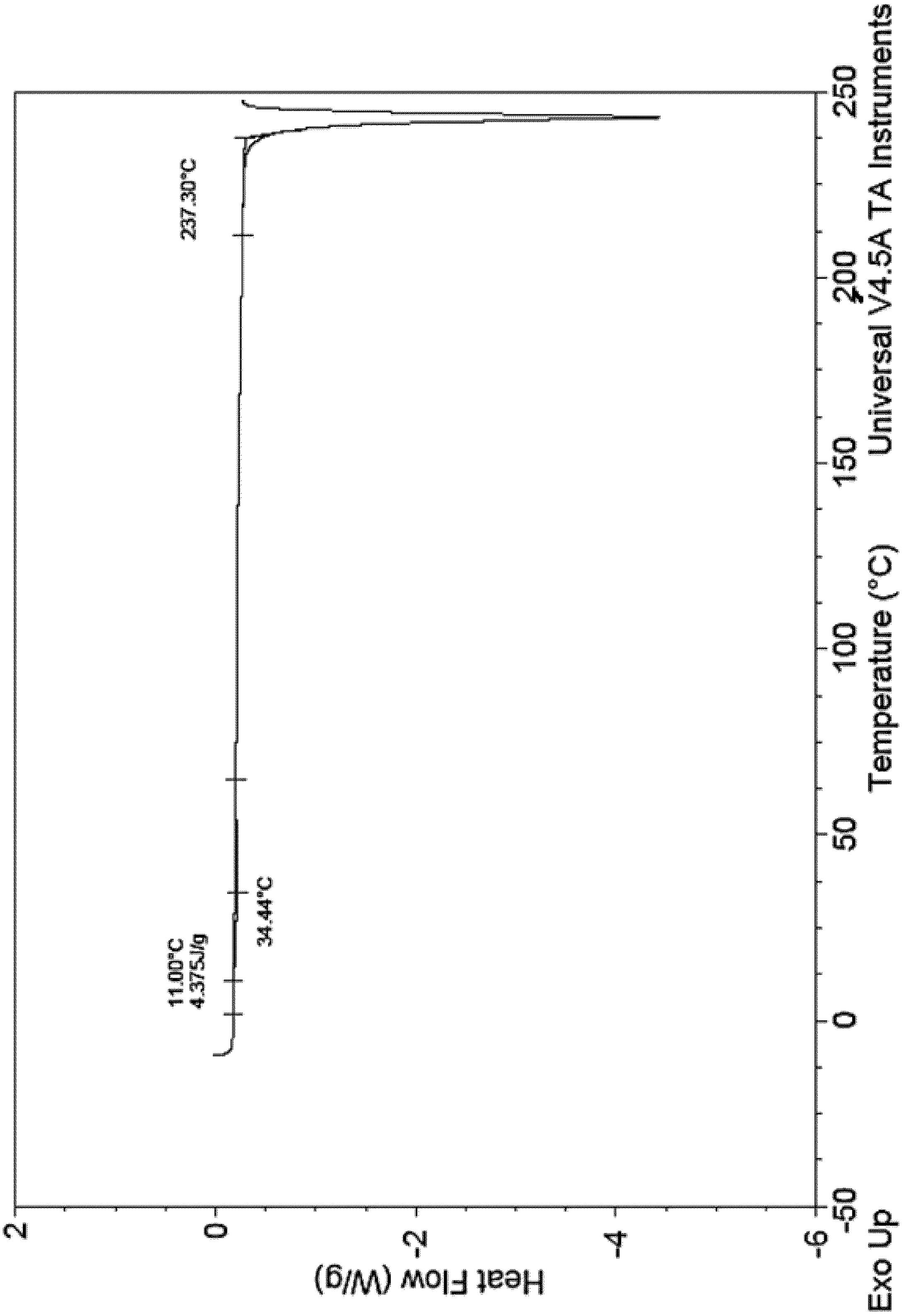
237.30°C
11.00°C
4.375J/g
34.44°C
2
0
-2
-4
-6
Heat Flow (W/g)
-50
0
50
100
150
200
250
Temperature (°C)
Exo Up
Universal V4.5A TA Instruments

[Fig. 1 3]

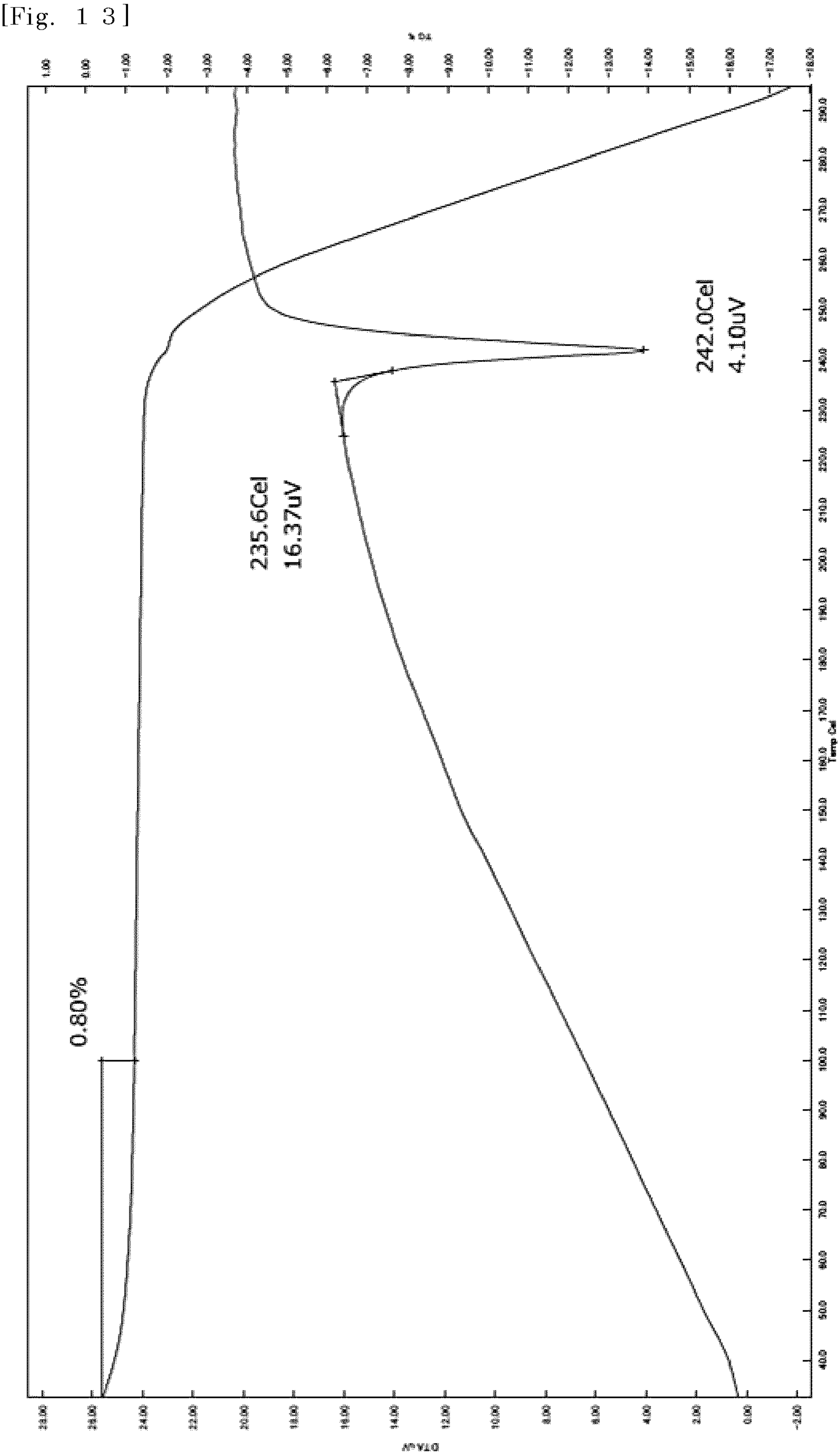

[Fig. 14]

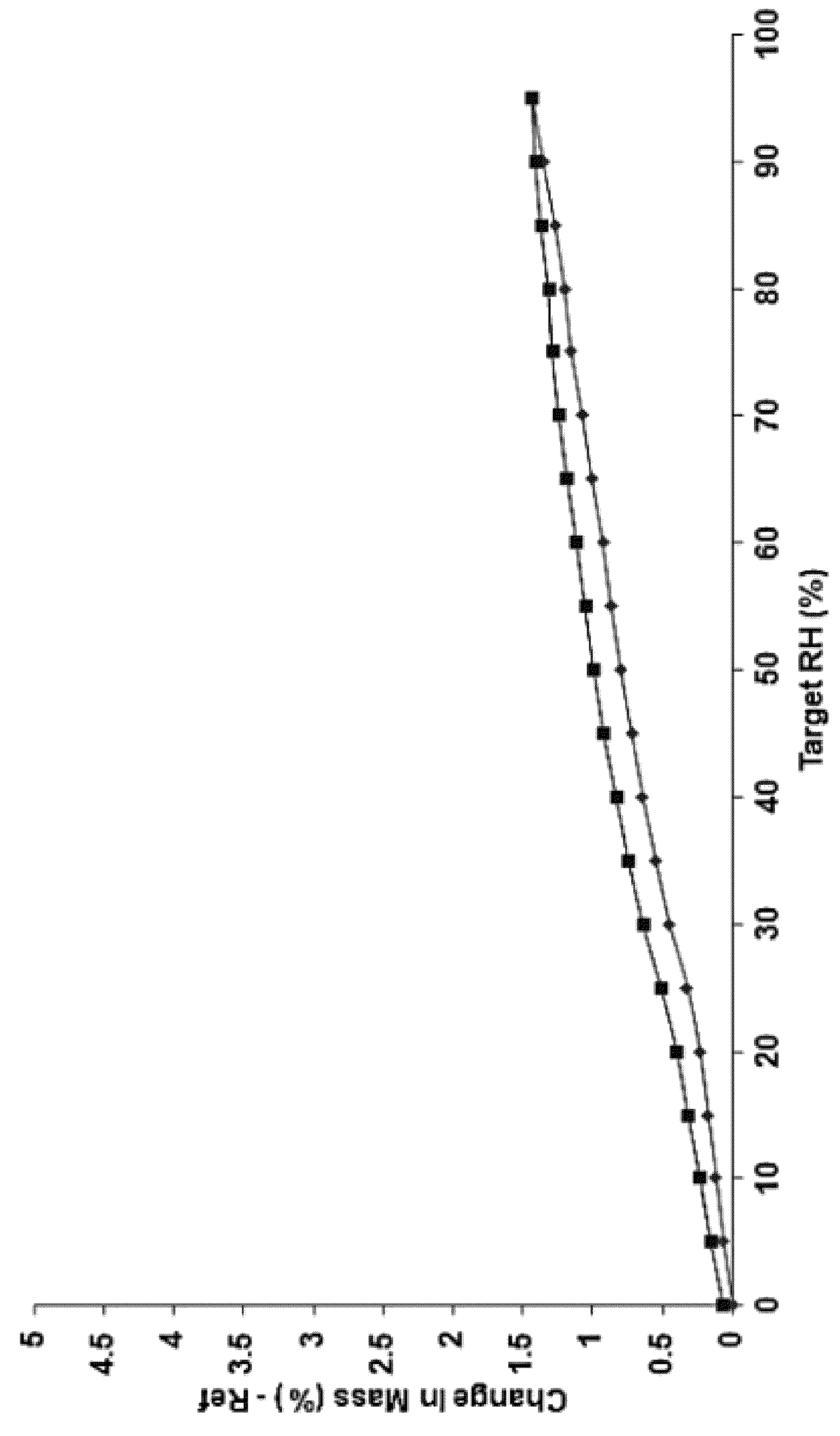
Change in Mass (%) - Ref
Target RH (%)
0
0.5
1
1.5
2
2.5
3
3.5
4
4.5
5
0
10
20
30
40
50
60
70
80
90
100

[Fig. 1 5]

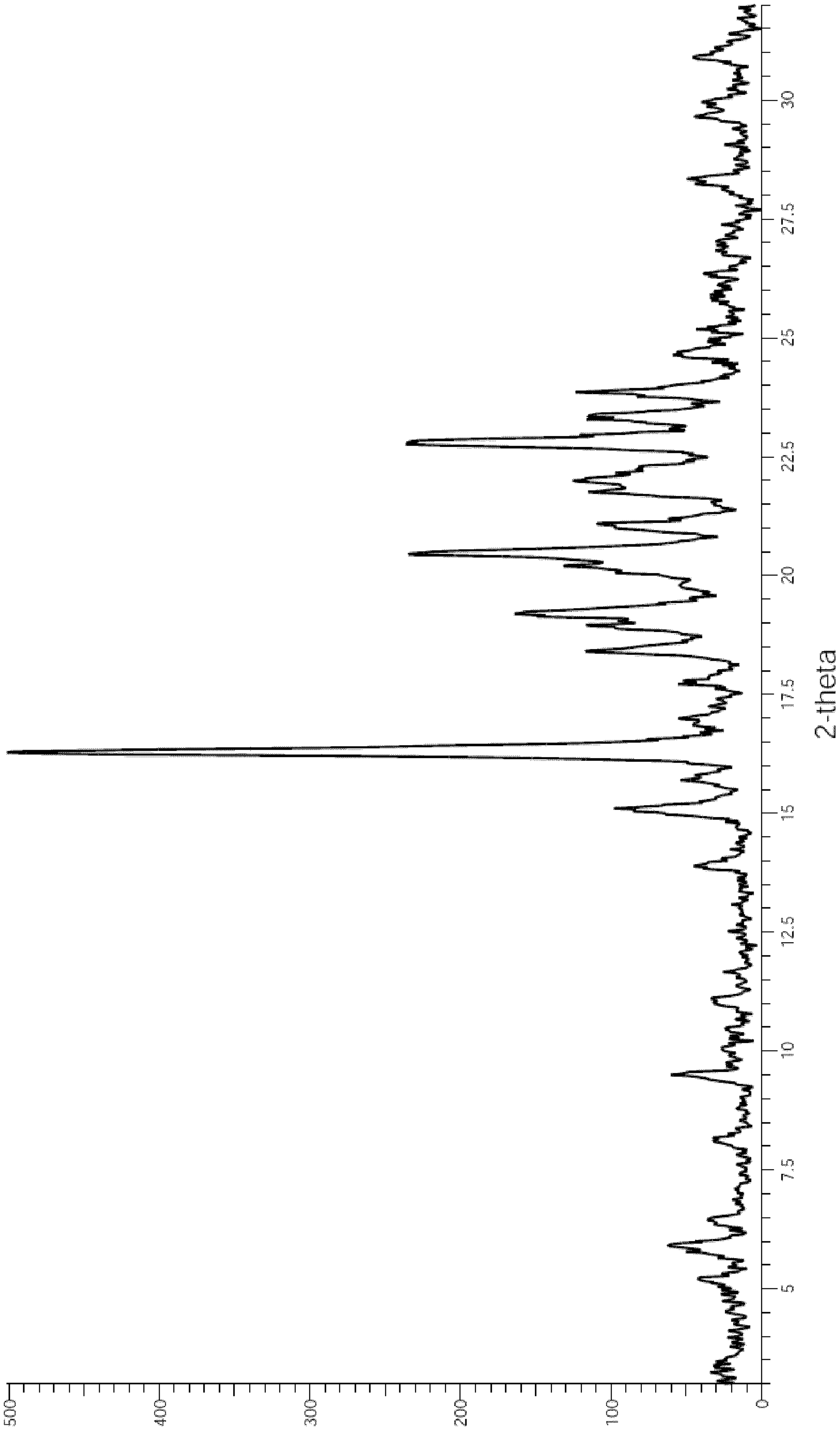

[Fig. 16]

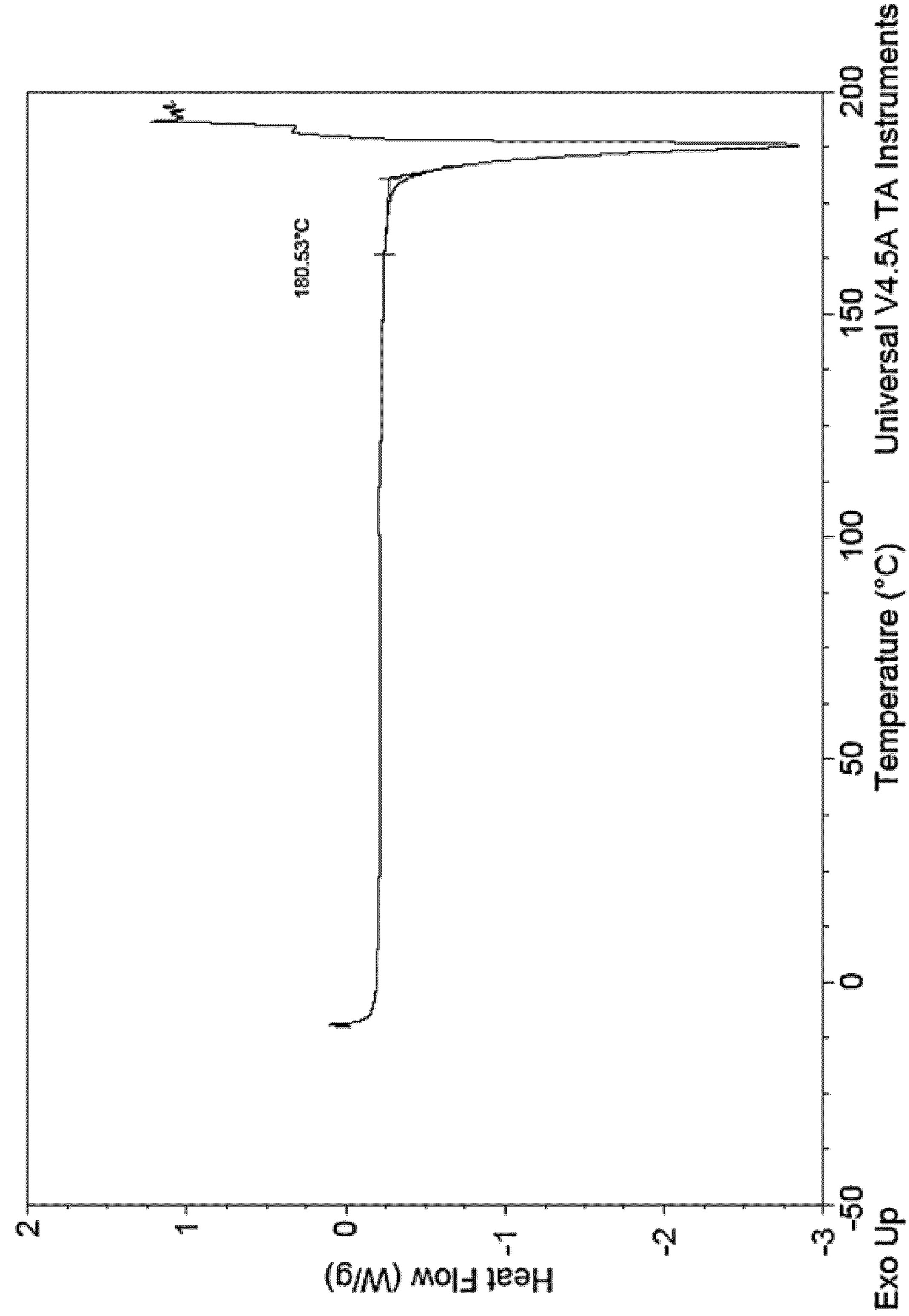
180.53°C
Heat Flow (W/g)
2
1
0
-1
-2
-3
-50
0
50
100
150
200
Temperature (°C)
Exo Up
Universal V4.5A TA Instruments

[Fig. 17]

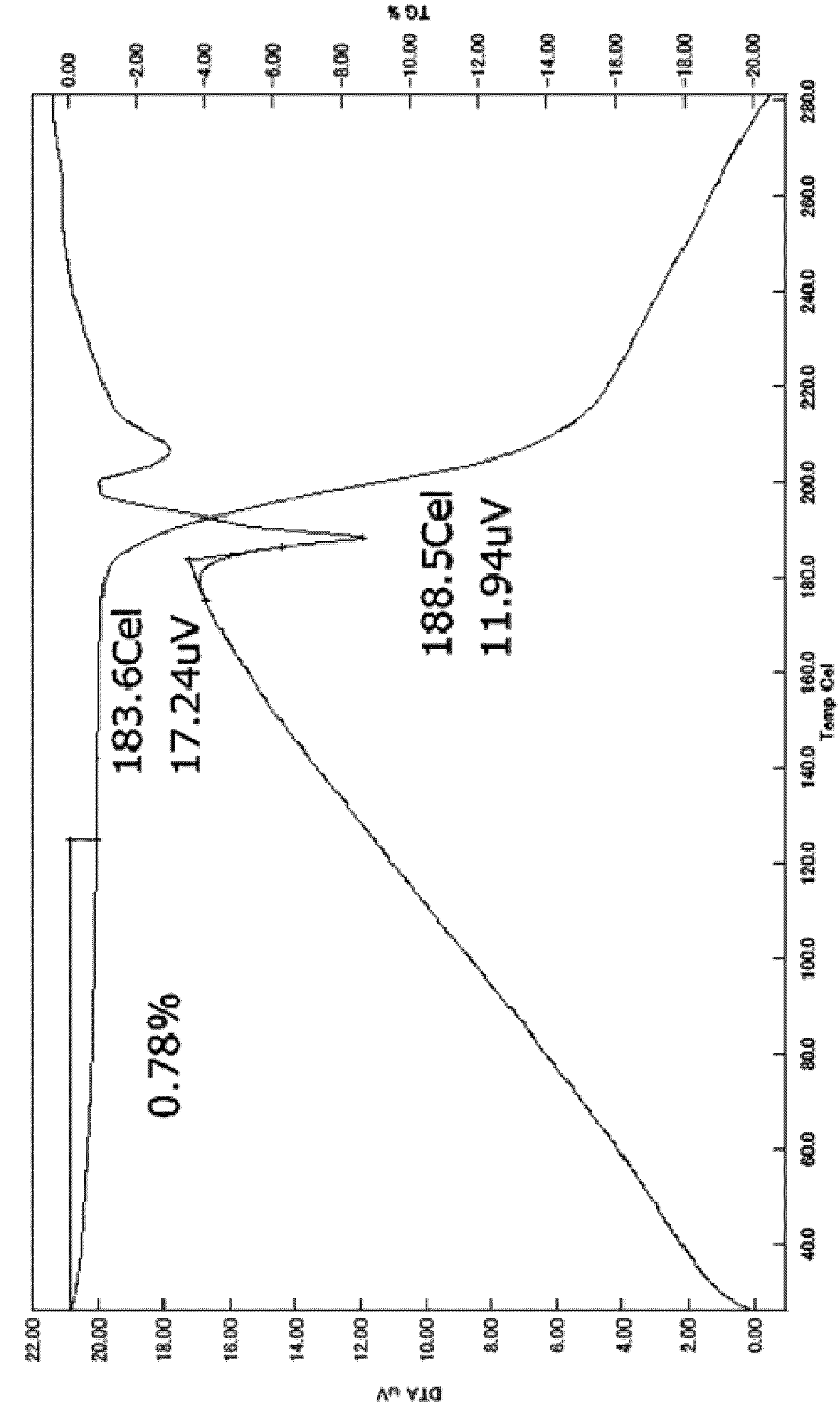
TG %
0.00
-2.00
-4.00
-6.00
-8.00
-10.00
-12.00
-14.00
-16.00
-18.00
-20.00
183.6Cel
17.24uV
188.5Cel
11.94uV
0.78%
40.0
60.0
80.0
100.0
120.0
140.0
160.0
180.0
200.0
220.0
240.0
260.0
280.0
Temp Cel
22.00
20.00
18.00
16.00
14.00
12.00
10.00
8.00
6.00
4.00
2.00
0.00
DTA uV

[Fig. 18]

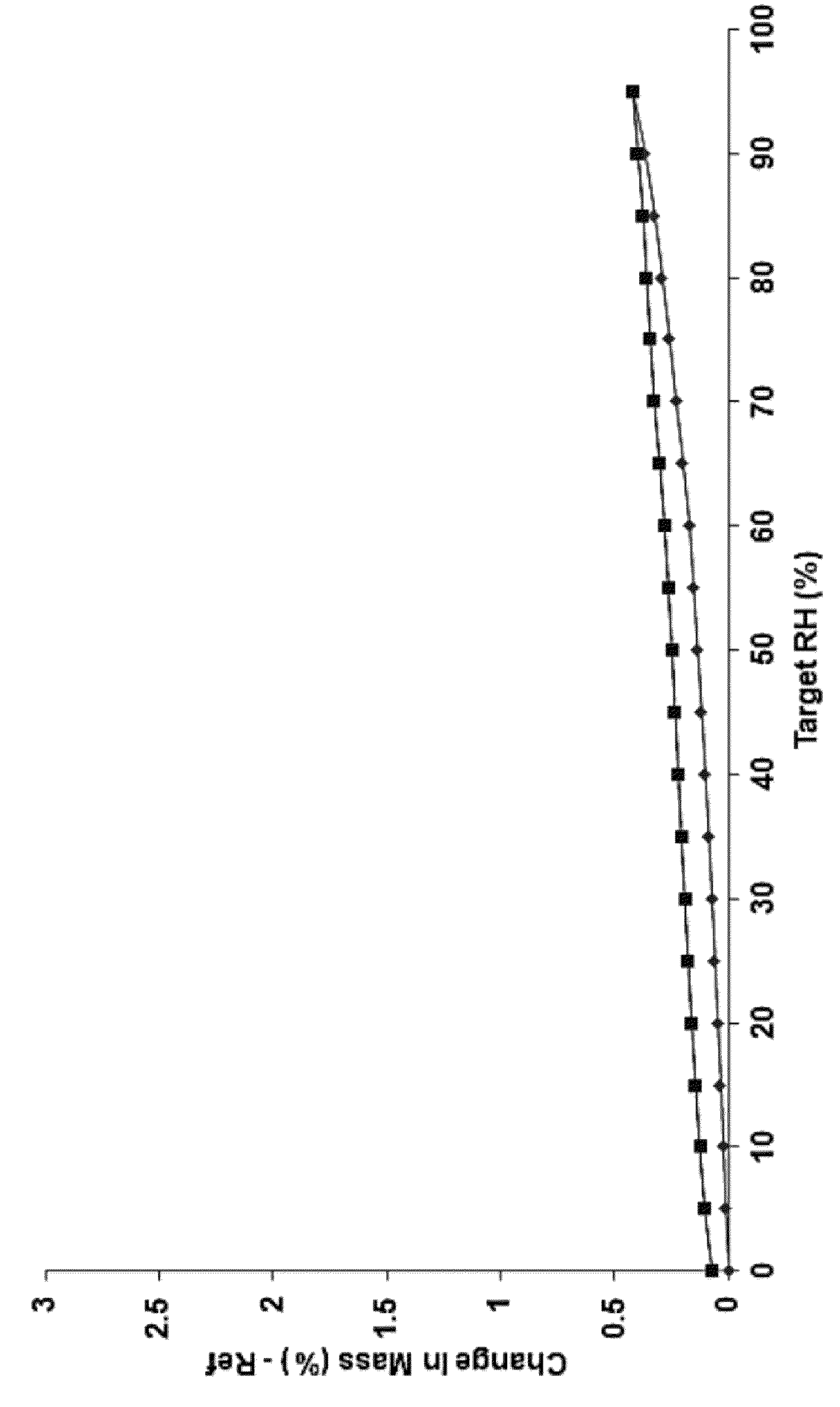

Change In Mass (%) - Ref
0
0.5
1
1.5
2
2.5
3
Target RH (%)
0
10
20
30
40
50
60
70
80
90
100

[Fig. 19]

| No. | Position | Intensity | No. | Position | Intensity | No. | Position | Intensity |
|---|---|---|---|---|---|---|---|---|
| 1 | 10.7400 | 456.0000 | 8 | 16.9200 | 820.0000 | 15 | 20.5000 | 560.0000 |
| 2 | 11.1400 | 289.0000 | 9 | 17.1200 | 483.0000 | 16 | 20.7000 | 426.0000 |
| 3 | 12.4200 | 317.0000 | 10 | 17.4600 | 429.0000 | 17 | 21.4600 | 425.0000 |
| 4 | 13.0400 | 974.0000 | 11 | 18.4200 | 1769.0000 | 18 | 23.7200 | 768.0000 |
| 5 | 14.0000 | 255.0000 | 12 | 19.2000 | 399.0000 | 19 | 24.2000 | 443.0000 |
| 6 | 15.5000 | 315.0000 | 13 | 19.8800 | 537.0000 | 20 | 24.5600 | 453.0000 |
| 7 | 16.5600 | 564.0000 | 14 | 20.1200 | 411.0000 | 21 | 26.1400 | 375.0000 |

[Fig. 20]

| No. | Position | Intensity | No. | Position | Intensity | No. | Position | Intensity |
|---|---|---|---|---|---|---|---|---|
| 1 | 10.8200 | 501.0000 | 8 | 16.7000 | 822.0000 | 15 | 22.9600 | 331.0000 |
| 2 | 11.1400 | 304.0000 | 9 | 17.1000 | 605.0000 | 16 | 23.8200 | 726.0000 |
| 3 | 12.4200 | 243.0000 | 10 | 18.3400 | 1676.0000 | 17 | 24.9600 | 341.0000 |
| 4 | 13.0800 | 870.0000 | 11 | 19.4800 | 406.0000 | 18 | 26.0800 | 364.0000 |
| 5 | 13.5400 | 215.0000 | 12 | 20.2000 | 639.0000 | 19 | 26.9200 | 306.0000 |
| 6 | 14.0200 | 270.0000 | 13 | 20.5200 | 625.0000 | | | |
| 7 | 15.6400 | 389.0000 | 14 | 21.5000 | 353.0000 | | | |

[Fig. 21]

| No. | Position | Intensity | No. | Position | Intensity | No. | Position | Intensity |
|---|---|---|---|---|---|---|---|---|
| 1 | 10.7400 | 527.0000 | 7 | 16.5600 | 612.0000 | 13 | 19.9800 | 442.0000 |
| 2 | 11.1400 | 313.0000 | 8 | 16.8800 | 710.0000 | 14 | 20.4400 | 590.0000 |
| 3 | 12.4000 | 281.0000 | 9 | 17.0800 | 505.0000 | 15 | 21.4400 | 388.0000 |
| 4 | 13.0400 | 945.0000 | 10 | 17.3800 | 437.0000 | 16 | 23.7400 | 658.0000 |
| 5 | 14.0000 | 304.0000 | 11 | 18.3800 | 1897.0000 | 17 | 24.0600 | 406.0000 |
| 6 | 15.5200 | 319.0000 | 12 | 19.2600 | 391.0000 | 18 | 26.1200 | 399.0000 |

[Fig. 2 2]

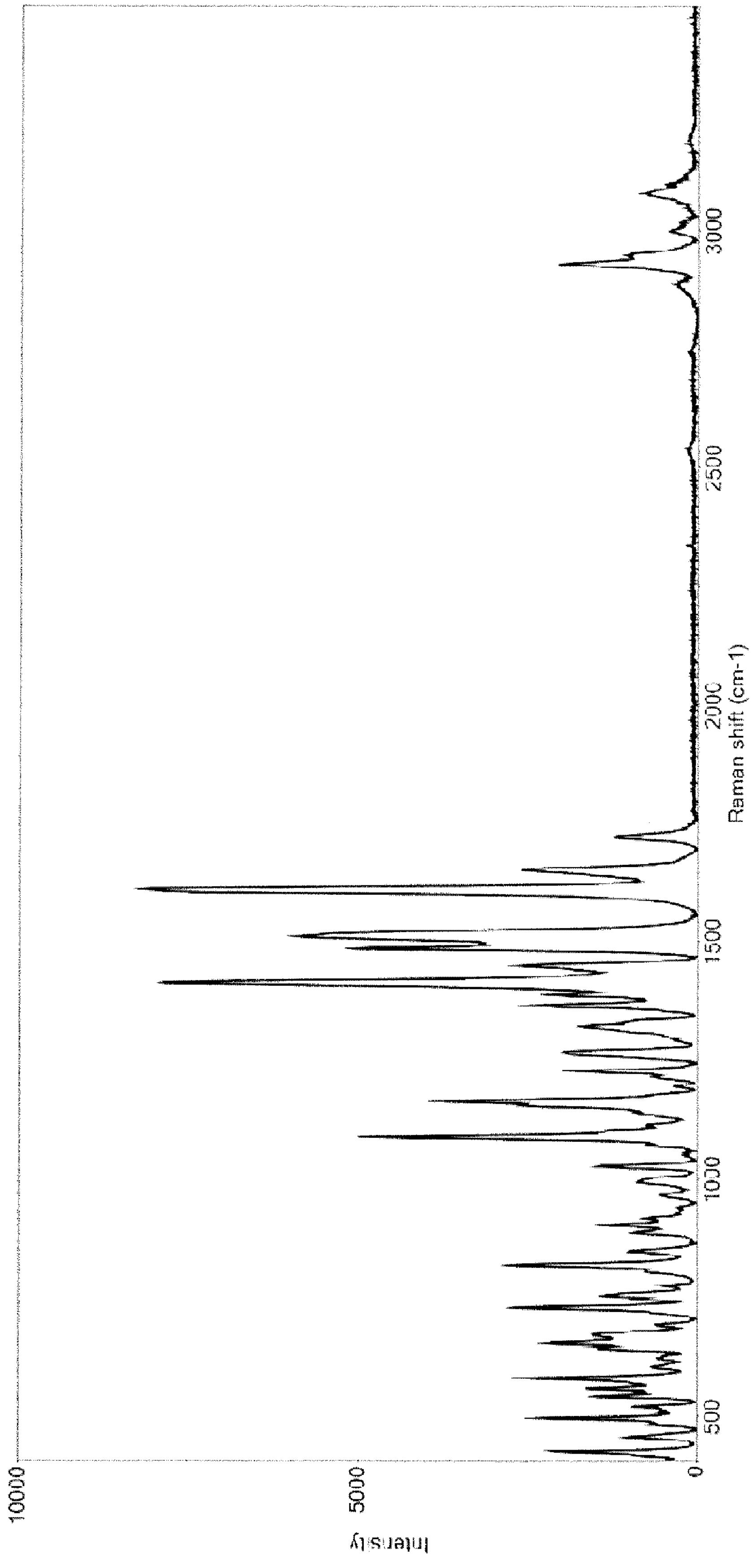

Intensity
10000
5000
0
500
1000
1500
2000
2500
3000
Raman shift (cm-1)

[Fig. 2 3]

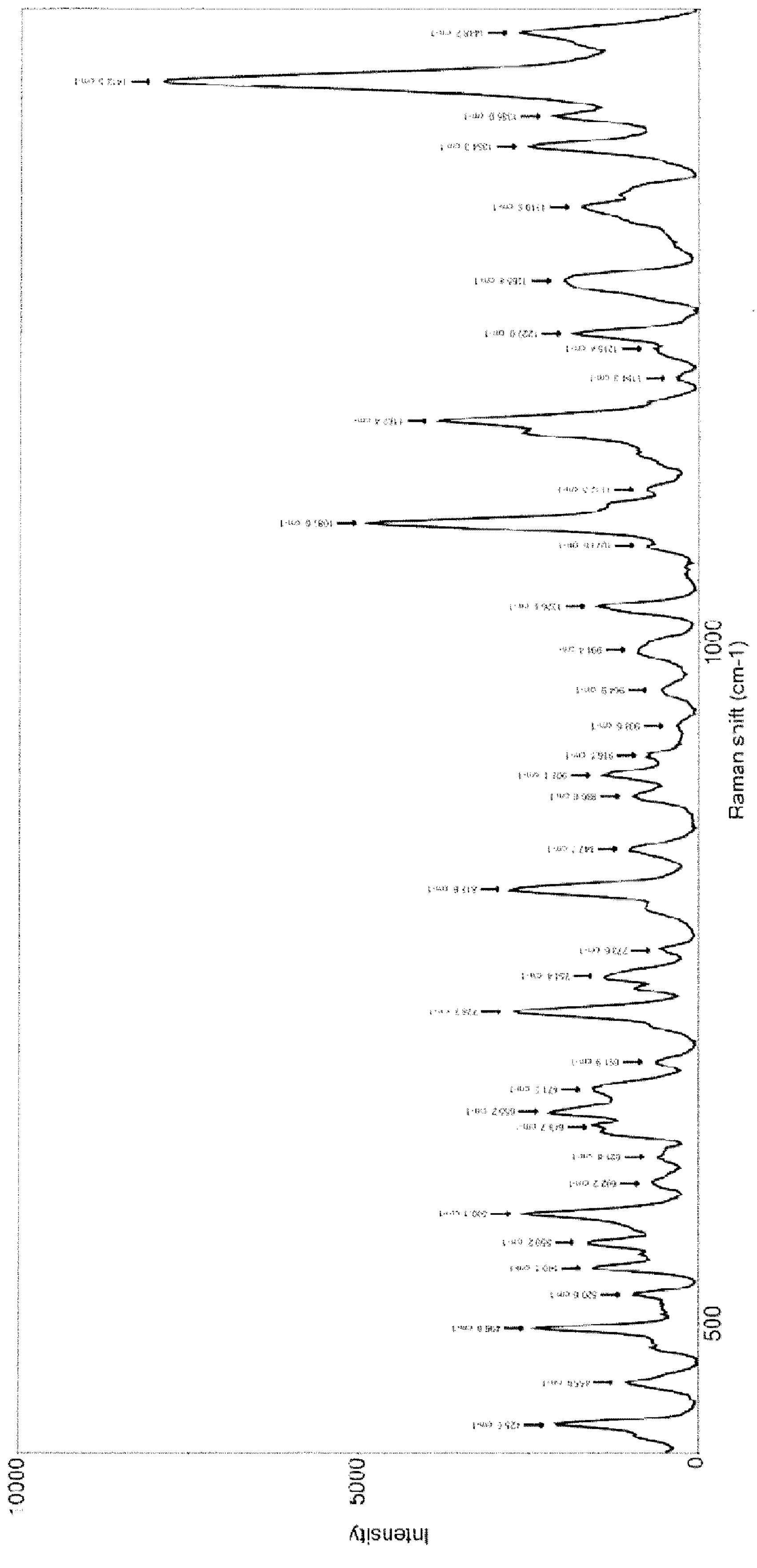

[Fig. 2 4]

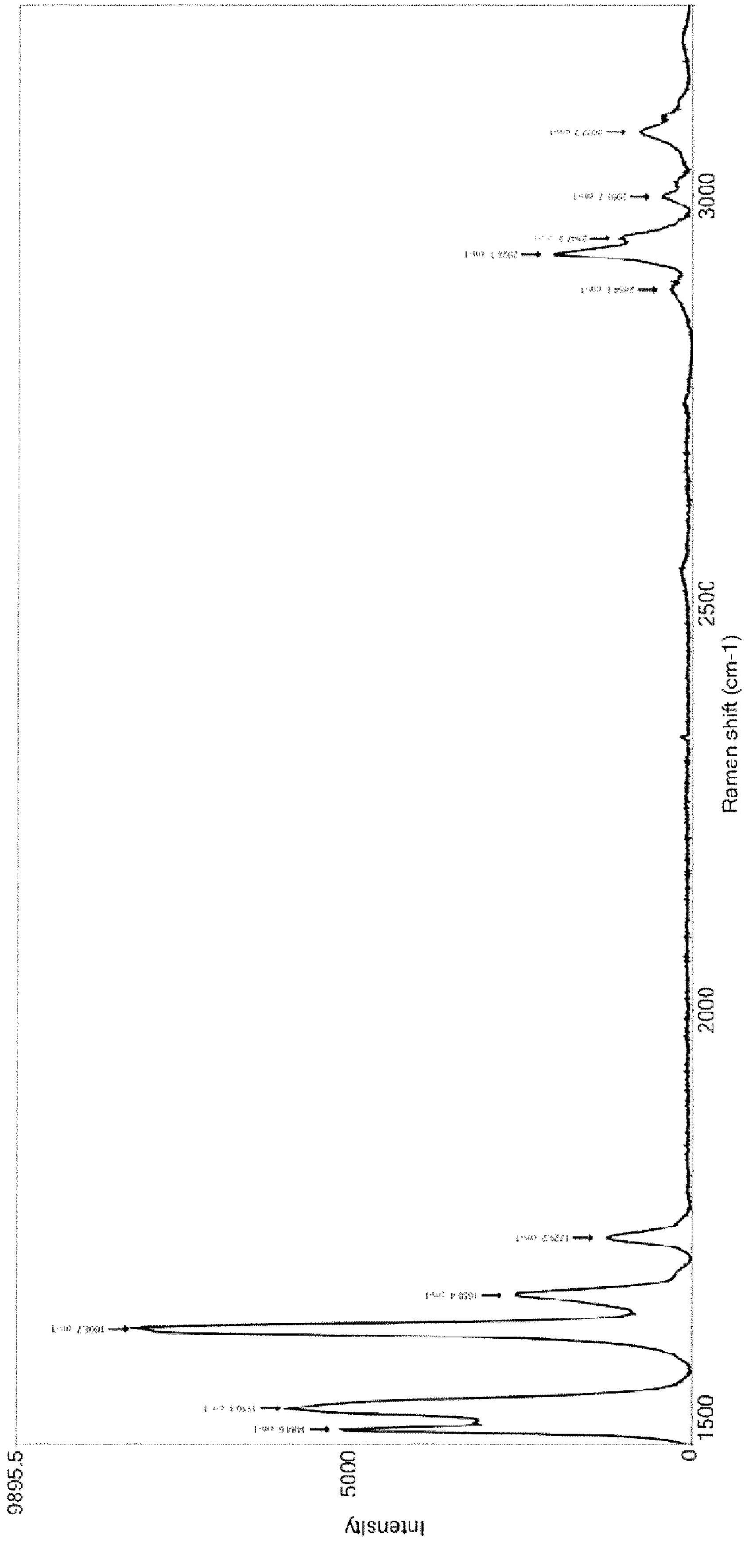

[Fig. 25]

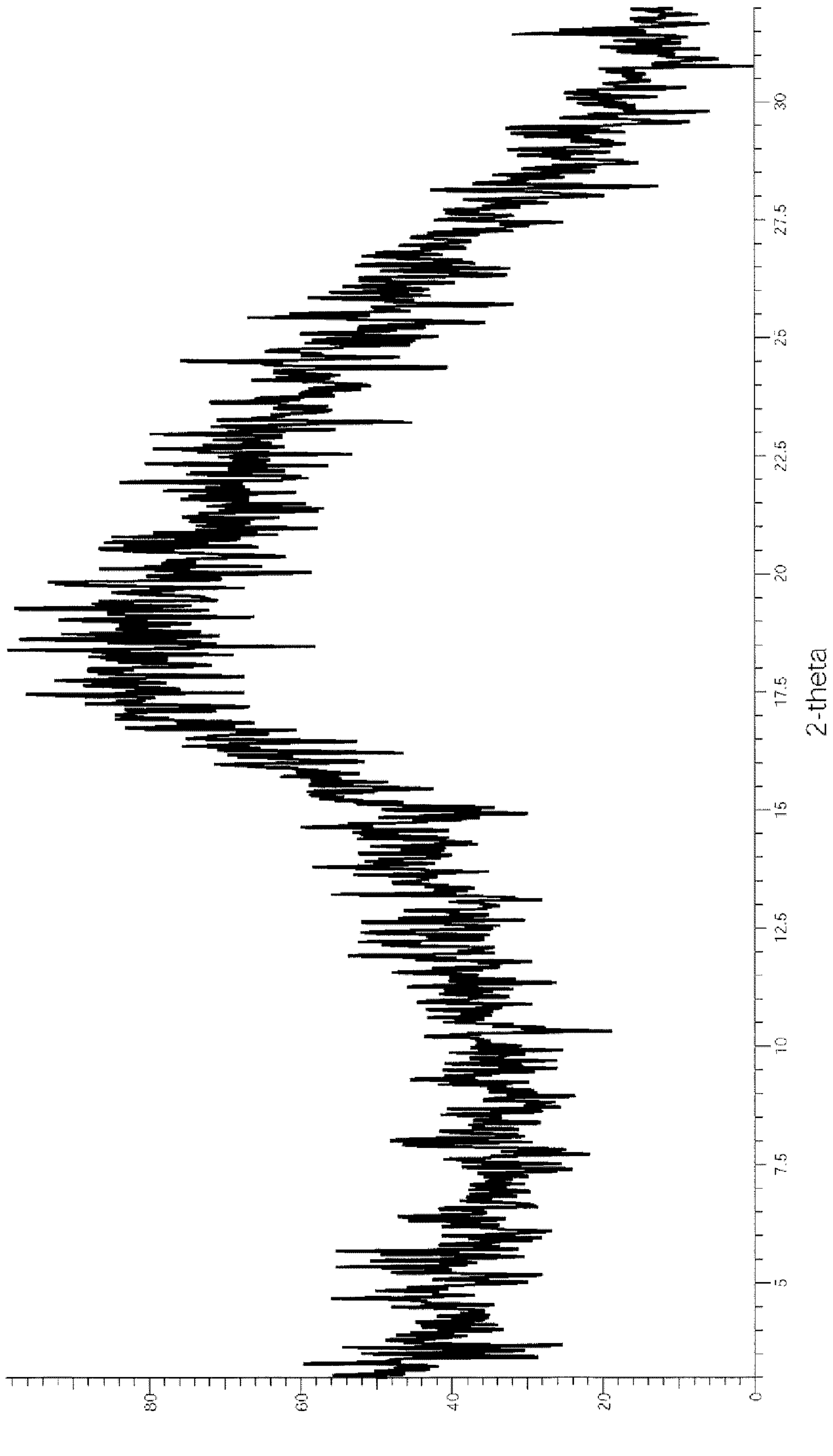

[Fig. 26]

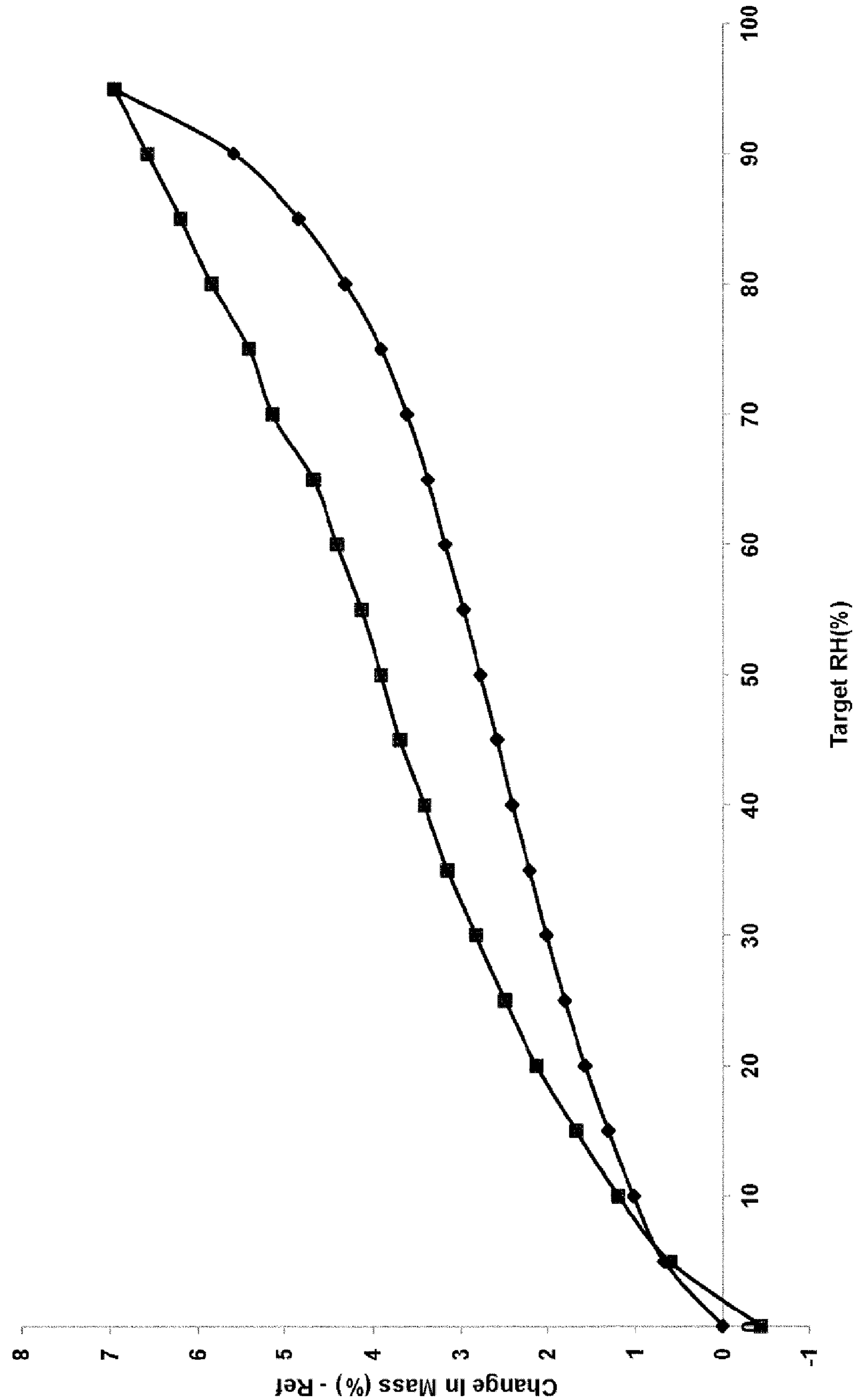
Change In Mass (%) - Ref
8
7
6
5
4
3
2
1
0
-1
Target RH(%)
0
10
20
30
40
50
60
70
80
90
100

[Fig. 2 7]

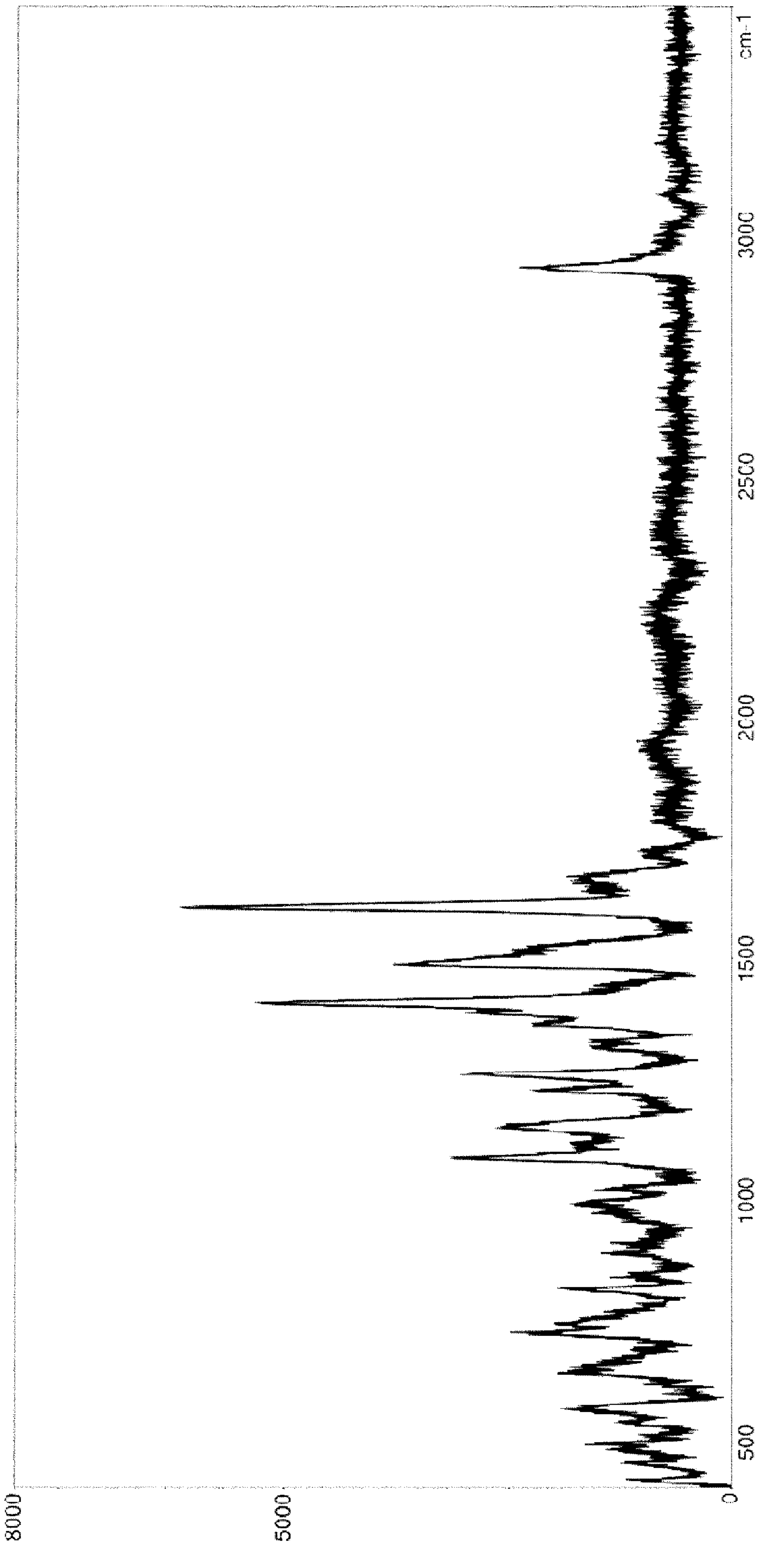

[Fig. 2 8]

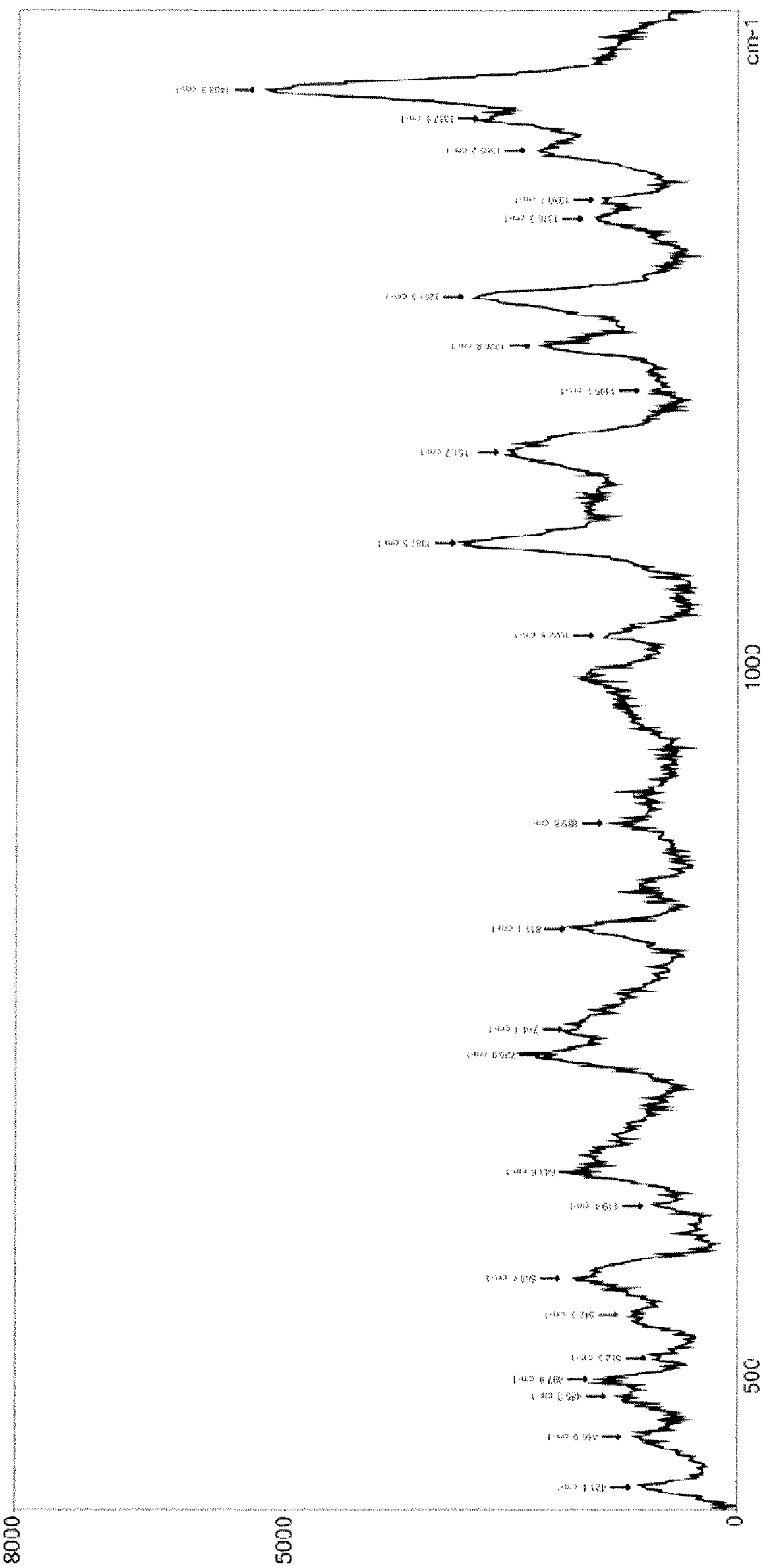

[Fig. 29]

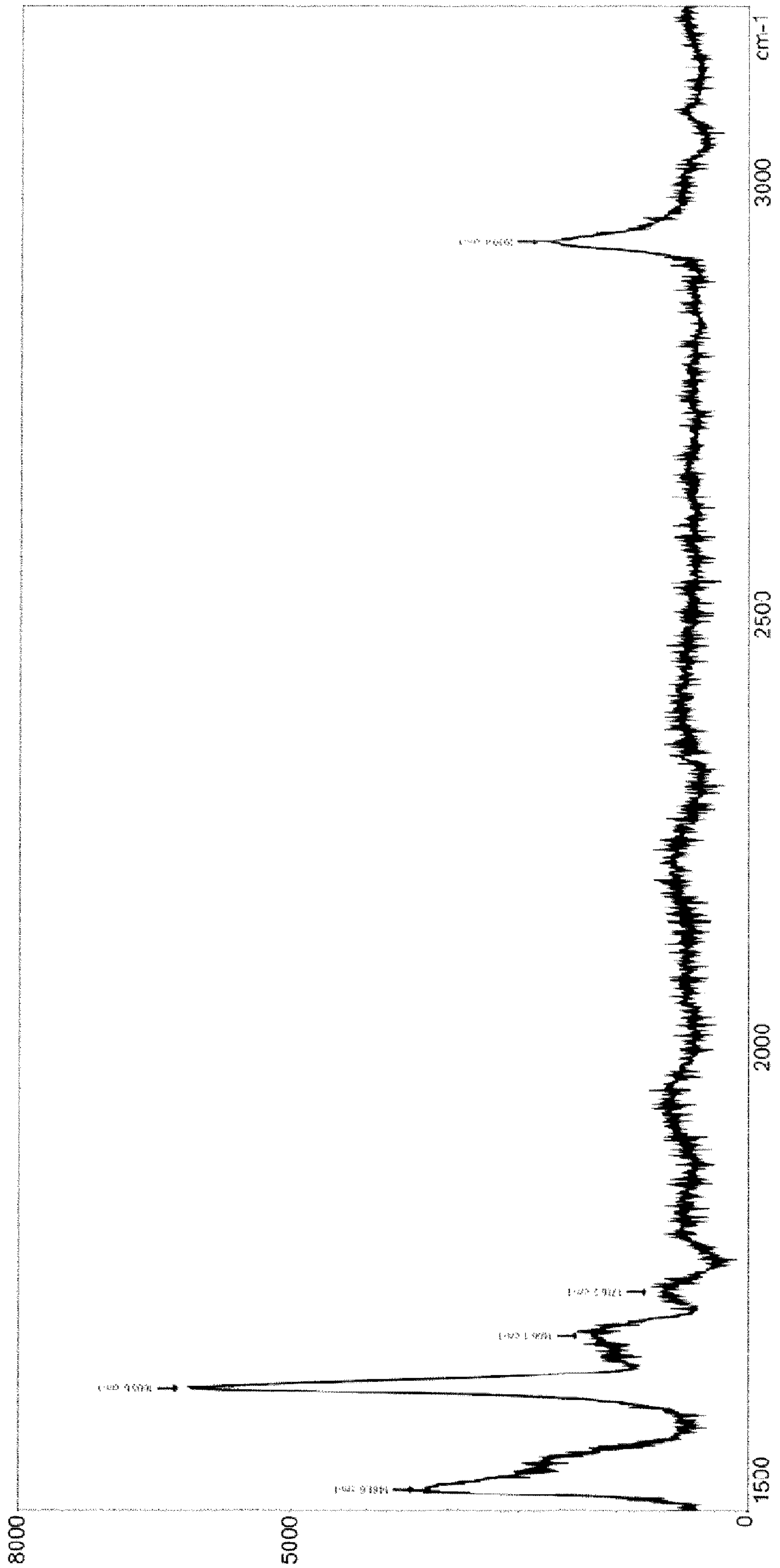

[Fig. 3 0]

| No. | Position | Intensity |
|---|---|---|
| 1 | 5.2000 | 61.9710 |
| 2 | 5.9000 | 81.7905 |
| 3 | 6.4400 | 55.5909 |
| 4 | 9.5000 | 79.9187 |
| 5 | 13.9000 | 64.5244 |
| 6 | 15.1000 | 117.7989 |
| 7 | 15.7000 | 73.7126 |
| 8 | 16.2800 | 520.8229 |
| 9 | 17.0000 | 74.8119 |
| 10 | 17.7200 | 75.1646 |
| 11 | 18.4000 | 136.5293 |
| 12 | 19.2000 | 183.7769 |
| 13 | 20.4400 | 254.2199 |
| 14 | 21.0800 | 129.1816 |
| 15 | 22.0000 | 144.8907 |
| 16 | 22.8000 | 255.1882 |
| 17 | 23.3600 | 134.9952 |
| 18 | 23.8600 | 143.1044 |
| 19 | 24.6400 | 78.7300 |

[Fig. 31]

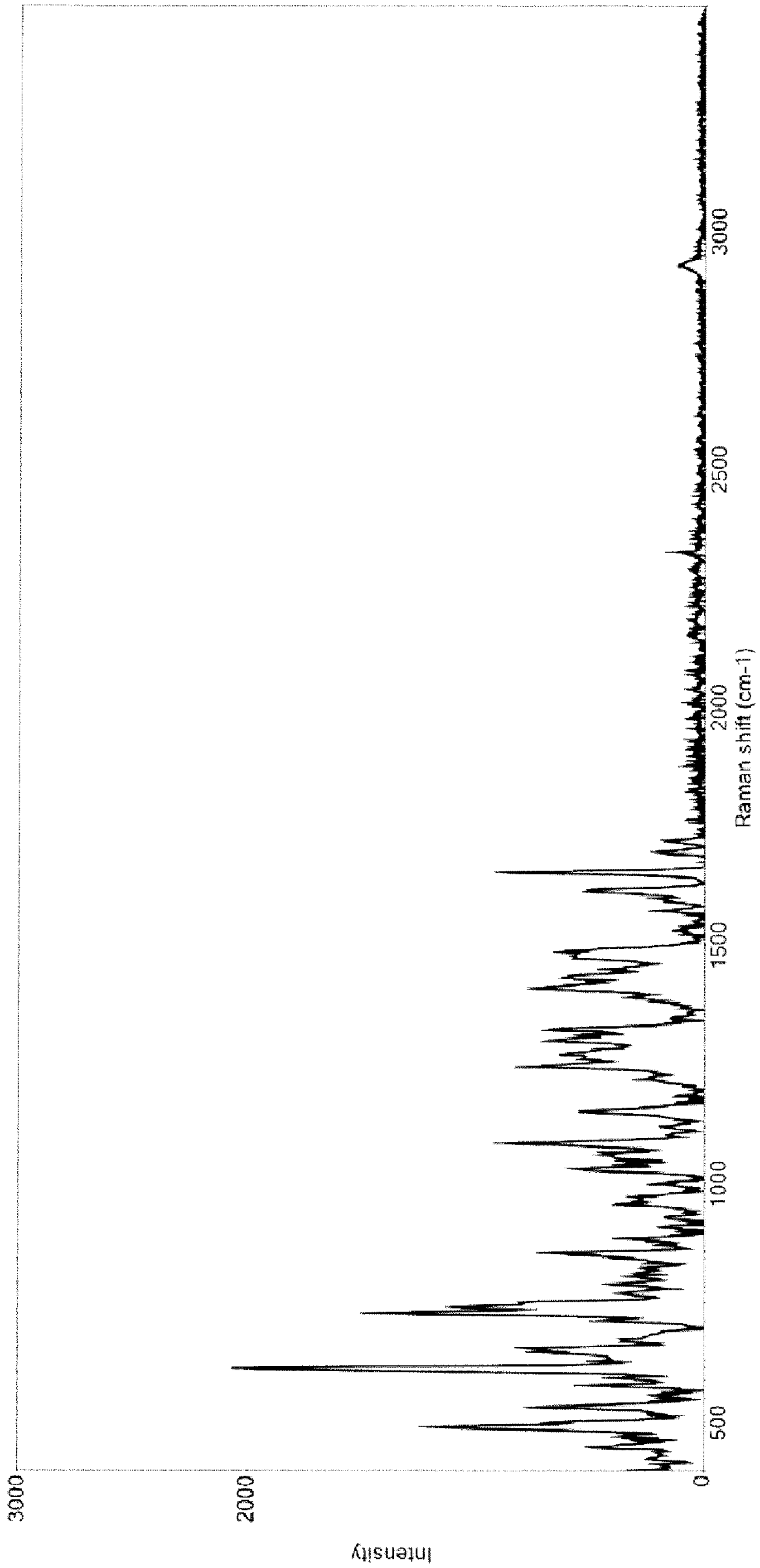

Intensity
3000
2000
0
Raman shift (cm-1)
500
1000
1500
2000
2500
3000

[Fig. 32]

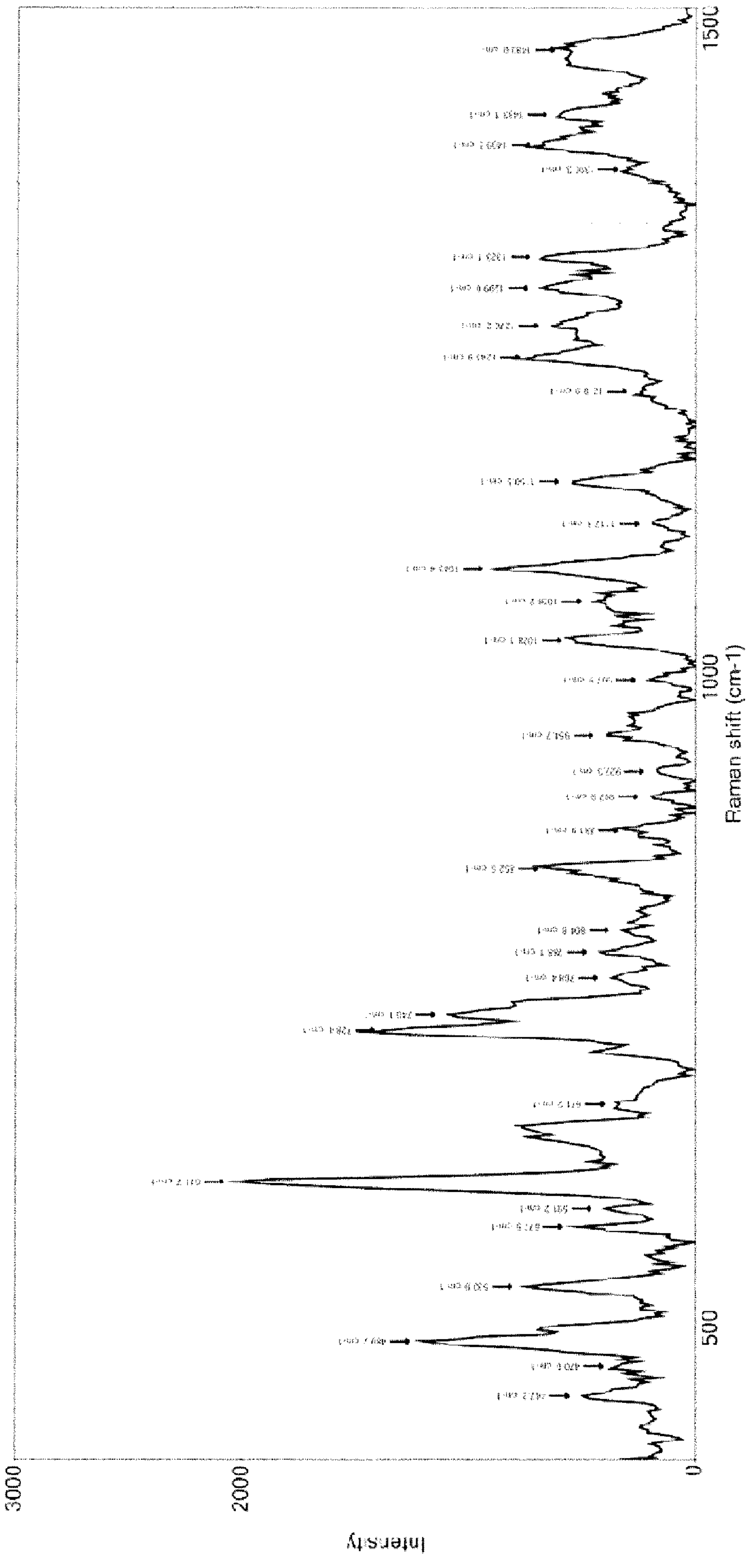

[Fig. 33]

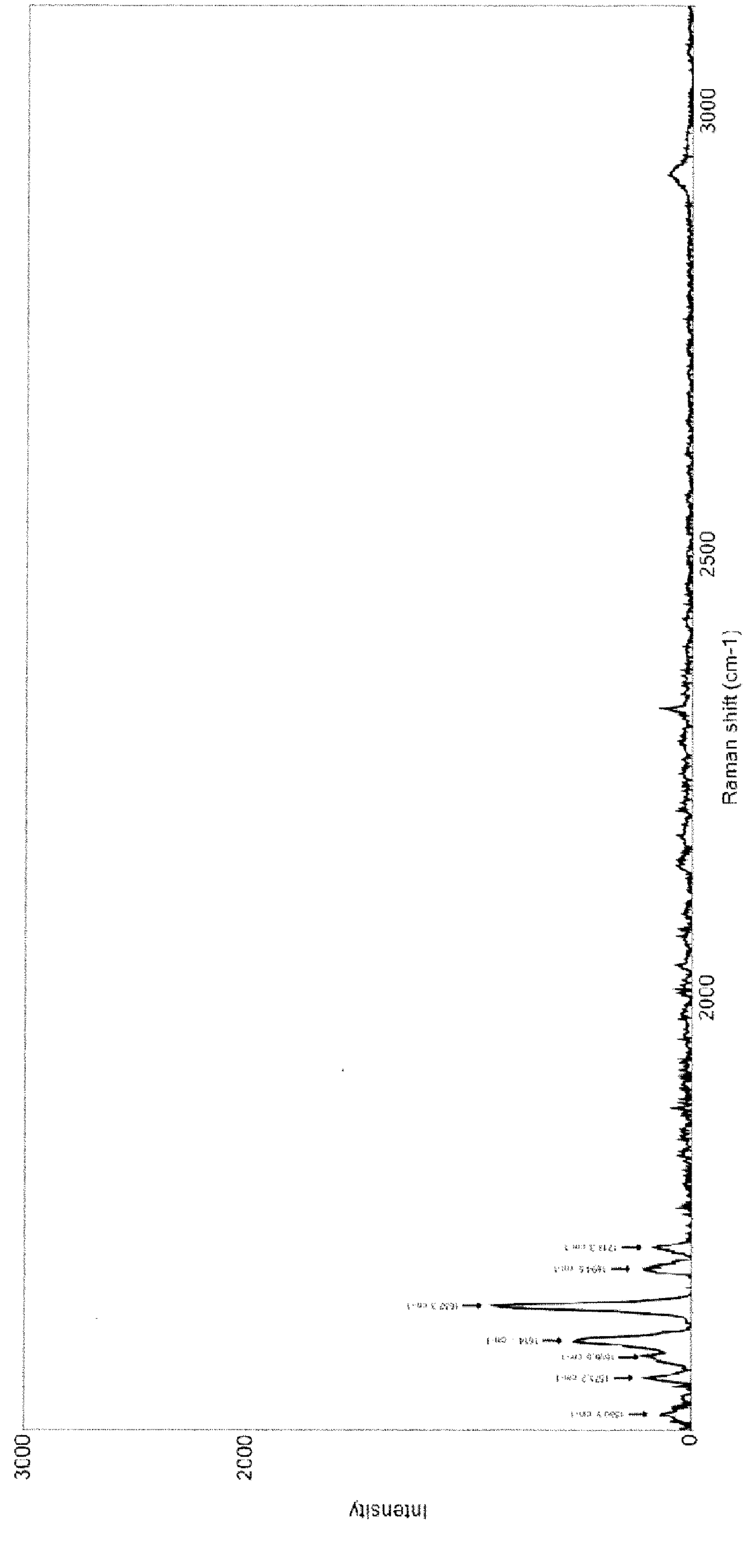

[Fig. 3 4]

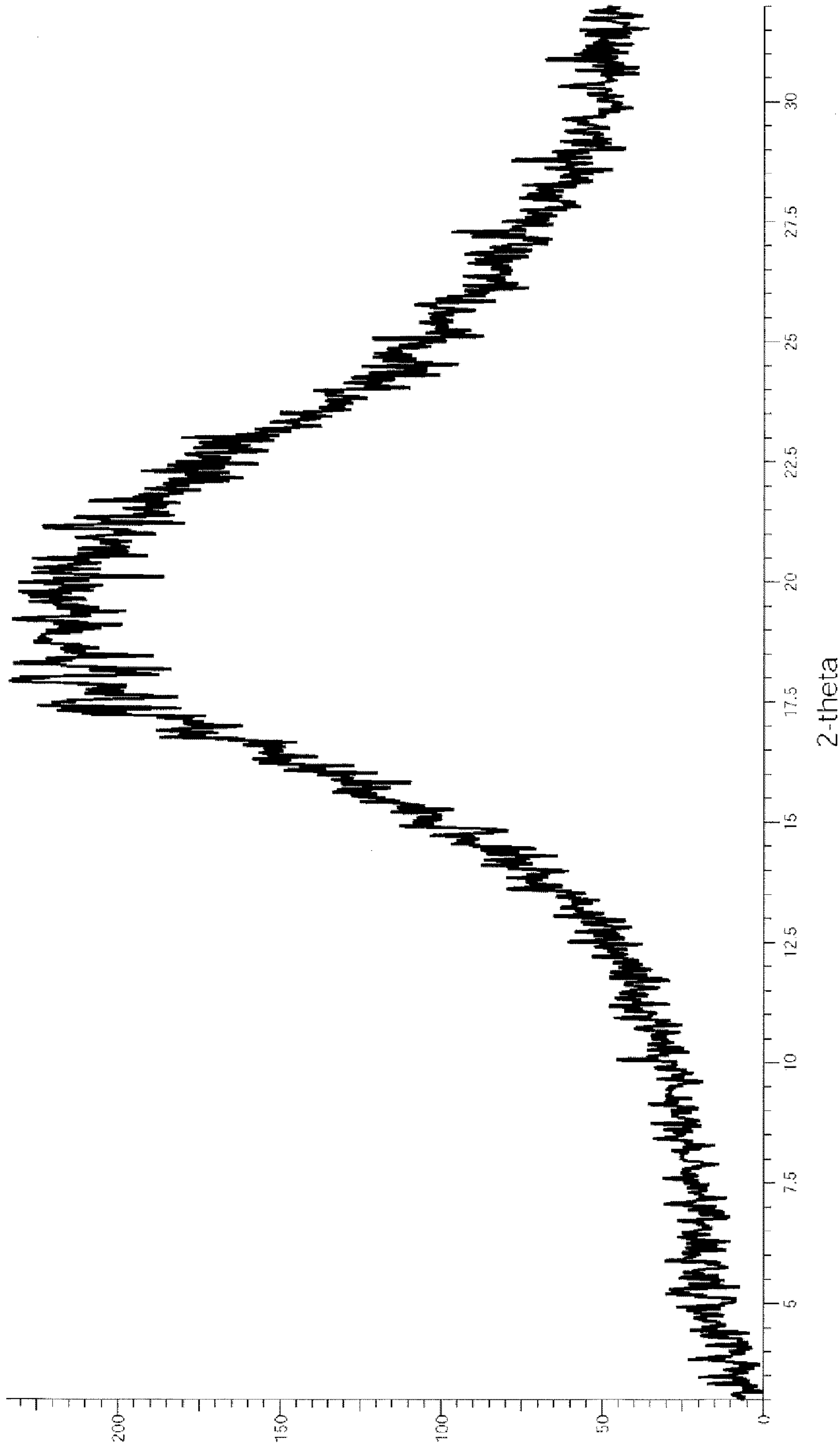

[Fig. 3 5]

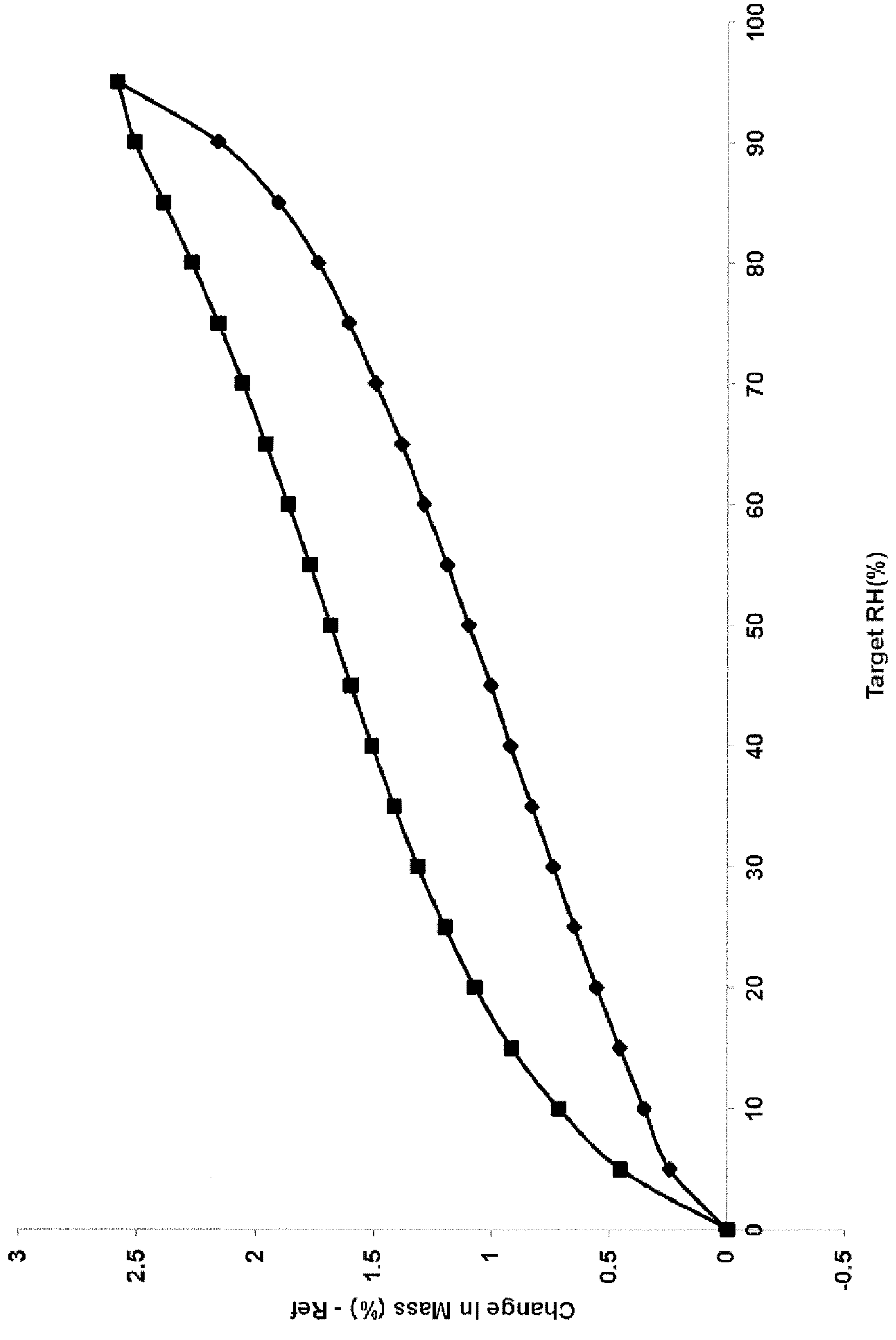
Change In Mass (%) - Ref
3
2.5
2
1.5
1
0.5
0
-0.5
Target RH(%)
0
10
20
30
40
50
60
70
80
90
100

[Fig. 3 6]

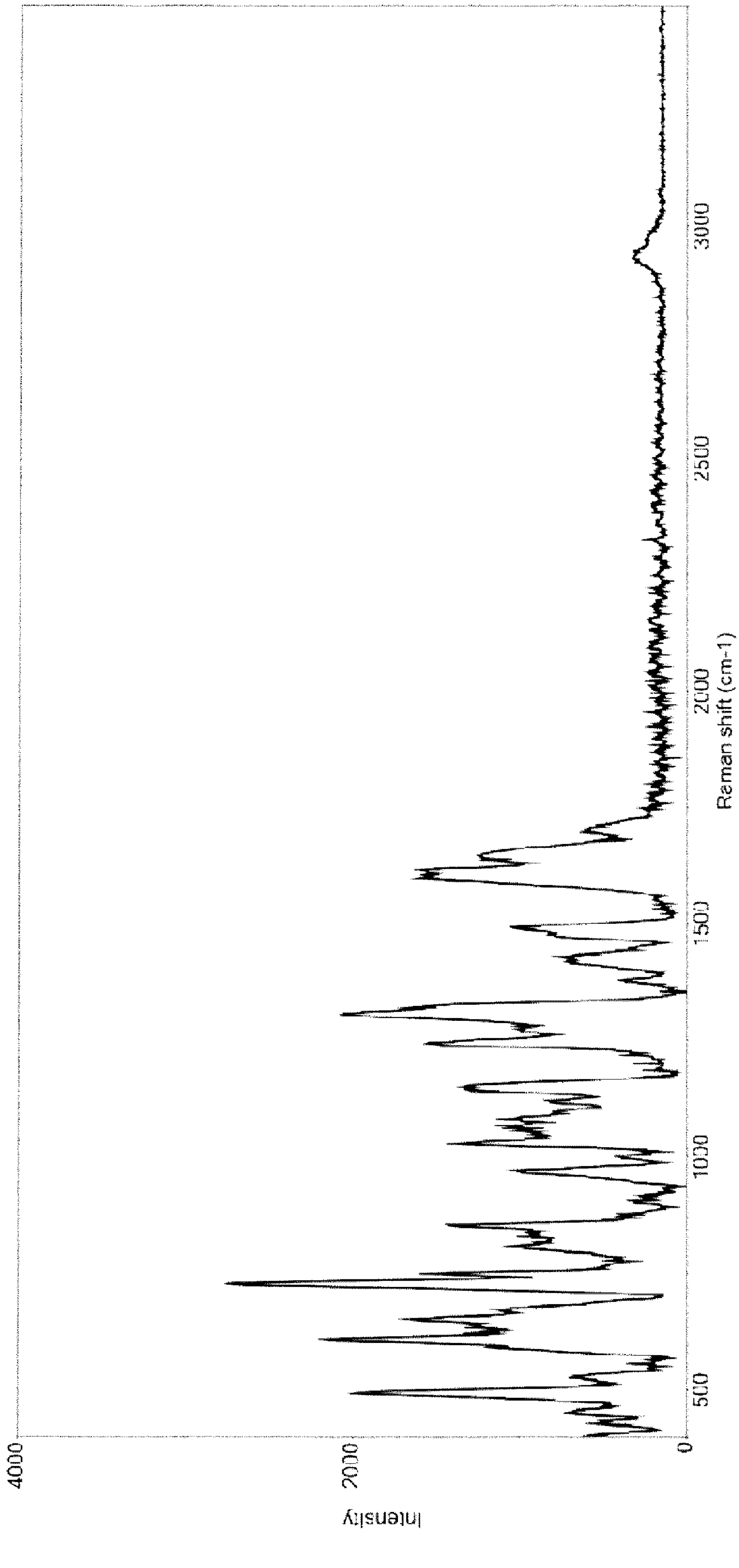
Intensity
4000
2000
0
Raman shift (cm-1)
500
1000
1500
2000
2500
3000

[Fig. 3 7]

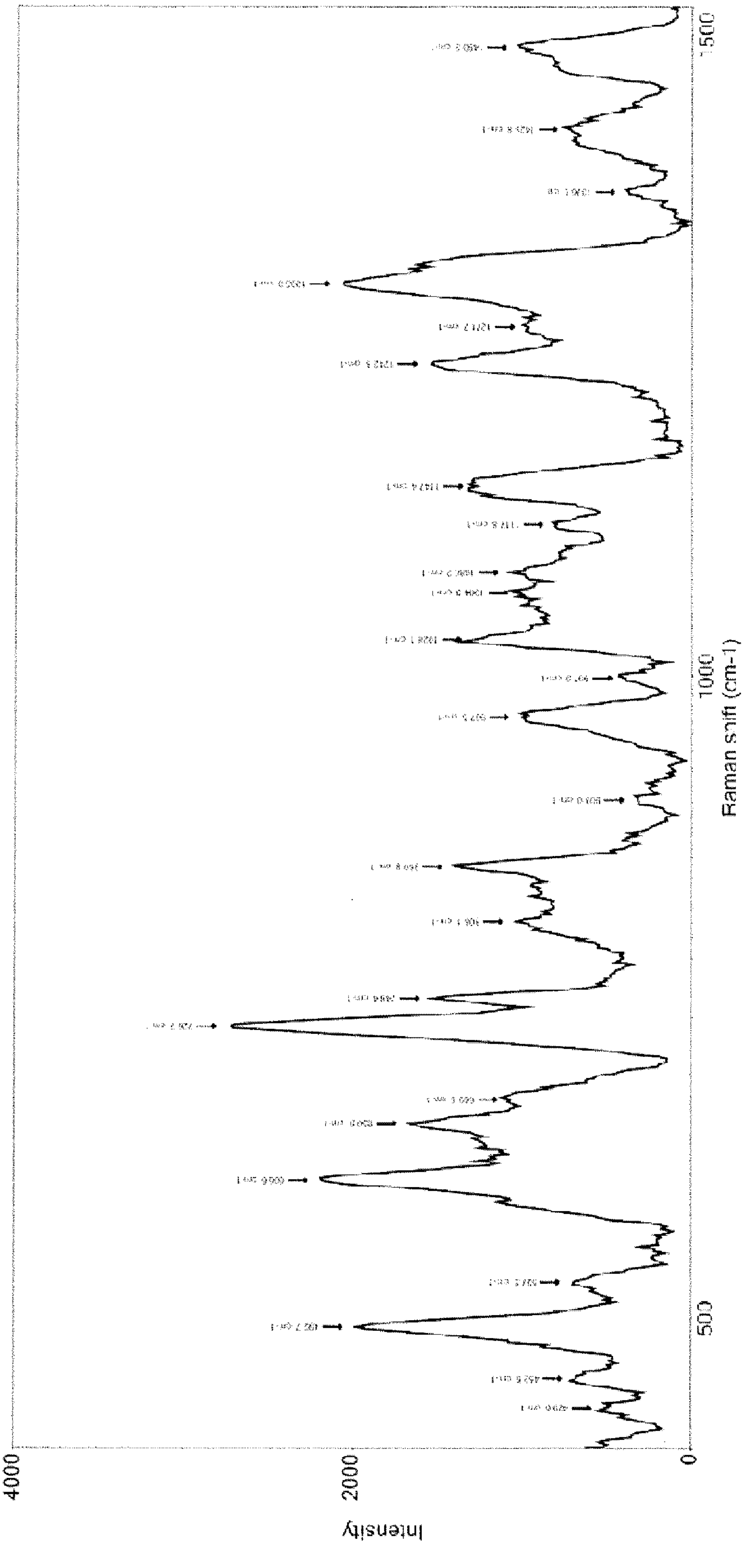

[Fig. 3 8]

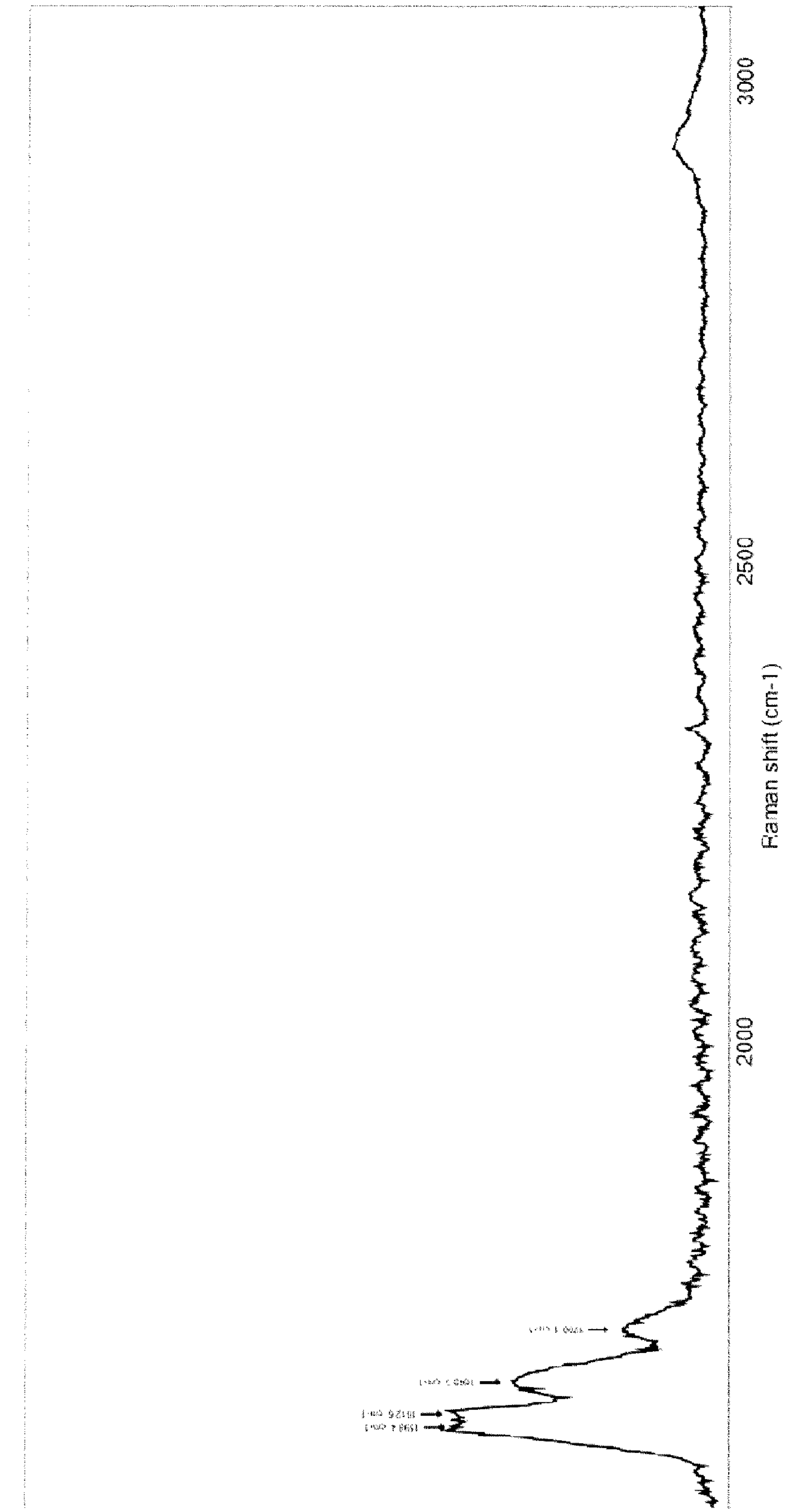

[Fig. 3 9]

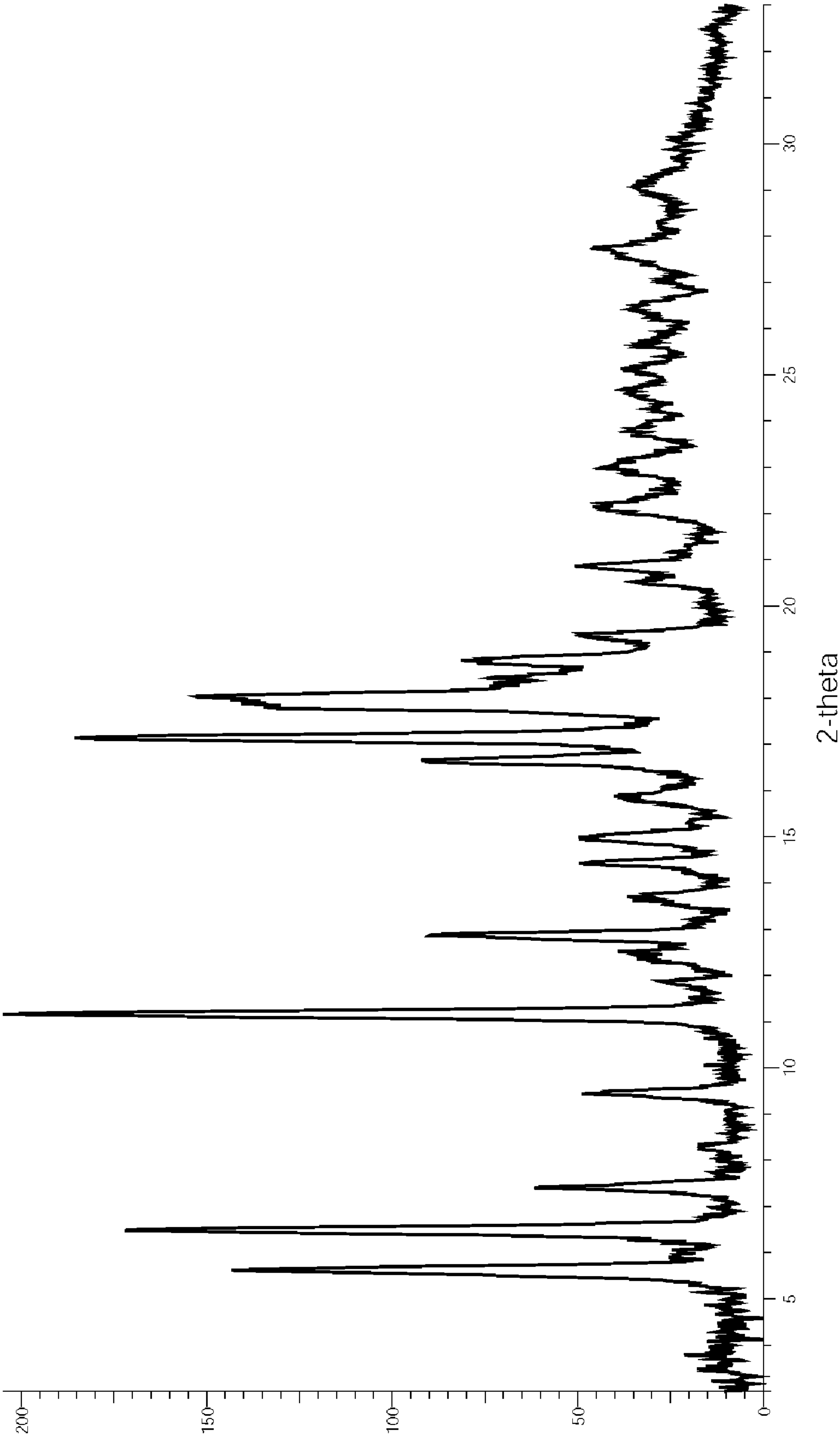

[Fig. 40]

| No. | Position | Intensity | No. | Position | Intensity | No. | Position | Intensity |
|---|---|---|---|---|---|---|---|---|
| 1 | 5.6200 | 152.2101 | 7 | 14.4200 | 58.3787 | 13 | 18.8200 | 90.2941 |
| 2 | 6.4800 | 181.0500 | 8 | 15.0000 | 58.5743 | 14 | 19.4000 | 58.3272 |
| 3 | 7.4000 | 70.5354 | 9 | 15.8800 | 49.0150 | 15 | 20.8600 | 59.7016 |
| 4 | 9.4400 | 57.6615 | 10 | 16.6600 | 101.0916 | 16 | 22.1400 | 54.8897 |
| 5 | 11.1600 | 213.8208 | 11 | 17.1400 | 194.3320 | 17 | 22.9800 | 53.0564 |
| 6 | 12.8600 | 98.8102 | 12 | 18.0400 | 161.9883 | 18 | 27.7400 | 54.8434 |

**INTERNATIONAL SEARCH REPORT**

International application No. **PCT/JP2023/021799**

**A. CLASSIFICATION OF SUBJECT MATTER**

***C07D 491/20***(2006.01)i; ***A61K 31/4427***(2006.01)i; ***A61P 3/04***(2006.01)i; ***A61P 43/00***(2006.01)i
FI: C07D491/20 CSP; A61K31/4427; A61P43/00 105; A61P3/04

According to International Patent Classification (IPC) or to both national classification and IPC

**B. FIELDS SEARCHED**

Minimum documentation searched (classification system followed by classification symbols)

C07D491/20; A61K31/4427; A61P3/04; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/013311 A1 (SHIONOGI & CO., LTD.) 17 January 2019 (2019-01-17) claims, examples 12, 14, paragraph [0064] | 1-17 |
| Y | 平山 令明 編, 有機化合物結晶作製ハンドブック —原理とノウハウ—, 丸善株式会社, 25 July 2008, pp. 57-85 p. 57, line 1 to p. 58, line 28, p. 72, lines 1-6, p. 78, lines 14-24, non-official translation (Edited by HIRAYAMA, Noriaki. Handbook for preparation of organic compound crystals -Principles and Expertise-. MARUZEN INC.) | 1-17 |
| A | JP 2014-506907 A (JANSSEN PHARMACEUTICA NAAMLOZE VENNOOTSCHAP) 20 March 2014 (2014-03-20) claims, examples | 1-17 |
| A | JP 2016-537369 A (-BRISTOL-MYERS SQUIBB COMPANY) 01 December 2016 (2016-12-01) claims, examples | 1-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 July 2023** | **18 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/021799**

| Patent document cited in search report | Publication date (day/month/year) | Patent family member(s) | Publication date (day/month/year) |
|---|---|---|---|
| WO 2019/013311 A1 | 17 January 2019 | US 2020/0291025 A<br>claims, examples 12, 14, paragraph [0407]<br>EP 3653625 A1<br>CN 111094288 A<br>KR 10-2020-0029549 A | |
| JP 2014-506907 A | 20 March 2014 | US 2014/0005200 A1<br>claims, examples<br>WO 2012/117027 A1<br>EP 2681219 A1<br>KR 10-2014-0010052 A | |
| JP 2016-537369 A | 01 December 2016 | US 2015/0133428 A1<br>claims, examples<br>WO 2015/073763 A1<br>EP 3068784 A1<br>CN 105916855 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019013311 A
- WO 2019013312 A


### Non-patent literature cited in the description


- *Journal of Biological Chemistry*, 2004, vol. 279, 18878-18886
- *Nature Medicine*, 2009, vol. 15 (4), 442-446
- *Isotopes in the Physical and Biomedical Sciences*, 1987, vol. 1
- **TOSHIO SAKURAI**. Guidance on X-ray Structural Analysis. Shokabo Co., Ltd., 1983
- **STOUT** ; **JENSEN**. X-Ray Structure Determination: A Practical Guide. Macmillan Co, 1968